# EUROPEAN PATENT APPLICATION

(11) **EP 4 295 911 A2**
(43) Date of publication of application: **27.12.2023**
(21) Application number: 23197405.6
(22) Date of filing: 05.11.2015
(51) Int. Cl.: A61P 25/28

(54) **HUMANIZED ANTI-COMPLEMENT FACTOR C1Q ANTIBODIES AND USES THEREOF**

(30) Priority: 05.11.2014 US 201462075793 P
(62) Divisional of application: 15857258.6
(71) Applicant: Annexon, Inc., Brisbane, CA 94005 (US)
(72) Inventor: ROSENTHAL, Amon, Woodside, 94062 (US); LEVITEN, Michael, Emerald Hills, 94062 (US)
(74) Representative: Vossius & Partner Patentanwälte Rechtsanwälte mbB

(57) **Abstract**

The present disclosure is directed to humanized anti-C1q antibodies and methods of using the same.

## Description

### RELATED APPLICATIONS

This Application claims the benefit of U.S. Provisional Application 62/075,793,
filed November 5th, 2014, the entire contents of which are incorporated herein by
reference.

BACKGROUND

1.          Field

The present disclosure relates to anti-C1q antibodies and methods of using the
same.

2.          Description of Related Art

Excessive complement activation has been associated with a range of disease
conditions, including numerous inflammatory and autoimmune diseases. More recently,
the complement system has also been shown to contribute to neurodegenerative disease
pathology. Specifically, complement factors, such as C1q, were shown to be expressed in
neuronal synapses and to mark these synapses for elimination. See, e.g., U.S. Patent
Publication Nos. 2012/0195880 and 2012/328601. While selective synapse loss is an
essential aspect of normal brain development ("synaptic pruning"), excessive synapse loss,
especially in a mature or aging brain, results in neurodegeneration and cognitive decline.
Elevated synaptic complement expression was found to contribute to synaptic loss in
normal aging and in neurodegenerative disease progression. Conversely, lowering neuronal
complement expression was found to be neuroprotective. Based on these findings,
neutralizing the activity of complement factors such as C1q is regarded as a promising
therapeutic strategy to prevent synapse loss and to slow neurodegenerative disease
progression as well as cognitive decline in normal aging.

Neurodegenerative diseases involving synapse loss and considered to be
amenable to treatments aiming at the neutralization of complement factors such as C1q
include Alzheimer's disease, amyotrophic lateral sclerosis, multiple sclerosis, glaucoma,
myotonic dystrophy, Down syndrome, Parkinson's disease, Huntington's disease, and the
like.

Only a limited number of complement neutralizing antibodies are known to date
(see, e.g., Klos A. et al., Mol Immunol. 2009, 46(14), 2753-2766; Carroll S. & Georgiou G.,
Immunobiology 2013, 218(8), 1041-1048; Tuzun et al., J. Neuroimmunol. 2007, 182, 167–
176; Nelson et al., J. Clin. Invest. 2006, 116:2892–2900; Heinz et al., J. Immunol. 1984,
133, 400-404; Jiang et al., J. Immunol. 1991, 146, 2324-2330; Trinder et al., Scand. J.
Immunol. 1999, 50, 635–641; Hwang et al., Mol. Immunol. 2008, 45, 2570–2580). Only
the C5 neutralizing antibody Eculizumab, an inhibitor of the terminal complement
activation pathway, has obtained regulatory approval to date; Eculizumab is marketed for
the treatment of paroxysmal nocturnal hemoglobinuria (PNH; Hillmen et al., N Engl J Med.
2006, 355(12):1233-43).
Thus, there is a need to develop further antibodies that specifically bind to and
neutralize biological activities of complement factors such as C1q.
All references cited herein, including patent applications and publications, are
hereby incorporated by reference in their entirety.

BRIEF SUMMARY
Provided herein are humanized anti-C1q antibodies and methods of using
humanized anti-C1q antibodies.
In certain aspects, the present disclosure provides a humanized antibody that
specifically binds to a C1q protein, wherein the antibody comprises a heavy chain variable
region and a human heavy chain constant region, wherein the heavy chain variable region
comprises an Fab region and the heavy chain constant region comprises an Fc region,
wherein the Fab region specifically binds to the C1q protein, and wherein the Fc region is
incapable of binding the C1q protein.
In some embodiments that may be combined with any of the preceding
embodiments, the Fc region is incapable of inducing complement activity. In some
embodiments that may be combined with any of the preceding embodiments, the Fc region
is incapable of inducing antibody-dependent cellular cytotoxicity (ADCC). In some
embodiments that may be combined with any of the preceding embodiments, the human
heavy chain constant region is a human IgG4 heavy chain constant region. In some
embodiments that may be combined with any of the preceding embodiments, the human
IgG4 heavy chain constant region comprises the amino acid sequence of SEQ ID NO: 37,
or an amino acid sequence with at least about 90% homology to the amino acid sequence of

SEQ ID NO: 37. In some embodiments that may be combined with any of the preceding
embodiments, the human IgG4 heavy chain constant region comprises an Fc region, and
wherein the Fc region comprises one or more modifications. In some embodiments that
may be combined with any of the preceding embodiments, the Fc region comprises one or
more amino acid substitutions. In some embodiments that may be combined with any of the
preceding embodiments, the Fc region comprises an amino acid substitution at position 248
according to Kabat numbering convention. In some embodiments that may be combined
with any of the preceding embodiments, the Fc region comprises a leucine to glutamate
amino acid substitution at position 248 according to Kabat numbering convention. In some
embodiments that may be combined with any of the preceding embodiments, the amino
acid substitution at position 248 according to Kabat numbering convention inhibits the Fc
region from interacting with an Fc receptor. In some embodiments that may be combined
with any of the preceding embodiments, the Fc region comprises an amino acid substitution
at position 241 according to Kabat numbering convention. In some embodiments that may
be combined with any of the preceding embodiments, the Fc region comprises a serine to
proline amino acid substitution at position 241 according to Kabat numbering convention.
In some embodiments that may be combined with any of the preceding embodiments, the
amino acid substitution at position 241 according to Kabat numbering convention prevents
arm switching in the antibody.  In some embodiments that may be combined with any of
the preceding embodiments, the antibody comprises, the Fc region comprises the amino
acid sequence of SEQ ID NO: 37, or an amino acid sequence with at least about 70%, at
least about 75%, at least about 80% at least about 85% at least about 90%, or at least about
90% homology to the amino acid sequence of SEQ ID NO: 37. In some embodiments that
may be combined with any of the preceding embodiments, the antibody comprises a heavy
chain variable domain and a light chain variable domain, wherein the heavy chain variable
domain comprises an amino acid sequence selected from SEQ ID NOs: 1-4, or an amino
acid sequence with at least about 90% homology to the amino acid sequence selected from
SEQ ID NOs: 1-4. In some embodiments that may be combined with any of the preceding
embodiments, the light chain variable domain comprises an amino acid sequence selected
from SEQ ID NOs: 5-8, or an amino acid sequence with at least about 90% homology to
the amino acid sequence selected from SEQ ID NOs: 5-8.
In certain aspects, the present disclosure provides a humanized anti-C1q
antibody, or an antigen-binding fragment thereof, the antibody comprising a heavy chain

variable domain and a light chain variable domain, wherein the heavy chain variable
domain comprises an amino acid sequence selected from SEQ ID NOs: 1-4, or an amino
acid sequence with at least about 90% homology to the amino acid sequence selected from
SEQ ID NOs: 1-4.
In certain aspects, the present disclosure provides a humanized anti-C1q
antibody, or an antigen-binding fragment thereof, the antibody comprising a heavy chain
variable domain and a light chain variable domain, wherein the light chain variable domain
comprises an amino acid sequence selected from SEQ ID NOs: 5-8, or an amino acid
sequence with at least about 90% homology to the amino acid sequence selected from SEQ
ID NOs: 5-8.
In certain aspects, the present disclosure provides a humanized anti-C1q
antibody, or an antigen-binding fragment thereof, the antibody comprising: a) a heavy chain
variable domain comprising an amino acid sequence selected from SEQ ID NOs: 1-4, or an
amino acid sequence with at least about 90% homology to the amino acid sequence selected
from SEQ ID NOs: 1-4; and/or b) a light chain variable domain comprising an amino acid
sequence selected from SEQ ID NOs: 5-8, or an amino acid sequence with at least about
90% homology to the amino acid sequence selected from SEQ ID NOs: 5-8.
In certain aspects, the present disclosure provides a humanized anti-C1q
antibody, or an antigen-binding fragment thereof, the antibody comprising: a) a heavy chain
variable domain comprising the amino acid sequence of SEQ ID NO: 1, or an amino acid
sequence with at least about 90% homology to the amino acid sequence of SEQ ID NO: 1;
and b) a light chain variable domain comprising an amino acid sequence selected from SEQ
ID NOs: 5-8, or an amino acid sequence with at least about 90% homology to the amino
acid sequence selected from SEQ ID NOs: 5-8. In certain aspects, the present disclosure
provides a humanized anti-C1q antibody, or an antigen-binding fragment thereof, the
antibody comprising: a) a heavy chain variable domain comprising the amino acid sequence
of SEQ ID NO: 2, or an amino acid sequence with at least about 90% homology to the
amino acid sequence of SEQ ID NO: 2; and b) a light chain variable domain comprising an
amino acid sequence selected from SEQ ID NOs: 5-8, or an amino acid sequence with at
least about 90% homology to the amino acid sequence selected from SEQ ID NOs: 5-8. In
certain aspects, the present disclosure provides a humanized anti-C1q antibody, or an
antigen-binding fragment thereof, the antibody comprising: a) a heavy chain variable
domain comprising the amino acid sequence of SEQ ID NO: 3, or an amino acid sequence

with at least about 90% homology to the amino acid sequence of SEQ ID NO: 3; and b) a
light chain variable domain comprising an amino acid sequence selected from SEQ ID
NOs: 5-8, or an amino acid sequence with at least about 90% homology to the amino acid
sequence selected from SEQ ID NOs: 5-8. In certain aspects, the present disclosure
provides a humanized anti-C1q antibody, or an antigen-binding fragment thereof, the
antibody comprising: a) a heavy chain variable domain comprising the amino acid sequence
of SEQ ID NO: 4, or an amino acid sequence with at least about 90% homology to the
amino acid sequence of SEQ ID NO: 4; and b) a light chain variable domain comprising an
amino acid sequence selected from SEQ ID NOs: 5-8, or an amino acid sequence with at
least about 90% homology to the amino acid sequence selected from SEQ ID NOs: 5-8.
In certain aspects, the present disclosure provides a humanized anti-C1q
antibody, or an antigen-binding fragment thereof, the antibody comprising: a) a heavy chain
variable domain comprising an amino acid sequence selected from SEQ ID NOs: 1-4, or an
amino acid sequence with at least about 90% homology to the amino acid sequence selected
from SEQ ID NOs: 1-4; and b) a light chain variable domain comprising the amino acid
sequence of SEQ ID NO: 5, or an amino acid sequence with at least about 90% homology
to the amino acid sequence of SEQ ID NO: 5. In certain aspects, the present disclosure
provides a humanized anti-C1q antibody, or an antigen-binding fragment thereof, the
antibody comprising: a) a heavy chain variable domain comprising an amino acid sequence
selected from SEQ ID NOs: 1-4, or an amino acid sequence with at least about 90%
homology to the amino acid sequence selected from SEQ ID NOs: 1-4; and b) a light chain
variable domain comprising the amino acid sequence of SEQ ID NO: 6, or an amino acid
sequence with at least about 90% homology to the amino acid sequence of SEQ ID NO: 6.
In certain aspects, the present disclosure provides a humanized anti-C1q antibody, or an
antigen-binding fragment thereof, the antibody comprising: a) a heavy chain variable
domain comprising an amino acid sequence selected from SEQ ID NOs: 1-4, or an amino
acid sequence with at least about 90% homology to the amino acid sequence selected from
SEQ ID NOs: 1-4; and b) a light chain variable domain comprising the amino acid
sequence of SEQ ID NO: 7, or an amino acid sequence with at least about 90% homology
to the amino acid sequence of SEQ ID NO: 7. In certain aspects, the present disclosure
provides a humanized anti-C1q antibody, or an antigen-binding fragment thereof, the
antibody comprising: a) a heavy chain variable domain comprising an amino acid sequence
selected from SEQ ID NOs: 1-4, or an amino acid sequence with at least about 90%

homology to the amino acid sequence selected from SEQ ID NOs: 1-4; and b) a light chain
variable domain comprising the amino acid sequence of SEQ ID NO: 8, or an amino acid
sequence with at least about 90% homology to the amino acid sequence of SEQ ID NO: 8.
In certain aspects, the present disclosure provides a humanized anti-C1q
antibody, or an antigen-binding fragment thereof, the antibody comprising: a) a heavy chain
variable domain comprising the amino acid sequence of SEQ ID NO: 1, or an amino acid
sequence with at least about 90% homology to the amino acid sequence of SEQ ID NO: 1;
and b) a light chain variable domain comprising the amino acid sequence of SEQ ID NO: 5,
or an amino acid sequence with at least about 90% homology to the amino acid sequence of
SEQ ID NO: 5. In certain aspects, the present disclosure provides a humanized anti-C1q
antibody, or an antigen-binding fragment thereof, the antibody comprising: a) a heavy chain
variable domain comprising the amino acid sequence of SEQ ID NO: 1, or an amino acid
sequence with at least about 90% homology to the amino acid sequence of SEQ ID NO: 1;
and b) a light chain variable domain comprising the amino acid sequence of SEQ ID NO: 6,
or an amino acid sequence with at least about 90% homology to the amino acid sequence of
SEQ ID NO: 6. In certain aspects, the present disclosure provides a humanized anti-C1q
antibody, or an antigen-binding fragment thereof, the antibody comprising: a) a heavy chain
variable domain comprising the amino acid sequence of SEQ ID NO: 1, or an amino acid
sequence with at least about 90% homology to the amino acid sequence of SEQ ID NO: 1;
and b) a light chain variable domain comprising the amino acid sequence of SEQ ID NO: 7,
or an amino acid sequence with at least about 90% homology to the amino acid sequence of
SEQ ID NO: 7. In certain aspects, the present disclosure provides a humanized anti-C1q
antibody, or an antigen-binding fragment thereof, the antibody comprising: a) a heavy chain
variable domain comprising the amino acid sequence of SEQ ID NO: 1, or an amino acid
sequence with at least about 90% homology to the amino acid sequence of SEQ ID NO: 1;
and b) a light chain variable domain comprising the amino acid sequence of SEQ ID NO: 8,
or an amino acid sequence with at least about 90% homology to the amino acid sequence of
SEQ ID NO: 8.
In certain aspects, the present disclosure provides a humanized anti-C1q
antibody, or an antigen-binding fragment thereof, the antibody comprising: a) a heavy chain
variable domain comprising the amino acid sequence of SEQ ID NO: 2, or an amino acid
sequence with at least about 90% homology to the amino acid sequence of SEQ ID NO: 2;
and b) a light chain variable domain comprising the amino acid sequence of SEQ ID NO: 5,

or an amino acid sequence with at least about 90% homology to the amino acid sequence of
SEQ ID NO: 5. In certain aspects, the present disclosure provides a humanized anti-C1q
antibody, or an antigen-binding fragment thereof, the antibody comprising: a) a heavy chain
variable domain comprising the amino acid sequence of SEQ ID NO: 2, or an amino acid
sequence with at least about 90% homology to the amino acid sequence of SEQ ID NO: 2;
and b) a light chain variable domain comprising the amino acid sequence of SEQ ID NO: 6,
or an amino acid sequence with at least about 90% homology to the amino acid sequence of
SEQ ID NO: 6. In certain aspects, the present disclosure provides a humanized anti-C1q
antibody, or an antigen-binding fragment thereof, the antibody comprising: a) a heavy chain
variable domain comprising the amino acid sequence of SEQ ID NO: 2, or an amino acid
sequence with at least about 90% homology to the amino acid sequence of SEQ ID NO: 2;
and b) a light chain variable domain comprising the amino acid sequence of SEQ ID NO: 7,
or an amino acid sequence with at least about 90% homology to the amino acid sequence of
SEQ ID NO: 7. In certain aspects, the present disclosure provides a humanized anti-C1q
antibody, or an antigen-binding fragment thereof, the antibody comprising: a) a heavy chain
variable domain comprising the amino acid sequence of SEQ ID NO: 2, or an amino acid
sequence with at least about 90% homology to the amino acid sequence of SEQ ID NO: 2;
and b) a light chain variable domain comprising the amino acid sequence of SEQ ID NO: 8,
or an amino acid sequence with at least about 90% homology to the amino acid sequence of
SEQ ID NO: 8.
In certain aspects, the present disclosure provides a humanized anti-C1q
antibody, or an antigen-binding fragment thereof, the antibody comprising: a) a heavy chain
variable domain comprising the amino acid sequence of SEQ ID NO: 3, or an amino acid
sequence with at least about 90% homology to the amino acid sequence of SEQ ID NO: 3;
and b) a light chain variable domain comprising the amino acid sequence of SEQ ID NO: 5,
or an amino acid sequence with at least about 90% homology to the amino acid sequence of
SEQ ID NO: 5. In certain aspects, the present disclosure provides a humanized anti-C1q
antibody, or an antigen-binding fragment thereof, the antibody comprising: a) a heavy chain
variable domain comprising the amino acid sequence of SEQ ID NO: 3, or an amino acid
sequence with at least about 90% homology to the amino acid sequence of SEQ ID NO: 3;
and b) a light chain variable domain comprising the amino acid sequence of SEQ ID NO: 6,
or an amino acid sequence with at least about 90% homology to the amino acid sequence of
SEQ ID NO: 6. In certain aspects, the present disclosure provides a humanized anti-C1q

antibody, or an antigen-binding fragment thereof, the antibody comprising: a) a heavy chain
variable domain comprising the amino acid sequence of SEQ ID NO: 3, or an amino acid
sequence with at least about 90% homology to the amino acid sequence of SEQ ID NO: 3;
and b) a light chain variable domain comprising the amino acid sequence of SEQ ID NO: 7,
or an amino acid sequence with at least about 90% homology to the amino acid sequence of
SEQ ID NO: 7. In certain aspects, the present disclosure provides a humanized anti-C1q
antibody, or an antigen-binding fragment thereof, the antibody comprising: a) a heavy chain
variable domain comprising the amino acid sequence of SEQ ID NO: 3, or an amino acid
sequence with at least about 90% homology to the amino acid sequence of SEQ ID NO: 3;
and b) a light chain variable domain comprising the amino acid sequence of SEQ ID NO: 8,
or an amino acid sequence with at least about 90% homology to the amino acid sequence of
SEQ ID NO: 8.
In certain aspects, the present disclosure provides a humanized anti-C1q
antibody, or an antigen-binding fragment thereof, the antibody comprising: a) a heavy chain
variable domain comprising the amino acid sequence of SEQ ID NO: 4, or an amino acid
sequence with at least about 90% homology to the amino acid sequence of SEQ ID NO: 4;
and b) a light chain variable domain comprising the amino acid sequence of SEQ ID NO: 5,
or an amino acid sequence with at least about 90% homology to the amino acid sequence of
SEQ ID NO: 5. In certain aspects, the present disclosure provides a humanized anti-C1q
antibody, or an antigen-binding fragment thereof, the antibody comprising: a) a heavy chain
variable domain comprising the amino acid sequence of SEQ ID NO: 4, or an amino acid
sequence with at least about 90% homology to the amino acid sequence of SEQ ID NO: 4;
and b) a light chain variable domain comprising the amino acid sequence of SEQ ID NO: 6,
or an amino acid sequence with at least about 90% homology to the amino acid sequence of
SEQ ID NO: 6. In certain aspects, the present disclosure provides a humanized anti-C1q
antibody, or an antigen-binding fragment thereof, the antibody comprising: a) a heavy chain
variable domain comprising the amino acid sequence of SEQ ID NO: 4, or an amino acid
sequence with at least about 90% homology to the amino acid sequence of SEQ ID NO: 4;
and b) a light chain variable domain comprising the amino acid sequence of SEQ ID NO: 7,
or an amino acid sequence with at least about 90% homology to the amino acid sequence of
SEQ ID NO: 7. In certain aspects, the present disclosure provides a humanized anti-C1q
antibody, or an antigen-binding fragment thereof, the antibody comprising: a) a heavy chain
variable domain comprising the amino acid sequence of SEQ ID NO: 4, or an amino acid

sequence with at least about 90% homology to the amino acid sequence of SEQ ID NO: 4;
and b) a light chain variable domain comprising the amino acid sequence of SEQ ID NO: 8,
or an amino acid sequence with at least about 90% homology to the amino acid sequence of
SEQ ID NO: 8.
In some embodiments that may be combined with any of the preceding
embodiments, the antibody binds specifically to both human C1q and mouse C1q. In some
embodiments that may be combined with any of the preceding embodiments, the antibody
binds specifically to rat C1q. In some embodiments that may be combined with any of the
preceding embodiments, the antibody binds specifically to human C1q, mouse C1q, and rat
C1q. In some embodiments that may be combined with any of the preceding embodiments,
the antibody or antigen-binding fragment thereof binds essentially the same C1q epitope as
the antibody M1 produced by the hybridoma cell line with ATCC Accession Number PTA-
120399 or anti-C1q binding fragments thereof. In some embodiments that may be
combined with any of the preceding embodiments, the antibody or antigen-binding
fragment thereof inhibits the binding of the monoclonal antibody M1 produced by a
hybridoma cell line with ATCC Accession Number PTA-120399 to human C1q or to
mouse C1q. In some embodiments that may be combined with any of the preceding
embodiments, the antibody is of the IgG class. In some embodiments that may be combined
with any of the preceding embodiments, the antibody has an IgG1, IgG2, IgG3, or IgG4
isotype. In some embodiments that may be combined with any of the preceding
embodiments, the antibody has an IgG4 isotype. In some embodiments that may be
combined with any of the preceding embodiments, the antibody comprises a human IgG4
constant region. In some embodiments that may be combined with any of the preceding
embodiments, the human IgG4 heavy chain constant region comprises the amino acid
sequence of SEQ ID NO: 37, or an amino acid sequence with at least about 90% homology
to the amino acid sequence of SEQ ID NO: 37. In some embodiments that may be
combined with any of the preceding embodiments, the human IgG4 constant region
comprises an Fc region. In some embodiments that may be combined with any of the
preceding embodiments, the Fc region is incapable of binding the C1q protein. In some
embodiments that may be combined with any of the preceding embodiments, the Fc region
is incapable of inducing complement activity. In some embodiments that may be combined
with any of the preceding embodiments, the Fc region is incapable of inducing antibody-
dependent cellular cytotoxicity (ADCC). In some embodiments that may be combined with

any of the preceding embodiments, the Fc region comprises one or more modifications. In
some embodiments that may be combined with any of the preceding embodiments, the Fc
region comprises one or more amino acid substitutions. In some embodiments that may be
combined with any of the preceding embodiments, the Fc region comprises an amino acid
substitution at position 248 according to Kabat numbering convention. In some
embodiments that may be combined with any of the preceding embodiments, the Fc region
comprises a leucine to glutamate amino acid substitution at position 248 according to Kabat
numbering convention. In some embodiments that may be combined with any of the
preceding embodiments, the amino acid substitution at position 248 according to Kabat
numbering convention inhibits the Fc region from interacting with an Fc receptor. In some
embodiments that may be combined with any of the preceding embodiments, the Fc region
comprises an amino acid substitution at position 241 according to Kabat numbering
convention. In some embodiments that may be combined with any of the preceding
embodiments, the Fc region comprises a serine to proline amino acid substitution at
position 241 according to Kabat numbering convention. In some embodiments that may be
combined with any of the preceding embodiments, the amino acid substitution at position
241 according to Kabat numbering convention prevents arm switching in the antibody. In
some embodiments that may be combined with any of the preceding embodiments, the
antibody is a bispecific antibody. In some embodiments that may be combined with any of
the preceding embodiments, the antibody has been engineered to increase brain penetration.
In some embodiments that may be combined with any of the preceding embodiments, the
antibody is a bispecific antibody recognizing a first antigen and a second antigen. In some
embodiments that may be combined with any of the preceding embodiments, the first
antigen is a C1q protein and the second antigen is an antigen facilitating transport across
the blood-brain-barrier. In some embodiments that may be combined with any of the
preceding embodiments, the second antigen is selected from transferrin receptor (TR),
insulin receptor (HIR), insulin-like growth factor receptor (IGFR), low-density lipoprotein
receptor related proteins 1 and 2 (LPR-1 and 2), diphtheria toxin receptor, CRM197, a
llama single domain antibody, TMEM 30(A), a protein transduction domain, TAT, Syn-B,
penetratin, a poly-arginine peptide, an angiopep peptide, and ANG1005. In some
embodiments that may be combined with any of the preceding embodiments, the antigen-
binding fragment is a Fab, F(ab')2 or Fab' fragment. In some embodiments that may be
combined with any of the preceding embodiments, the antibody fragment has better brain

penetration as compared to its corresponding full-length antibody. In some embodiments
that may be combined with any of the preceding embodiments, the antibody fragment has a
shorter half-life as compared to its corresponding full-length antibody. In some
embodiments that may be combined with any of the preceding embodiments, the antibody
has a dissociation constant (K_{D}) for human C1q that ranges from less than about 10 pM to
less than about 5 pM. In some embodiments that may be combined with any of the
preceding embodiments, the antibody has dissociation constant (K_{D}) for mouse C1q that
ranges from less than about 125 nM to less than about 5 pM. In some embodiments that
may be combined with any of the preceding embodiments, the antibody specifically binds
to and neutralizes a biological activity of C1q. In some embodiments that may be combined
with any of the preceding embodiments, the biological activity is (1) C1q binding to an
autoantibody, (2) C1q binding to C1r, (3) C1q binding to C1s, (4) C1q binding to
phosphatidylserine, (5) C1q binding to pentraxin-3, (6) C1q binding to C-reactive protein
(CRP), (7) C1q binding to globular C1q receptor (gC1qR), (8) C1q binding to complement
receptor 1 (CR1), (9) C1q binding to beta-amyloid, or (10) C1q binding to calreticulin. In
some embodiments that may be combined with any of the preceding embodiments, the
biological activity is (1) activation of the classical complement activation pathway, (2)
activation of antibody and complement dependent cytotoxicity, (3) CH50 hemolysis, (4)
synapse loss, (5) B-cell antibody production, (6) dendritic cell maturation, (7) T-cell
proliferation, (8) cytokine production (9) microglia activation, (10) Arthus reaction, (11)
phagocytosis of synapses or nerve endings, or (12) activation of complement receptor 3
(CR3/C3) expressing cells. In some embodiments that may be combined with any of the
preceding embodiments, CH50 hemolysis comprises human, mouse, and/or rat CH50
hemolysis. In some embodiments that may be combined with any of the preceding
embodiments, the antibody is capable of neutralizing from at least about 50%, to at least
about 95% of CH50 hemolysis. In some embodiments that may be combined with any of
the preceding embodiments, the antibody is capable of neutralizing at least 50% of CH50
hemolysis at a dose of less than 150 ng, less than 100 ng, less than 50 ng, or less than 20
ng. In some embodiments that may be combined with any of the preceding embodiments,
the antibody binds C1q with a binding stoichiometry that ranges from 20:1 to 1.0:1 or less
than 1.0:1.
In certain aspects, the present disclosure provides an isolated polynucleotide
comprising a nucleic acid sequence encoding a humanized anti-C1q antibody of any of the

preceding embodiments. In certain aspects, the present disclosure provides an isolated host
cell comprising a nucleic acid sequence of any of the preceding embodiments. In certain
aspects, the present disclosure provides a pharmaceutical composition comprising a
humanized anti-C1q antibody of any of the preceding embodiments and a pharmaceutically
acceptable carrier.
In certain aspects, the present disclosure provides a method of treating or
preventing a disease associated with complement activation in an individual in need of such
treatment, the method comprising the step of administering a therapeutically effective dose
of a humanized anti-C1q antibody of any of the preceding embodiments. In other aspects,
the present disclosure provides a humanized anti-C1q antibody of any of the preceding
embodiments for use in treating or preventing a disease associated with complement
activation in an individual in need of such treatment. In other aspects, the present
disclosure provides use of a humanized anti-C1q antibody of any of the preceding
embodiments in the manufacture of a medicament for treating or preventing a disease
associated with complement activation in an individual in need of such treatment.
In some embodiments that may be combined with any of the preceding
embodiments, the disease associated with complement activation is a neurodegenerative
disorder. In some embodiments that may be combined with any of the preceding
embodiments, the neurodegenerative disorder is associated with the loss of synapses or
nerve connections. In some embodiments that may be combined with any of the preceding
embodiments, the neurodegenerative disorder is associated with synapse loss that is
dependent on the complement receptor 3(CR3)/C3 or complement receptor CR1. In some
embodiments that may be combined with any of the preceding embodiments, the
neurodegenerative disorder is associated with pathological activity-dependent synaptic
pruning. In some embodiments that may be combined with any of the preceding
embodiments, the neurodegenerative disorder is associated with synapse phagocytosis by
microglia. In some embodiments that may be combined with any of the preceding
embodiments, the neurodegenerative disorder is selected from Alzheimer's disease,
amyotrophic lateral sclerosis, multiple sclerosis, glaucoma, myotonic dystrophy, Guillain-
Barre' syndrome (GBS), Myastenia Gravis, Bullous Pemphigoid, spinal muscular atrophy,
Down syndrome, Parkinson's disease, and Huntington's disease. In some embodiments
that may be combined with any of the preceding embodiments, the disease associated with
complement activation is an inflammatory disease, an autoimmune disease, or metabolic

disorder. In some embodiments that may be combined with any of the preceding
embodiments, the inflammatory disease, autoimmune disease, or metabolic disorder is
selected from diabetes, obesity, rheumatoid arthritis (RA), acute respiratory distress
syndrome (ARDS), remote tissue injury after ischemia and reperfusion, complement
activation during cardiopulmonary bypass surgery, dermatomyositis, pemphigus, lupus
nephritis and resultant glomerulonephritis and vasculitis, cardiopulmonary bypass,
cardioplegia-induced coronary endothelial dysfunction, type II membranoproliferative
glomerulonephritis, IgA nephropathy, acute renal failure, cryoglobulemia, antiphospholipid
syndrome, Chronic open-angle glaucoma, acute closed angle glaucoma, macular
degenerative diseases, age-related macular degeneration (AMD), (AMD-wet), Geographic
atrophy choroidal neovascularization (CNV), uveitis, diabetic retinopathy, ischemia-related
retinopathy, endophthalmitis, intraocular neovascular disease, diabetic macular edema,
pathological myopia, von Hippel-Lindau disease, histoplasmosis of the eye, Neuromyelitis
Optica (NMO), Central Retinal Vein Occlusion (CRVO), corneal neovascularization,
retinal neovascularization, Leber's hereditary optic neuropathy, optic neuritis, Behcet's
retinopathy, ischemic optic neuropathy, retinal vasculitis, ANCA vasculitis, Purtscher
retinopathy, Sjogren's dry eye disease, dry AMD, sarcoidosis, temporal arteritis,
polyarteritis nodosa, multiple sclerosis, allo-transplantation, hyperacute rejection,
hemodialysis, chronic occlusive pulmonary distress syndrome (COPD), asthma, and
aspiration pneumonia. In some embodiments that may be combined with any of the
preceding embodiments, the disease associated with complement activation is an
autoimmune disease selected from myasthenia gravis, Diabetes mellitus type 1,
Hashimoto's thyroiditis, Addison's disease, Coeliac disease, Crohn's disease, pernicious
anaemia, Pemphigus vulgaris, vitiligo, autoimmune hemolytic anemias, paraneoplastic
syndromes, a vasculitis disease, hypocomplementemic urticarial vasculitis (HUV),
polymyalgia rheumatica, temporal arteritis, and Wegener's granulomatosis.
In certain aspects, the present disclosure provides a kit comprising a humanized
anti-C1q antibody of any of the preceding embodiments, and a package insert comprising
instructions for using the antibody to treat or prevent a disease associated with complement
activation in an individual in need of such treatment. In some embodiments, the disease
associated with complement activation is a neurodegenerative disorder. In some
embodiments, the neurodegenerative disorder is associated with loss of synapses or loss
nerve connections. In some embodiments, the neurodegenerative disorder is associated

with synapse loss that is dependent on the complement receptor 3(CR3)/C3 or complement
receptor CR1. In some embodiments, the neurodegenerative disorder is associated with
pathological activity-dependent synaptic pruning. In some embodiments, the
neurodegenerative disorder is associated with synapse phagocytosis by microglia. In some
embodiments, the neurodegenerative disorder is selected from Alzheimer's disease,
amyotrophic lateral sclerosis, multiple sclerosis, glaucoma, myotonic dystrophy, Down
syndrome, Parkinson's disease, and Huntington's disease. In some embodiments, the
disease associated with complement activation is an inflammatory disease, autoimmune
disease, or metabolic disorder. In some embodiments, the inflammatory disease,
autoimmune disease, or metabolic disorder is selected from diabetes, obesity, rheumatoid
arthritis (RA), acute respiratory distress syndrome (ARDS), remote tissue injury after
ischemia and reperfusion, complement activation during cardiopulmonary bypass surgery,
dermatomyositis, pemphigus, lupus nephritis and resultant glomerulonephritis and
vasculitis, cardiopulmonary bypass, cardioplegia-induced coronary endothelial dysfunction,
type II membranoproliferative glomerulonephritis, IgA nephropathy, acute renal failure,
cryoglobulemia, antiphospholipid syndrome, Chronic open-angle glaucoma, acute closed
angle glaucoma, macular degenerative diseases, age-related macular degeneration (AMD),
Geographic atrophy, choroidal neovascularization (CNV), uveitis, diabetic retinopathy,
ischemia-related retinopathy, endophthalmitis, intraocular neovascular disease, diabetic
macular edema, pathological myopia, von Hippel-Lindau disease, histoplasmosis of the
eye, Neuromyelitis Optica (NMO), Central Retinal Vein Occlusion (CRVO), corneal
neovascularization, retinal neovascularization, Leber's hereditary optic neuropathy, optic
neuritis, Behcet's retinopathy, ischemic optic neuropathy, retinal vasculitis, ANCA
vasculitis, Purtscher retinopathy, Sjogren's dry eye disease, dry AMD, sarcoidosis,
temporal arteritis, polyarteritis nodosa, multiple sclerosis, allo-transplantation, hyperacute
rejection, hemodialysis, chronic occlusive pulmonary distress syndrome (COPD), asthma,
and aspiration pneumonia. In some embodiments, the disease associated with complement
activation is an autoimmune disease selected from myasthenia gravis, Diabetes mellitus
type 1, Hashimoto's thyroiditis, Addison's disease, Coeliac disease, Crohn's disease,
pernicious anaemia, Pemphigus vulgaris, vitiligo, autoimmune hemolytic anemias,
paraneoplastic syndromes, a vasculitis disease, hypocomplementemic urticarial vasculitis
(HUV), polymyalgia rheumatica, temporal arteritis, and Wegener's granulomatosis.

In certain aspects, the present disclosure provides a kit comprising a humanized
anti-C1q antibody of any of the preceding embodiments. In some embodiments, the kit is
for diagnostic or therapeutic uses as disclosed herein.
In certain aspects, the present disclosure provides a method of detecting
synapses in an individual, by a) administering a humanized anti-C1q antibody of any of the
preceding embodiments to the individual, and b) detecting antibody bound to synapses,
thereby detecting synapses in the individual. In other aspects, the present disclosure
provides a humanized anti-C1q antibody of any of the preceding embodiments for use in
detecting synapses in an individual. In other aspects, the present disclosure provides use of
a humanized anti-C1q antibody of any of the preceding embodiments in the manufacture of
a medicament for detecting synapses in an individual. In some embodiments that may be
combined with any of the preceding embodiments, the antibody bound to synapses is
detected using imaging techniques selected from positron emission tomography (PET), X-
ray computed tomography, single-photon emission computed tomography (SPECT),
computed tomography (CT), and computed axial tomography (CAT). In some
embodiments that may be combined with any of the preceding embodiments, the detection
of antibody bound to synapses provides a quantitative measure of the number of synapses
in the individual. In some embodiments that may be combined with any of the preceding
embodiments, the individual has a neurodegenerative disease or autoimmune disease. In
some embodiments that may be combined with any of the preceding embodiments, the
number of synapses in the individual is measured repeatedly over a period of time and a
loss of synapses in the individual is detected over time. In some embodiments that may be
combined with any of the preceding embodiments, the loss of synapses over time is a
measure for the efficacy of a treatment for the neurodegenerative disease or autoimmune
disease.
In certain aspects, the present disclosure provides a method of detecting
synapses in a biological sample, by a) contacting the biological sample with a humanized
anti-C1q antibody of any of the preceding embodiments, and b) detecting antibody bound
to synapses, thereby detecting synapses in the individual.
In some embodiments that may be combined with any of the preceding
embodiments, the method further comprises a step before step a) of obtaining the biological
sample from an individual. In some embodiments that may be combined with any of the
preceding embodiments, the biological sample comprises a biopsy specimen, a tissue, or a

cell. In some embodiments that may be combined with any of the preceding embodiments,
the antibody is detected by immunofluorescence microscopy, immunocytochemistry,
immunohistochemistry, ELISA, FACS analysis, immunoprecipitation, or micro-positron
emission tomography.
It is to be understood that one, some, or all of the properties of the various
embodiments described herein may be combined to form other embodiments of the
compositions and methods provided herein. These and other aspects of the compositions
and methods provided herein will become apparent to one of skill in the art.

DESCRIPTION OF THE FIGURES

FIG. 1 depicts plasmid maps for light chain and heavy chain expression vectors.
FIG. 1A depicts a plasmid map for light chain expression vector pANTVk. FIG. 1B
depicts a plasmid map for and heavy chain expression vector pANTVhG4 (S241P L248E).
Both VH and Vκ vectors contain genomic DNA fragments incorporating introns and polyA
sequences. Expression of both chains is driven by a CMV promoter and selection (on the
heavy chain vector) is via a DHFR mini gene.
FIG. 2A depicts an alignment of amino acid sequence of the heavy chain
variable region (VH) of the M1 antibody and the amino acid sequences of the humanized
VH variants VH1-VH2. FIG. 2B depicts an alignment of amino acid sequence of the
heavy chain variable region (VH) of the M1 antibody and the amino acid sequences of the
humanized VH variants VH3-VH4. FIG. 2C depicts an alignment of amino acid sequence
of the kappa light chain variable region (Vκ) of the M1 antibody and the amino acid
sequences of the humanized Vκ variants Vκ1-Vκ2. FIG. 2D depicts an alignment of amino
acid sequence of the kappa light chain variable region (Vκ) of the M1 antibody and the
amino acid sequences of the humanized Vκ variants Vκ3-Vκ4.
FIG. 3 depicts a Coomassie Blue-stained SDS-PAGE gel of protein A-purified
antibodies. 2μg of each sample was loaded on a NuPage 4-12% Bis-Tris gel and run at
200V for 35min. Size marker is pre-stained protein standard Fermentas PageRuler Plus.
FIG. 4 depicts competition ELISA assays for human C1q. A dilution series of
purified humanized anti-C1q antibodies were competed against a fixed concentration of
biotinylated monoclonal antibody M1 for binding to human C1q. Bound biotinylated M1
antibody was detected using streptavidin-peroxidase conjugate and TMB substrate. FIG.
4A depicts the results with the humanized antibodies VH1/Vκ1, VH1/Vκ2, and VH1/Vκ3.

FIG. 4B depicts the results with the humanized antibodies VH1/Vκ4, VH2/Vκ1, VH2/Vκ2,
VH2/Vκ3, and VH2/Vκ4. FIG. 4C depicts the results with the humanized
antibodiesVH3/Vκ1, VH3/Vκ2, VH3/Vκ3, and VH3/Vκ4. FIG. 4D depicts the results with
the humanized antibodies VH4/Vκ1, VH4/Vκ2, VH4/Vκ3, and VH4/Vκ4.
FIG. 5 depicts competition ELISA assays for mouse C1q. A dilution series of
purified humanized anti-C1q antibodies were competed against a fixed concentration of the
chimeric M1 antibody for binding to mouse C1q. Bound biotinylated chimeric M1 was
detected using streptavidin-peroxidase conjugate and TMB substrate.
FIG. 6 depicts a Coomassie Blue-stained SDS-PAGE gel of gel filtration-
purified antibodies. 1 μg of each sample was loaded on a NuPage 4-12% Bis-Tris gel and
run at 200V for 35min. Size marker (M) is pre-stained protein standard Fermentas
PageRuler Plus. Lane 1 depicts Fab VH3/Vκ3 reduced; lane 2 depicts Fab VH3/Vκ3 non-
reduced; lane 3 depicts IgG V VH3/Vκ3 reduced; and lane 4 depicts IgG VH3/Vκ3 non-
reduced.
FIG. 7 illustrates the C1q-neutralizing activities of anti-C1q antibodies in
human, and rat CH50 hemolytic assays in a dose-response format. FIG. 7A illustrates
results from a human CH50 hemolytic assay. FIG. 7B illustrates results from a rat CH50
hemolytic assay. "ANN-005" corresponds to the monoclonal antibody M1, "3E2"
corresponds to a chimeric M1 antibody, "2B12" corresponds to antibody VH1/Vκ1, "5H7"
corresponds to antibody VH3/Vκ3, "3F1" corresponds to antibody VH3/Vκ4, and "1D3"
corresponds to antibody VH4/Vκ3.
FIG. 8 depicts the time course of serum 5H7 levels in monkeys for single IV
dose at 15 and 100 mg/Kg.
FIG. 9 illustrates the time course of serum C1q levels in monkeys for single IV
dose at 15 and 100 mg/Kg. FIG 9A depicts the time course of serum C1q levels in
monkeys using the JL1-M1 assay. FIG. 9B depicts the time course of serum C1q levels in
monkeys using the JL1-JL1 assay.
FIG 10 shows sustained reduction of serum hemolysis in monkeys for single IV
dose at 15 and 100 mg/Kg.

DETAILED DESCRIPTION
General Techniques
The techniques and procedures described or referenced herein are generally well
understood and commonly employed using conventional methodology by those skilled in
the art, such as, for example, the widely utilized methodologies described in Sambrook et
al., Molecular Cloning: A Laboratory Manual 3d edition (2001) Cold Spring Harbor
Laboratory Press, Cold Spring Harbor, N.Y.; Current Protocols in Molecular Biology
(F.M. Ausubel, et al. eds., (2003)); the series Methods in Enzymology (Academic Press,
Inc.): PCR 2: A Practical Approach (M.J. MacPherson, B.D. Hames and G.R. Taylor eds.
(1995)), Harlow and Lane, eds. (1988) Antibodies, A Laboratory Manual, and Animal Cell
Culture (R.I. Freshney, ed. (1987)); Oligonucleotide Synthesis (M.J. Gait, ed., 1984);
Methods in Molecular Biology, Humana Press; Cell Biology: A Laboratory Notebook (J.E.
Cellis, ed., 1998) Academic Press; Animal Cell Culture (R.I. Freshney), ed., 1987);
Introduction to Cell and Tissue Culture (J.P. Mather and P.E. Roberts, 1998) Plenum Press;
Cell and Tissue Culture: Laboratory Procedures (A. Doyle, J.B. Griffiths, and D.G.
Newell, eds., 1993-8) J. Wiley and Sons; Handbook of Experimental Immunology (D.M.
Weir and C.C. Blackwell, eds.); Gene Transfer Vectors for Mammalian Cells (J.M. Miller
and M.P. Calos, eds., 1987); PCR: The Polymerase Chain Reaction, (Mullis et al., eds.,
1994); Current Protocols in Immunology (J.E. Coligan et al., eds., 1991); Short Protocols
in Molecular Biology (Wiley and Sons, 1999); Immunobiology (C.A. Janeway and P.
Travers, 1997); Antibodies (P. Finch, 1997); Antibodies: A Practical Approach (D. Catty.,
ed., IRL Press, 1988-1989); Monoclonal Antibodies: A Practical Approach (P. Shepherd
and C. Dean, eds., Oxford University Press, 2000); Using Antibodies: A Laboratory
Manual (E. Harlow and D. Lane (Cold Spring Harbor Laboratory Press, 1999); The
Antibodies (M. Zanetti and J. D. Capra, eds., Harwood Academic Publishers, 1995); and
Cancer: Principles and Practice of Oncology (V.T. DeVita et al., eds., J.B. Lippincott
Company, 1993).
Definitions
As used herein, the term "preventing" includes providing prophylaxis with
respect to occurrence or recurrence of a particular disease, disorder, or condition in an
individual. An individual may be predisposed to, susceptible to a particular disease,
disorder, or condition, or at risk of developing such a disease, disorder, or condition, but
has not yet been diagnosed with the disease, disorder, or condition.

As used herein, an individual "*at risk*" of developing a particular disease,
disorder, or condition may or may not have detectable disease or symptoms of disease, and
may or may not have displayed detectable disease or symptoms of disease prior to the
treatment methods described herein. "At risk" denotes that an individual has one or more
risk factors, which are measurable parameters that correlate with development of a
particular disease, disorder, or condition, as known in the art. An individual having one or
more of these risk factors has a higher probability of developing a particular disease,
disorder, or condition than an individual without one or more of these risk factors.
As used herein, the term "*treatment*" refers to clinical intervention designed to
alter the natural course of the individual being treated during the course of clinical
pathology. Desirable effects of treatment include decreasing the rate of progression,
ameliorating or palliating the pathological state, and remission or improved prognosis of a
particular disease, disorder, or condition. An individual is successfully "treated", for
example, if one or more symptoms associated with a particular disease, disorder, or
condition are mitigated or eliminated.
An "*effective amount*" refers to at least an amount effective, at dosages and for
periods of time necessary, to achieve the desired therapeutic or prophylactic result. An
effective amount can be provided in one or more administrations.
A "*therapeutically effective amount*" is at least the minimum concentration
required to effect a measurable improvement of a particular disease, disorder, or condition.
A therapeutically effective amount herein may vary according to factors such as the disease
state, age, sex, and weight of the patient, and the ability of the anti-C1q antibody to elicit a
desired response in the individual. A therapeutically effective amount is also one in which
any toxic or detrimental effects of the anti-C1q antibody are outweighed by the
therapeutically beneficial effects.
*Chronic*" administration refers to administration of the medicament(s) in a
continuous as opposed to acute mode, so as to maintain the initial therapeutic effect
(activity) for an extended period of time. "*Intermittent*" administration refers to treatment
that is not consecutively done without interruption, but rather is cyclic in nature.
As used herein, administration "*in conjunction*" with another compound or
composition includes simultaneous administration and/or administration at different times.
Administration in conjunction also encompasses administration as a co-formulation or

administration as separate compositions, including at different dosing frequencies or
intervals, and using the same route of administration or different routes of administration.
An "individual" for purposes of treatment, prevention, or reduction of risk refers
to any animal classified as a mammal, including humans, domestic and farm animals, and
zoo, sport, or pet animals, such as dogs, horses, rabbits, cattle, pigs, hamsters, gerbils, mice,
ferrets, rats, cats, and the like. In some embodiments, the individual is human.
As used herein, "autoantibody" means any antibody that recognizes a host
antigen.
The term "immunoglobulin" (Ig) is used interchangeably with "antibody"
herein. The term "antibody" herein is used in the broadest sense and specifically covers
monoclonal antibodies, polyclonal antibodies, multispecific antibodies (e.g. bispecific
antibodies) formed from at least two intact antibodies, and antibody fragments so long as
they exhibit the desired biological activity.
The basic 4-chain antibody unit is a heterotetrameric glycoprotein composed of
two identical light (L) chains and two identical heavy (H) chains. The pairing of a VH and
VL together forms a single antigen-binding site. For the structure and properties of the
different classes of antibodies, see, e.g., Basic and Clinical Immunology, 8th Ed., Daniel P.
Stites, Abba I. Terr and Tristram G. Parslow (eds.), Appleton & Lange, Norwalk, CT, 1994,
page 71 and Chapter 6.
The L chain from any vertebrate species can be assigned to one of two clearly
distinct types, called kappa ("κ") and lambda ("λ"), based on the amino acid sequences of
their constant domains. Depending on the amino acid sequence of the constant domain of
their heavy chains (CH), immunoglobulins can be assigned to different classes or isotypes.
There are five classes of immunoglobulins: IgA, IgD, IgE, IgG, and IgM, having heavy
chains designated alpha ("α"), delta ("δ"), epsilon ("ε"), gamma ("γ") and mu ("μ"),
respectively. The γ and α classes are further divided into subclasses (isotypes) on the basis
of relatively minor differences in the CH sequence and function, e.g., humans express the
following subclasses: IgG1, IgG2, IgG3, IgG4, IgA1, and IgA2. The subunit structures and
three dimensional configurations of different classes of immunoglobulins are well known
and described generally in, for example, Abbas et al., Cellular and Molecular Immunology,
4th ed. (W.B. Saunders Co., 2000).
"Native antibodies" are usually heterotetrameric glycoproteins of about 150,000
daltons, composed of two identical light (L) chains and two identical heavy (H) chains.

Each light chain is linked to a heavy chain by one covalent disulfide bond, while the
number of disulfide linkages varies among the heavy chains of different immunoglobulin
isotypes. Each heavy and light chain also has regularly spaced intrachain disulfide bridges.
Each heavy chain has at one end a variable domain (VH) followed by a number of constant
domains. Each light chain has a variable domain at one end (VL) and a constant domain at
its other end; the constant domain of the light chain is aligned with the first constant
domain of the heavy chain, and the light chain variable domain is aligned with the variable
domain of the heavy chain. Particular amino acid residues are believed to form an interface
between the light chain and heavy chain variable domains.
An "isolated" antibody, such as an anti-C1q antibody of the present disclosure,
is one that has been identified, separated and/or recovered from a component of its
production environment (e.g., naturally or recombinantly). In some embodiments, the
isolated polypeptide is free of association with all other contaminant components from its
production environment. Contaminant components from its production environment, such
as those resulting from recombinant transfected cells, are materials that would typically
interfere with research, diagnostic or therapeutic uses for the antibody, and may include
enzymes, hormones, and other proteinaceous or non-proteinaceous solutes. In some
embodiments, the polypeptide will be purified: (1) to greater than 95% by weight of
antibody as determined by, for example, the Lowry method, and in some embodiments, to
greater than 99% by weight; (2) to a degree sufficient to obtain at least 15 residues of N-
terminal or internal amino acid sequence by use of a spinning cup sequenator, or (3) to
homogeneity by SDS-PAGE under non-reducing or reducing conditions using Coomassie
blue or silver stain. Isolated antibody includes the antibody in situ within recombinant T-
cells since at least one component of the antibody's natural environment will not be
present. Ordinarily, however, an isolated polypeptide or antibody will be prepared by at
least one purification step.
The "variable region" or "variable domain" of an antibody, such as an anti-C1q
antibody of the present disclosure, refers to the amino-terminal domains of the heavy or
light chain of the antibody. The variable domains of the heavy chain and light chain may
be referred to as "VH" and "VL", respectively. These domains are generally the most
variable parts of the antibody (relative to other antibodies of the same class) and contain the
antigen binding sites.

The term "*variable*" refers to the fact that certain segments of the variable
domains differ extensively in sequence among antibodies, such as anti-C1q antibodies of
the present disclosure. The V domain mediates antigen binding and defines the specificity
of a particular antibody for its particular antigen. However, the variability is not evenly
distributed across the entire span of the variable domains. Instead, it is concentrated in
three segments called hypervariable regions (HVRs) both in the light-chain and the heavy
chain variable domains. The more highly conserved portions of variable domains are called
the framework regions (FR). The variable domains of native heavy and light chains each
comprise four FR regions, largely adopting a beta-sheet configuration, connected by three
HVRs, which form loops connecting, and in some cases forming part of, the beta-sheet
structure. The HVRs in each chain are held together in close proximity by the FR regions
and, with the HVRs from the other chain, contribute to the formation of the antigen binding
site of antibodies (*see* Kabat et al., *Sequences of Immunological Interest*, Fifth Edition,
National Institute of Health, Bethesda, MD (1991)). The constant domains are not involved
directly in the binding of antibody to an antigen, but exhibit various effector functions, such
as participation of the antibody in antibody-dependent-cellular toxicity.
The term "*monoclonal antibody*" as used herein refers to an antibody, such as
an anti- C1q antibody of the present disclosure, obtained from a population of substantially
homogeneous antibodies, *i.e.*, the individual antibodies comprising the population are
identical except for possible naturally occurring mutations and/or post-translation
modifications (*e.g.*, isomerizations, amidations) that may be present in minor amounts.
Monoclonal antibodies are highly specific, being directed against a single antigenic site. In
contrast to polyclonal antibody preparations which typically include different antibodies
directed against different determinants (epitopes), each monoclonal antibody is directed
against a single determinant on the antigen. In addition to their specificity, the monoclonal
antibodies are advantageous in that they are synthesized by the hybridoma culture,
uncontaminated by other immunoglobulins. The modifier "monoclonal" indicates the
character of the antibody as being obtained from a substantially homogeneous population of
antibodies, and is not to be construed as requiring production of the antibody by any
particular method. For example, the monoclonal antibodies to be used in accordance with
the present disclosure may be made by a variety of techniques, including, for example, the
hybridoma method (*e.g.*, Kohler and Milstein., *Nature*, 256:495-97 (1975); Hongo et al.,
*Hybridoma*, 14 (3):253-260 (1995), Harlow et al., *Antibodies: A Laboratory Manual*, (Cold

Spring Harbor Laboratory Press, 2d ed. 1988); Hammerling et al., in: Monoclonal
Antibodies and T-Cell Hybridomas 563-681 (Elsevier, N.Y., 1981)), recombinant DNA
methods (see, e.g., U.S. Patent No. 4,816,567), phage-display technologies (see, e.g.,
Clackson et al., Nature, 352:624-628 (1991); Marks et al., J. Mol. Biol. 222:581-597
(1992); Sidhu et al., J. Mol. Biol. 338(2): 299-310 (2004); Lee et al., J. Mol. Biol.
340(5):1073-1093 (2004); Fellouse, Proc. Nat'l Acad. Sci. USA 101(34):12467-472 (2004);
and Lee et al., J. Immunol. Methods 284(1-2):119-132 (2004), and technologies for
producing human or human-like antibodies in animals that have parts or all of the human
immunoglobulin loci or genes encoding human immunoglobulin sequences (see, e.g., WO
1998/24893; WO 1996/34096; WO 1996/33735; WO 1991/10741; Jakobovits et al., Proc.
Nat'l Acad. Sci. USA 90:2551 (1993); Jakobovits et al., Nature 362:255-258 (1993);
Bruggemann et al., Year in Immunol. 7:33 (1993); U.S. Patent Nos. 5,545,807; 5,545,806;
5,569,825; 5,625,126; 5,633,425; and 5,661,016; Marks et al., Bio/Technology 10:779-783
(1992); Lonberg et al., Nature 368:856-859 (1994); Morrison, Nature 368:812-813 (1994);
Fishwild et al., Nature Biotechnol. 14:845-851 (1996); Neuberger, Nature Biotechnol.
14:826 (1996); and Lonberg and Huszar, Intern. Rev. Immunol. 13:65-93 (1995).
The terms "full-length antibody," "intact antibody" or "whole antibody" are
used interchangeably to refer to an antibody, such as and anti-C1q antibody of the present
disclosure, in its substantially intact form, as opposed to an antibody fragment. Specifically
whole antibodies include those with heavy and light chains including an Fc region. The
constant domains may be native sequence constant domains (e.g., human native sequence
constant domains) or amino acid sequence variants thereof. In some cases, the intact
antibody may have one or more effector functions.
An "antibody fragment" comprises a portion of an intact antibody, the antigen
binding and/or the variable region of the intact antibody. Examples of antibody fragments
include Fab, Fab', F(ab')2 and Fv fragments; diabodies; linear antibodies (see U.S. Patent
5,641,870, Example 2; Zapata et al., Protein Eng. 8(10):1057-1062 (1995)); single-chain
antibody molecules and multispecific antibodies formed from antibody fragments.
Papain digestion of antibodies, such as anti-C1q antibodies of the present
disclosure, produces two identical antigen-binding fragments or regions, called "Fab"
fragments or regions, and a residual "Fc" fragment or region, a designation reflecting the
ability to crystallize readily. The Fab fragment or region consists of an entire L chain along
with the variable region domain of the H chain (VH), and the first constant domain of one

heavy chain (C_{H}1). Each Fab fragment or region is monovalent with respect to antigen
binding, *i.e.*, it has a single antigen-binding site. Pepsin treatment of an antibody yields a
single large F(ab')₂ fragment or region which roughly corresponds to two disulfide linked
Fab fragments having different antigen-binding activity and is still capable of cross-linking
antigen. Fab' fragments or regions differ from Fab fragments by having a few additional
residues at the carboxy terminus of the C_{H}1 domain including one or more cysteines from
the antibody hinge region. Fab'-SH is the designation herein for Fab' in which the cysteine
residue(s) of the constant domains bear a free thiol group. F(ab')₂ antibody fragments
originally were produced as pairs of Fab' fragments which have hinge cysteines between
them. Other chemical couplings of antibody fragments are also known.
**[0060]** The Fc fragment or region comprises the carboxy-terminal portions of both H
chains held together by disulfides. The effector functions of antibodies are determined by
sequences in the Fc region, the region which is also recognized by Fc receptors (FcR) found
on certain types of cells.
**[0061]** "*Fv*" is the minimum antibody fragment which contains a complete antigen-
recognition and -binding site. This fragment consists of a dimer of one heavy- and one
light-chain variable region domain in tight, non-covalent association. From the folding of
these two domains emanate six hypervariable loops (3 loops each from the H and L chain)
that contribute the amino acid residues for antigen binding and confer antigen binding
specificity to the antibody. However, even a single variable domain (or half of an Fv
comprising only three HVRs specific for an antigen) has the ability to recognize and bind
antigen, although at a lower affinity than the entire binding site.
**[0062]** "*Single-chain Fv*" also abbreviated as "*sFv*" or "*scFv*" are antibody fragments
that comprise the VH and VL antibody domains connected into a single polypeptide chain.
In some embodiments, the sFv polypeptide further comprises a polypeptide linker between
the V_{H} and V_{L} domains which enables the sFv to form the desired structure for antigen
binding. For a review of the sFv, *see* Plückthun in *The Pharmacology of Monoclonal
Antibodies*, vol. 113, Rosenburg and Moore eds., Springer-Verlag, New York, pp. 269-315
(1994).
**[0063]** "*Functional fragments*" of antibodies, such as anti-C1q antibodies of the present
disclosure, comprise a portion of an intact antibody, generally including the antigen binding
or variable region of the intact antibody or the F region of an antibody which retains or has
modified FcR binding capability. Examples of antibody fragments include linear antibody,

single-chain antibody molecules and multispecific antibodies formed from antibody
fragments.
The term "*diabodies*" refers to small antibody fragments prepared by
constructing sFv fragments (see preceding paragraph) with short linkers (about 5-10)
residues) between the V_{H} and V_{L} domains such that inter-chain but not intra-chain pairing
of the V domains is achieved, thereby resulting in a bivalent fragment, *i.e.*, a fragment
having two antigen-binding sites. Bispecific diabodies are heterodimers of two "crossover"
sFv fragments in which the V_{H} and V_{L} domains of the two antibodies are present on
different polypeptide chains. Diabodies are described in greater detail in, for example, EP
404,097; WO 93/11161; Hollinger et al., *Proc. Nat'l Acad. Sci. USA* 90:6444-48 (1993).
As used herein, a "chimeric antibody" refers to an antibody (immunoglobulin),
such as an anti-C1q antibody of the present disclosure, in which a portion of the heavy
and/or light chain is identical with or homologous to corresponding sequences in antibodies
derived from a particular species or belonging to a particular antibody class or subclass,
while the remainder of the chain(s) is(are) identical with or homologous to corresponding
sequences in antibodies derived from another species or belonging to another antibody class
or subclass, as well as fragments of such antibodies, so long as they exhibit the desired
biological activity (U.S. Patent No. 4,816,567; Morrison et al., *Proc. Nat'l Acad. Sci. USA*,
81:6851-55 (1984)). Chimeric antibodies of interest herein include PRIMATIZED^{®}
antibodies wherein the antigen-binding region of the antibody is derived from an antibody
produced by, *e.g.*, immunizing macaque monkeys with an antigen of interest. As used
herein, "humanized antibody" is used a subset of "chimeric antibodies."
"*Humanized*" forms of non-human (*e.g.*, murine) antibodies, such as anti-C1q
antibodies of the present disclosure, are chimeric antibodies that contain minimal sequence
derived from non-human immunoglobulin. In one embodiment, a humanized antibody is a
human immunoglobulin (recipient antibody) in which residues from an HVR of the
recipient are replaced by residues from an HVR of a non-human species (donor antibody)
such as mouse, rat, rabbit or non-human primate having the desired specificity, affinity,
and/or capacity. In some instances, FR residues of the human immunoglobulin are replaced
by corresponding non-human residues. Furthermore, humanized antibodies may comprise
residues that are not found in the recipient antibody or in the donor antibody. These
modifications may be made to further refine antibody performance, such as binding affinity.
In general, a humanized antibody will comprise substantially all of at least one, and

typically two, variable domains, in which all or substantially all of the hypervariable loops
correspond to those of a non-human immunoglobulin sequence, and all or substantially all
of the FR regions are those of a human immunoglobulin sequence, although the FR regions
may include one or more individual FR residue substitutions that improve antibody
performance, such as binding affinity, isomerization, immunogenicity, and the like. The
number of these amino acid substitutions in the FR is typically no more than 6 in the H
chain, and in the L chain, no more than 3. The humanized antibody optionally will also
comprise at least a portion of an immunoglobulin constant region (Fc), typically that of a
human immunoglobulin. For further details, see, e.g., Jones et al., Nature 321:522-525
(1986); Riechmann et al., Nature 332:323-329 (1988); and Presta, Curr. Op. Struct. Biol.
2:593-596 (1992). See also, for example, Vaswani and Hamilton, Ann. Allergy, Asthma &
Immunol. 1:105-115 (1998); Harris, Biochem. Soc. Transactions 23:1035-1038 (1995);
Hurle and Gross, Curr. Op. Biotech. 5:428-433 (1994); and U.S. Patent Nos. 6,982,321 and
7,087,409.
A "human antibody" is one that possesses an amino-acid sequence
corresponding to that of an antibody, such as an anti-C1q antibody of the present
disclosure, produced by a human and/or has been made using any of the techniques for
making human antibodies as disclosed herein. This definition of a human antibody
specifically excludes a humanized antibody comprising non-human antigen-binding
residues. Human antibodies can be produced using various techniques known in the art,
including phage-display libraries. Hoogenboom and Winter, J. Mol. Biol., 227:381 (1991);
Marks et al., J. Mol. Biol., 222:581 (1991). Also available for the preparation of human
monoclonal antibodies are methods described in Cole et al., Monoclonal Antibodies and
Cancer Therapy, Alan R. Liss, p. 77 (1985); Boerner et al., J. Immunol., 147(1):86-95
(1991). See also van Dijk and van de Winkel, Curr. Opin. Pharmacol. 5:368-74 (2001).
Human antibodies can be prepared by administering the antigen to a transgenic animal that
has been modified to produce such antibodies in response to antigenic challenge, but whose
endogenous loci have been disabled, e.g., immunized xenomice (see, e.g., U.S. Patent Nos.
6,075,181 and 6,150,584 regarding XENOMOUSE™ technology). See also, for example,
Li et al., Proc. Nat'l Acad. Sci. USA, 103:3557-3562 (2006) regarding human antibodies
generated via a human B-cell hybridoma technology.
The term "hypervariable region," "HVR," or "HV," when used herein refers to
the regions of an antibody-variable domain, such as that of an anti-C1q antibody of the

present disclosure, that are hypervariable in sequence and/or form structurally defined
loops. Generally, antibodies comprise six HVRs; three in the VH (H1, H2, H3), and three
in the VL (L1, L2, L3). In native antibodies, H3 and L3 display the most diversity of the
six HVRs, and H3 in particular is believed to play a unique role in conferring fine
specificity to antibodies. *See, e.g.*, Xu et al., *Immunity* 13:37-45 (2000); Johnson and Wu in
*Methods in Molecular Biology* 248:1-25 (Lo, ed., Human Press, Totowa, NJ, 2003)).
Indeed, naturally occurring camelid antibodies consisting of a heavy chain only are
functional and stable in the absence of light chain. See, *e.g.*, Hamers-Casterman et al.,
*Nature* 363:446-448 (1993) and Sheriff et al., *Nature Struct. Biol.* 3:733-736 (1996).
**[0069]** A number of HVR delineations are in use and are encompassed herein. The
HVRs that are Kabat complementarity-determining regions (CDRs) are based on sequence
variability and are the most commonly used (Kabat et al., *supra*). Chothia refers instead to
the location of the structural loops (Chothia and Lesk *J. Mol. Biol.* 196:901-917 (1987)).
The AbM HVRs represent a compromise between the Kabat CDRs and Chothia structural
loops, and are used by Oxford Molecular's AbM antibody-modeling software. The
"contact" HVRs are based on an analysis of the available complex crystal structures. The
residues from each of these HVRs are noted below.

Loop | Kabat | AbM | Chothia | Contact
L1 | L24-L34 | L24-L34 | L26-L32 | L30-L36
L2 | L50-L56 | L50-L56 | L50-L52 | L46-L55
L3 | L89-L97 | L89-L97 | L91-L96 | L89-L96
H1 | H31-H35B | H26-H35B | H26-H32 | H30-H35B (Kabat numbering)
H1 | H31-H35 | H26-H35 | H26-H32 | H30-H35 (Chothia numbering)
H2 | H50-H65 | H50-H58 | H53-H55 | H47-H58
H3 | H95-H102 | H95-H102 | H96-H101 | H93-H101

**[0070]** HVRs may comprise "extended HVRs" as follows: 24-36 or 24-34 (L1), 46-56
or 50-56 (L2), and 89-97 or 89-96 (L3) in the VL, and 26-35 (H1), 50-65 or 49-65 (H2),
and 93-102, 94-102, or 95-102 (H3) in the VH. The variable-domain residues are
numbered according to Kabat et al., *supra*, for each of these extended-HVR definitions.
**[0071]** "*Framework*" or "*FR*" residues are those variable-domain residues other than
the HVR residues as herein defined.
**[0072]** The phrase "*variable-domain residue-numbering as in Kabat*" or "*amino-acid-
position numbering as in Kabat*," and variations thereof, refers to the numbering system

used for heavy-chain variable domains or light-chain variable domains of the compilation
of antibodies in Kabat et al., supra. Using this numbering system, the actual linear amino
acid sequence may contain fewer or additional amino acids corresponding to a shortening
of, or insertion into, a FR or HVR of the variable domain. For example, a heavy-chain
variable domain may include a single amino acid insert (residue 52a according to Kabat)
after residue 52 of H2 and inserted residues (e.g., residues 82a, 82b, and 82c, etc. according
to Kabat) after heavy-chain FR residue 82. The Kabat numbering of residues may be
determined for a given antibody by alignment at regions of homology of the sequence of
the antibody with a "standard" Kabat numbered sequence.
The Kabat numbering system is generally used when referring to a residue in the
variable domain (approximately residues 1-107 of the light chain and residues 1-113 of the
heavy chain) (e.g., Kabat et al., Sequences of Immunological Interest. 5th Ed. Public Health
Service, National Institutes of Health, Bethesda, Md. (1991)). The "EU numbering system"
or "EU index" is generally used when referring to a residue in an immunoglobulin heavy
chain constant region (e.g., the EU index reported in Kabat et al., supra). The "EU index
as in Kabat" refers to the residue numbering of the human IgG1 EU antibody. Unless
stated otherwise herein, references to residue numbers in the variable domain of antibodies
means residue numbering by the Kabat numbering system. Unless stated otherwise herein,
references to residue numbers in the constant domain of antibodies means residue
numbering by the EU numbering system (e.g., see United States Patent Publication No.
2010-280227).
An "acceptor human framework" as used herein is a framework comprising the
amino acid sequence of a VL or VH framework derived from a human immunoglobulin
framework or a human consensus framework. An acceptor human framework "derived
from" a human immunoglobulin framework or a human consensus framework may
comprise the same amino acid sequence thereof, or it may contain pre-existing amino acid
sequence changes. In some embodiments, the number of pre-existing amino acid changes
are 10 or less, 9 or less, 8 or less, 7 or less, 6 or less, 5 or less, 4 or less, 3 or less, or 2 or
less. In some embodiments, where pre-existing amino acid changes are present in a VH,
those changes occur at only three, two, or one of positions 71H, 73H and 78H; for instance,
the amino acid residues at those positions may by 71A, 73T and/or 78A. In one
embodiment, the VL acceptor human framework is identical in sequence to the VL human
immunoglobulin framework sequence or human consensus framework sequence.

A "*human consensus framework*" is a framework that represents the most
commonly occurring amino acid residues in a selection of human immunoglobulin VL or
VH framework sequences. Generally, the selection of human immunoglobulin VL or VH
sequences is from a subgroup of variable domain sequences. Generally, the subgroup of
sequences is a subgroup as in Kabat et al., *Sequences of Proteins of Immunological Interest*,
5th Ed. Public Health Service, National Institutes of Health, Bethesda, MD (1991).
Examples include for the VL, the subgroup may be subgroup kappa I, kappa II, kappa III or
kappa IV as in Kabat *et al., supra.* Additionally, for the VH, the subgroup may be
subgroup I, subgroup II, or subgroup III as in Kabat *et al., supra.*
An "*amino-acid modification*" at a specified position, *e.g.*, of an anti-C1q
antibody of the present disclosure, refers to the substitution or deletion of the specified
residue, or the insertion of at least one amino acid residue adjacent the specified residue.
Insertion "adjacent" to a specified residue means insertion within one to two residues
thereof. The insertion may be N-terminal or C-terminal to the specified residue. In some
embodiments, the amino acid modification herein is a substitution.
An "*affinity-matured*" antibody, such as an anti- C1q antibody of the present
disclosure, is one with one or more alterations in one or more HVRs thereof that result in an
improvement in the affinity of the antibody for antigen, compared to a parent antibody that
does not possess those alteration(s). In one embodiment, an affinity-matured antibody has
nanomolar or even picomolar affinities for the target antigen. Affinity-matured antibodies
are produced by procedures known in the art. For example, Marks et al., *Bio/Technology*
10:779-783 (1992) describes affinity maturation by VH- and VL-domain shuffling.
Random mutagenesis of HVR and/or framework residues is described by, for example:
Barbas et al. *Proc Nat. Acad. Sci. USA* 91:3809-3813 (1994); Schier et al. *Gene* 169:147-
155 (1995); Yelton et al. *J. Immunol.* 155:1994-2004 (1995); Jackson et al., *J. Immunol.*
154(7):3310-9 (1995); and Hawkins *et al, J. Mol. Biol.* 226:889-896 (1992).
As use herein, the term "*specifically recognizes*" or "*specifically binds*" refers to
measurable and reproducible interactions such as attraction or binding between a target and
an antibody, such as an anti-C1q antibody of the present disclosure, that is determinative of
the presence of the target in the presence of a heterogeneous population of molecules
including biological molecules. For example, an antibody, such as an anti-C1q antibody of
the present disclosure, that specifically or preferentially binds to a target or an epitope is an
antibody that binds this target or epitope with greater affinity, avidity, more readily, and/or

with greater duration than it binds to other targets or other epitopes of the target. It is also
understood by reading this definition that, for example, an antibody (or a moiety) that
specifically or preferentially binds to a first target may or may not specifically or
preferentially bind to a second target. As such, "specific binding" or "preferential binding"
does not necessarily require (although it can include) exclusive binding. An antibody that
specifically binds to a target may have an association constant of at least about 10 ³ M ⁻¹ or
10 ⁴ M ⁻¹, sometimes about 10 ⁵ M ⁻¹ or 10 ⁶ M ⁻¹, in other instances about 10 ⁶ M ⁻¹ or 10 ⁷ M
⁻¹, about 10 ⁸ M ⁻¹ to 10 ⁹ M ⁻¹, or about 10 ¹⁰ M ⁻¹ to 10 ¹¹ M ⁻¹ or higher. A variety of
immunoassay formats can be used to select antibodies specifically immunoreactive with a
particular protein. For example, solid-phase ELISA immunoassays are routinely used to
select monoclonal antibodies specifically immunoreactive with a protein. See, e.g., Harlow
and Lane (1988) Antibodies, A Laboratory Manual, Cold Spring Harbor Publications, New
York, for a description of immunoassay formats and conditions that can be used to
determine specific immunoreactivity.
As used herein, an "interaction" between a complement protein, such as
complement factor C1q, and a second protein encompasses, without limitation, protein-
protein interaction, a physical interaction, a chemical interaction, binding, covalent binding,
and ionic binding. As used herein, an antibody "inhibits interaction" between two proteins
when the antibody disrupts, reduces, or completely eliminates an interaction between the
two proteins. An antibody of the present disclosure, or fragment thereof, "inhibits
interaction" between two proteins when the antibody or fragment thereof binds to one of
the two proteins.
A "blocking" antibody, an "antagonist" antibody, an "inhibitory" antibody, or a
"neutralizing" antibody is an antibody, such as an anti-C1q antibody of the present
disclosure that inhibits or reduces one or more biological activities of the antigen it binds,
such as interactions with one or more proteins. In some embodiments, blocking antibodies,
antagonist antibodies, inhibitory antibodies, or "neutralizing" antibodies substantially or
completely inhibit one or more biological activities or interactions of the antigen.
Antibody "effector functions" refer to those biological activities attributable to
the Fc region (a native sequence Fc region or amino acid sequence variant Fc region) of an
antibody, and vary with the antibody isotype.
The term "Fc region" herein is used to define a C-terminal region of an
immunoglobulin heavy chain, including native-sequence Fc regions and variant Fc regions.

Although the boundaries of the Fc region of an immunoglobulin heavy chain might vary,
the human IgG heavy-chain Fc region is usually defined to stretch from an amino acid
residue at position Cys226, or from Pro230, to the carboxyl-terminus thereof. The C-
terminal lysine (residue 447 according to the EU numbering system) of the Fc region may
be removed, for example, during production or purification of the antibody, or by
recombinantly engineering the nucleic acid encoding a heavy chain of the antibody.
Accordingly, a composition of intact antibodies may comprise antibody populations with
all K447 residues removed, antibody populations with no K447 residues removed, and
antibody populations having a mixture of antibodies with and without the K447 residue.
Suitable native-sequence Fc regions for use in the antibodies of the present disclosure
include human IgG1, IgG2, IgG3 and IgG4.
A "native sequence Fc region" comprises an amino acid sequence identical to
the amino acid sequence of an Fc region found in nature. Native sequence human Fc
regions include a native sequence human IgG1 Fc region (non-A and A allotypes); native
sequence human IgG2 Fc region; native sequence human IgG3 Fc region; and native
sequence human IgG4 Fc region as well as naturally occurring variants thereof.
A "variant Fc region" comprises an amino acid sequence which differs from
that of a native sequence Fc region by virtue of at least one amino acid modification. In
some embodiments, the variant Fc region differs in one or more amino acid substitution(s).
In some embodiments, the variant Fc region has at least one amino acid substitution
compared to a native sequence Fc region or to the Fc region of a parent polypeptide, e.g.
from about one to about ten amino acid substitutions, and, in some embodiments, from
about one to about five amino acid substitutions in a native sequence Fc region or in the Fc
region of the parent polypeptide. The variant Fc region herein will, in some embodiments,
possess at least about 80% homology with a native sequence Fc region and/or with an Fc
region of a parent polypeptide, and, in some embodiments, at least about 90% homology
therewith, and, in some embodiments, at least about 95% homology therewith.
"Fc receptor" or "FcR" describes a receptor that binds to the Fc region of an
antibody. In some embodiments, the FcR is a native sequence human FcR. Moreover, in
some embodiments, a FcR is one which binds an IgG antibody (a gamma receptor) and
includes receptors of the FcγRI, FcγRII, and FcγRIII subclasses, including allelic variants
and alternatively spliced forms of these receptors, FcγRII receptors include FcγRIIA (an
"activating receptor") and FcγRIIB (an "inhibiting receptor"), which have similar amino

acid sequences that differ primarily in the cytoplasmic domains thereof. Activating
receptor FcγRIIA contains an immunoreceptor tyrosine-based activation motif ("ITAM") in
its cytoplasmic domain. Inhibiting receptor FcγRIIB contains an immunoreceptor tyrosine-
based inhibition motif ("ITIM") in its cytoplasmic domain (see, e.g., M. Daëron, Annu. Rev.
Immunol. 15:203-234 (1997)). FcRs are reviewed in Ravetch and Kinet, Annu. Rev.
Immunol. 9:457-92 (1991); Capel et al., Immunomethods 4:25-34 (1994); and de Haas et
al., J. Lab. Clin. Med. 126: 330-41 (1995). Other FcRs, including those to be identified in
the future, are encompassed by the term "FcR" herein. FcRs can also increase the serum
half-life of antibodies.
Binding to FcRn in vivo and serum half-life of human FcRn high-affinity
binding polypeptides can be assayed, e.g., in transgenic mice or transfected human cell
lines expressing human FcRn, or in primates to which the polypeptides having a variant Fc
region are administered. WO 2004/42072 (Presta) describes antibody variants with
improved or diminished binding to FcRs. See also, e.g., Shields et al., J. Biol. Chem.
9(2):6591-6604 (2001).
The term "kon", as used herein, is intended to refer to the rate constant for
association of an antibody to an antigen.
The term "koff", as used herein, is intended to refer to the rate constant for
dissociation of an antibody from the antibody/antigen complex.
The term "KD", as used herein, is intended to refer to the equilibrium dissociation
constant of an antibody-antigen interaction.
As used herein, "percent (%) amino acid sequence identity" and "homology"
with respect to a peptide, polypeptide or antibody sequence refers to the percentage of
amino acid residues in a candidate sequence that are identical with the amino acid residues
in the specific peptide or polypeptide sequence, after aligning the sequences and
introducing gaps, if necessary, to achieve the maximum percent sequence identity, and not
considering any conservative substitutions as part of the sequence identity. Alignment for
purposes of determining percent amino acid sequence identity can be achieved in various
ways that are within the skill in the art, for instance, using publicly available computer
software such as BLAST, BLAST-2, ALIGN or MEGALIGN™ (DNASTAR) software.
Those skilled in the art can determine appropriate parameters for measuring alignment,
including any algorithms known in the art needed to achieve maximal alignment over the
full length of the sequences being compared.

An "isolated" molecule or cell is a molecule or a cell that is identified and
separated from at least one contaminant molecule or cell with which it is ordinarily
associated in the environment in which it was produced. In some embodiments, the
isolated molecule or cell is free of association with all components associated with the
production environment. The isolated molecule or cell is in a form other than in the form
or setting in which it is found in nature. Isolated molecules therefore are distinguished
from molecules existing naturally in cells; isolated cells are distinguished from cells
existing naturally in tissues, organs, or individuals. In some embodiments, the isolated
molecule is an anti-C1q antibody of the present disclosure. In other embodiments, the
isolated cell is a host cell or hybridoma cell producing an anti-C1q antibody of the present
disclosure.
An "isolated" nucleic acid molecule encoding an antibody, such as an anti-C1q
antibody of the present disclosure, is a nucleic acid molecule that is identified and separated
from at least one contaminant nucleic acid molecule with which it is ordinarily associated
in the environment in which it was produced. In some embodiments, the isolated nucleic
acid is free of association with all components associated with the production environment.
The isolated nucleic acid molecules encoding the polypeptides and antibodies herein is in a
form other than in the form or setting in which it is found in nature. Isolated nucleic acid
molecules therefore are distinguished from nucleic acid encoding the polypeptides and
antibodies herein existing naturally in cells.
The term "vector," as used herein, is intended to refer to a nucleic acid molecule
capable of transporting another nucleic acid to which it has been linked. One type of vector
is a "plasmid," which refers to a circular double stranded DNA into which additional DNA
segments may be ligated. Another type of vector is a phage vector. Another type of vector
is a viral vector, wherein additional DNA segments may be ligated into the viral genome.
Certain vectors are capable of autonomous replication in a host cell into which they are
introduced (e.g., bacterial vectors having a bacterial origin of replication and episomal
mammalian vectors). Other vectors (e.g., non-episomal mammalian vectors) can be
integrated into the genome of a host cell upon introduction into the host cell, and thereby
are replicated along with the host genome. Moreover, certain vectors are capable of
directing the expression of genes to which they are operatively linked. Such vectors are
referred to herein as "recombinant expression vectors," or simply, "expression vectors." In
general, expression vectors of utility in recombinant DNA techniques are often in the form

of plasmids. In the present specification, "plasmid" and "vector" may be used
interchangeably as the plasmid is the most commonly used form of vector.
"Polynucleotide," or "nucleic acid," as used interchangeably herein, refer to
polymers of nucleotides of any length, and include DNA and RNA. The nucleotides can be
deoxyribonucleotides, ribonucleotides, modified nucleotides or bases, and/or their analogs,
or any substrate that can be incorporated into a polymer by DNA or RNA polymerase or by
a synthetic reaction. A polynucleotide may comprise modified nucleotides, such as
methylated nucleotides and their analogs. If present, modification to the nucleotide
structure may be imparted before or after assembly of the polymer. The sequence of
nucleotides may be interrupted by non-nucleotide components. A polynucleotide may
comprise modification(s) made after synthesis, such as conjugation to a label. Other types
of modifications include, for example, "caps," substitution of one or more of the naturally
occurring nucleotides with an analog, internucleotide modifications such as, for example,
those with uncharged linkages (e.g., methyl phosphonates, phosphotriesters,
phosphoamidates, carbamates, etc.) and with charged linkages (e.g., phosphorothioates,
phosphorodithioates, etc.), those containing pendant moieties, such as, for example,
proteins (e.g., nucleases, toxins, antibodies, signal peptides, ply-L-lysine, etc.), those with
intercalators (e.g., acridine, psoralen, etc.), those containing chelators (e.g., metals,
radioactive metals, boron, oxidative metals, etc.), those containing alkylators, those with
modified linkages (e.g., alpha anomeric nucleic acids, etc.), as well as unmodified forms of
the polynucleotides(s). Further, any of the hydroxyl groups ordinarily present in the sugars
may be replaced, for example, by phosphonate groups, phosphate groups, protected by
standard protecting groups, or activated to prepare additional linkages to additional
nucleotides, or may be conjugated to solid or semi-solid supports. The 5' and 3' terminal
OH can be phosphorylated or substituted with amines or organic capping group moieties of
from 1 to 20 carbon atoms. Other hydroxyls may also be derivatized to standard protecting
groups. Polynucleotides can also contain analogous forms of ribose or deoxyribose sugars
that are generally known in the art, including, for example, 2'-O-methyl-, 2'-O-allyl-, 2'-
fluoro- or 2'-azido-ribose, carbocyclic sugar analogs, α-anomeric sugars, epimeric sugars
such as arabinose, xyloses or lyxoses, pyranose sugars, furanose sugars, sedoheptuloses,
acyclic analogs, and basic nucleoside analogs such as methyl riboside. One or more
phosphodiester linkages may be replaced by alternative linking groups. These alternative
linking groups include, but are not limited to, embodiments wherein phosphate is replaced

by P(O)S ("thioate"), P(S)S ("dithioate"), (O)NR₂ ("amidate"), P(O)R, P(O)OR', CO, or
CH₂ ("formacetal"), in which each R or R' is independently H or substituted or
unsubstituted alkyl (1-20 C) optionally containing an ether (-O-) linkage, aryl, alkenyl,
cycloalkyl, cycloalkenyl or araldyl. Not all linkages in a polynucleotide need be identical.
The preceding description applies to all polynucleotides referred to herein, including RNA
and DNA.
A "*host cell*" includes an individual cell or cell culture that can be or has been a
recipient for vector(s) for incorporation of polynucleotide inserts. Host cells include
progeny of a single host cell, and the progeny may not necessarily be completely identical
(in morphology or in genomic DNA complement) to the original parent cell due to natural,
accidental, or deliberate mutation. A host cell includes cells transfected *in vivo* with a
polynucleotide(s) of this disclosure.
"*Carriers*" as used herein include pharmaceutically acceptable carriers,
excipients, or stabilizers that are nontoxic to the cell or mammal being exposed thereto at
the dosages and concentrations employed. Often the physiologically acceptable carrier is
an aqueous pH buffered solution. Examples of physiologically acceptable carriers include
buffers such as phosphate, citrate, and other organic acids; antioxidants including ascorbic
acid; low molecular weight (less than about 10 residues) polypeptide; proteins, such as
serum albumin, gelatin, or immunoglobulins; hydrophilic polymers such as
polyvinylpyrrolidone; amino acids such as glycine, glutamine, asparagine, arginine or
lysine; monosaccharides, disaccharides, and other carbohydrates including glucose,
mannose, or dextrins; chelating agents such as EDTA; sugar alcohols such as mannitol or
sorbitol; salt-forming counterions such as sodium; and/or nonionic surfactants such as
TWEEN^{TM}, polyethylene glycol (PEG), and PLURONICS^{TM}.
The term "*about*" as used herein refers to the usual error range for the respective
value readily known to the skilled person in this technical field. Reference to "*about*" a
value or parameter herein includes (and describes) embodiments that are directed to that
value or parameter *per se*.
As used herein and in the appended claims, the singular forms "a," "an," and
"the" include plural reference unless the context clearly indicates otherwise. For example,
reference to an "antibody" is a reference to from one to many antibodies, such as molar
amounts, and includes equivalents thereof known to those skilled in the art, and so forth.

It is understood that aspects and embodiments of the present disclosure
described herein include "comprising," "consisting," and "consisting essentially of" aspects
and embodiments.
Overview
The present disclosure provides humanized anti-C1q antibodies and uses
thereof. The humanized anti-C1q antibodies of the present disclosure specifically bind a
C1q protein of this disclosure. In some embodiments, the humanized anti-C1q antibodies
are C1q neutralizing antibodies. In some embodiments, the humanized anti-C1q antibodies
of this disclosure may bind to C1 complex.
In certain aspects, the present disclosure provides a humanized antibody that
specifically binds to a C1q protein, wherein the antibody comprises a heavy chain variable
region and a human heavy chain constant region, wherein the heavy chain variable region
comprises an Fab region and the heavy chain constant region comprises an Fc region,
wherein the Fab region specifically binds to the C1q protein, and wherein the Fc region is
incapable of binding the C1q protein.
In certain aspects, the present disclosure provides a humanized anti-C1q
antibody, or an antigen-binding fragment thereof, the antibody comprising a heavy chain
variable domain and a light chain variable domain, wherein the heavy chain variable
domain comprises an amino acid sequence selected from SEQ ID NOs: 1-4, or an amino
acid sequence with at least about 90% homology to the amino acid sequence selected from
SEQ ID NOs: 1-4.
In certain aspects, the present disclosure provides a humanized anti-C1q
antibody, or an antigen-binding fragment thereof, the antibody comprising a heavy chain
variable domain and a light chain variable domain, wherein the light chain variable domain
comprises an amino acid sequence selected from SEQ ID NOs: 5-8, or an amino acid
sequence with at least about 90% homology to the amino acid sequence selected from SEQ
ID NOs: 5-8.
In certain aspects, the present disclosure provides a humanized anti-C1q
antibody, or an antigen-binding fragment thereof, the antibody comprising: a heavy chain
variable domain comprising an amino acid sequence selected from SEQ ID NOs: 1-4, or an
amino acid sequence with at least about 90% homology to the amino acid sequence selected
from SEQ ID NOs: 1-4; and/or a light chain variable domain comprising an amino acid

sequence selected from SEQ ID NOs: 5-8, or an amino acid sequence with at least about
90% homology to the amino acid sequence selected from SEQ ID NOs: 5-8.
In some embodiments, the humanized anti-C1q antibodies of the present
disclosure neutralize a biological activity of C1q. Uses for humanized anti-C1q antibodies
include, without limitation, the detection of complement factor C1q, e.g., in individuals
having a neurodegenerative disorder associated with complement factor 1 (CF1)-dependent
pathological synapse loss. Additional non-limiting uses include the inhibition of the
classical pathway of complement activation, e.g., in cases where the classical complement
pathway is activated by autoantibodies. Further non-limiting uses for humanized anti-C1q
antibodies include the diagnosis and treatment of disorders that are associated with elevated
expression of complement factors, such as C1q, or associated with the activation of the
complement pathway. Such disorders may include, without limitation, autoimmune
disorders, inflammatory disorders, and neurodegenerative disorders, including
neurodegenerative disorders associated with synapse loss.
In another aspect, the present disclosure provides an isolated nucleic acid
molecule encoding an antibody of the present disclosure.
The present disclosure also provides isolated host cells containing a nucleic acid
molecule that encodes an antibody of this disclosure. Additionally, pharmaceutical
compositions are provided containing anti-C1q antibodies, such as humanized C1q
neutralizing antibodies of this disclosure, in combination with pharmaceutically acceptable
carriers. The present disclosure also provides a kit containing a humanized anti-C1q
antibody for use in any of the methods described herein.
The present disclosure further provides methods of using the humanized anti-
C1q antibodies of the present disclosure (e.g., humanized C1q neutralizing antibodies of
this disclosure) to treat or prevent a neurodegenerative disease or autoimmune disease in an
individual in need of such treatment, to detect synapses in an individual having a
neurodegenerative disease or autoimmune disease, and to detect synapses in a biological
sample. The present disclosure also provides kits containing humanized anti-C1q
antibodies of the present disclosure (e.g., humanized C1q neutralizing antibodies of this
disclosure).
Complement Proteins
The antibodies of this disclosure specifically recognize complement factor C1q
and/or C1q in the C1 complex of the classical complement activation pathway. The

recognized complement factor may be derived, without limitation, from any organism
having a complement system, including any mammalian organism such as human, mouse,
rat, rabbit, monkey, dog, cat, cow, horse, camel, sheep, goat, or pig.
As used herein "C1 complex" refers to a protein complex that may include,
without limitation, one C1q protein, two C1r proteins, and two C1s proteins (e.g., C1qr²s²).
As used herein "complement factor C1q" refers to both wild type sequences and
naturally occurring variant sequences.
A non-limiting example of a complement factor C1q recognized by antibodies
of this disclosure is human C1q, including the three polypeptide chains A, B, and C:
C1q, chain A (homo sapiens), Accession No. Protein Data Base: NP_057075.1; GenBank
No.: NM_015991:
>gi|7705753|ref|NP_057075.1| complement C1q subcomponent subunit A precursor [Homo
sapiens]




 (SEQ ID NO:9).
C1q, chain B (homo sapiens), Accession No. Protein Data Base: NP_000482.3; GenBank
No.: NM_000491.3:
>gi|87298828|ref|NP_000482.3| complement C1q subcomponent subunit B precursor
[Homo sapiens]
MMMKIPWGSIPVLMLLLLLGLIDISQAQLSCTGPPAIPGIPGIPGTPGPDGQPGTPGIK
GEKGLPGLAGDHGEFGEKGDPGIPGNPGKVGPKGPMGPKGGPGAPGAPGPKGESG
DYKATQKIAFSATRTINVPLRRDQTIRFDHVITNMNNNYEPRSGKFTCKVPGLYYFT
YHASSRGNLCVNLMRGRERAQKVVTFCDYAYNTFQVTTGGMVLKLEQGENVFLQ
ATDKNSLLGMEGANSIFSGFLLFPDMEA (SEQ ID NO:10).
C1q, chain C (homo sapiens), Accession No. Protein Data Base: NP_001107573.1;
GenBank No.: NM_001114101.1:
>gi|166235903|ref|NP_001107573.1| complement C1q subcomponent subunit C precursor
[Homo sapiens]





 (SEQ ID NO:11).
Accordingly, a humanized anti-C1q antibody of the present disclosure may bind
to polypeptide chain A, polypeptide chain B, and/or polypeptide chain C of a C1q protein.
In some embodiments, a humanized anti-C1q antibody of the present disclosure binds to
polypeptide chain A, polypeptide chain B, and/or polypeptide chain C of human C1q or a
homolog thereof, such as mouse, rat, rabbit, monkey, dog, cat, cow, horse, camel, sheep,
goat, or pig C1q.
Humanized anti-C1q Antibodies
Humanized antibodies of the present disclosure specifically bind to a
complement factor C1q and/or C1q protein in the C1 complex of the classical complement
pathway.  In some embodiments, the humanized anti-C1q antibodies specifically bind to
human C1q.  In some embodiments, the humanized anti-C1q antibodies specifically bind to
human and mouse C1q.  In some embodiments, the humanized anti-C1q antibodies
specifically bind to rat C1q.  In some embodiments, the humanized anti-C1q antibodies
specifically bind to human C1q, mouse C1q, and rat C1q.
In some embodiments, humanized anti-C1q antibodies of the present disclosure
include a heavy chain variable region that contains an Fab region and a heavy chain
constant regions that contains an Fc region, where the Fab region specifically binds to a
C1q protein of the present disclosure, but the Fc region is incapable of binding the C1q
protein.  In some embodiments, the Fc region is from a human IgG1, IgG2, IgG3, or IgG4
isotype.  In some embodiments, the Fc region is incapable of inducing complement activity
and/or incapable of inducing antibody-dependent cellular cytotoxicity (ADCC). In some
embodiments, the Fc region comprises one or more modifications, including, without
limitation, amino acid substitutions.  In certain embodiments, the Fc region of humanized
anti-C1q antibodies of the present disclosure comprise an amino acid substitution at
position 248 according to Kabat numbering convention or a position corresponding to
position 248 according to Kabat numbering convention, and/or at position 241 according to
Kabat numbering convention or a position corresponding to position 241 according to
Kabat numbering convention. In some embodiments, the amino acid substitution at position
248 or a positions corresponding to position 248 inhibits the Fc region from interacting
with an Fc receptor.  In some embodiments, the amino acid substitution at position 248 or a

positions corresponding to position 248 is a leucine to glutamate amino acid substitution. In
some embodiments, the amino acid substitution at position 241 or a positions corresponding
to position 241 prevents arm switching in the antibody. In some embodiments, the amino
acid substitution at position 241 or a positions corresponding to position 241 is a serine to
proline amino acid substitution. In certain embodiments, the Fc region of humanized anti-
C1q antibodies of the present disclosure comprises the amino acid sequence of SEQ ID
NO: 37, or an amino acid sequence with at least about 70%, at least about 75%, at least
about 80% at least about 85% at least about 90%, or at least about 95% homology to the
amino acid sequence of SEQ ID NO: 37.
In some embodiments, the humanized anti-C1q antibodies of the present
disclosure neutralize a biological activity of complement factor C1q. In some
embodiments, the antibodies inhibit the interaction between complement factor C1q and
other complement factors, such as C1r or C1s or between C1q and an antibody, such as an
autoantibody. As disclosed herein, an autoantibody of the present disclosure includes,
without limitation, an antibody that recognizes a host antigen and activates the classical
pathway of complement activation. In the first step of this activation process complement
factor C1q binds to the autoantibody-autoantigen-immune complex. In some embodiments,
the antibodies inhibit the interaction between complement factor C1q and a non-
complement factor. A non-complement factor may include phosphatidylserine, pentraxin-
3, C-reactive protein (CRP), globular C1q receptor (gC1qR), complement receptor 1 (CR1),
β-amyloid, and calreticulin. In some embodiments, the antibodies inhibit the classical
complement activation pathway. In certain embodiments, the antibodies further inhibit the
alternative pathway. In some embodiments, the antibodies inhibit autoantibody- and
complement-dependent cytotoxicity (CDC). In some embodiments, the antibodies inhibit
complement-dependent cell-mediated cytotoxicity (CDCC). In some embodiments, the
antibodies inhibit B-cell antibody production, dendritic cell maturation, T-cell proliferation,
cytokine production, or microglia activation. In some embodiments, the antibodies inhibit
the Arthus reaction. In some embodiments, the antibodies inhibit phagocytosis of synapses
or nerve endings. In some embodiments, the antibodies inhibit the activation of
complement receptor 3 (CR3/C3) expressing cells.
The functional properties of the antibodies of the present disclosure, such as
dissociation constants for antigens, inhibition of protein-protein interactions (e.g., C1q-
autoantibody interactions), inhibition of autoantibody-dependent and complement-

dependent cytotoxicity (CDC), inhibition of complement-dependent cell-mediated
cytotoxicity (CDCC), or lesion formation, may, without limitation, be measured in *in vitro*,
*ex vivo*, or *in vivo* experiments.
The dissociation constants (K_{D}) of the humanized anti-C1q antibodies for C1q
may be less than 125 nM, less than 120 nM, less than 115 nM, less than 110 nM, less than
100 nM, less than 90 nM, less than 80 nM, less than 70 nM, less than 60 nM, less than 50
nM, less than 40 nM, less than 30 nM, less than 20 nM, less than 10 nM, less than 9 nM,
less than 8 nM, less than 7 nM, less than 6 nM, less than 5 nM, less than 4 nM, less than 3
nM, less than 2 nM, less than 1 nM, less than 0.5 nM, less than 0.1 nM, less than 0.05 nM,
less than 0.01 nM, or less than 0.005 nM.  In some embodiments, dissociation constants
range from less than about 125 nM to less than about 5 pM.
In some embodiments, dissociation constants of humanized anti-C1q antibodies
of the present disclosure are less than about 10 pM to less than about 5 pM for human C1q.
In some embodiments, dissociation constants are less than about 10 pM, less than about 9.9
pm, less than about 9.8 pM, less than about 9.7 pM, less than about 9.6 pM, less than about
9.5 pM, less than about 9.4 pM, less than about 9.3 pM, less than about 9.2 pM, less than
about 9.1 pM, less than about 9 pM, less than about 8.9 pm, less than about 8.8 pM, less
than about 8.7 pM, less than about 8.6 pM, less than about 8.5 pM, less than about 8.4 pM,
less than about 8.3 pM, less than about 8.2 pM, less than about 8.1 pM, less than about 8
pM, less than about 7.9 pm, less than about 7.8 pM, less than about 7.7 pM, less than about
7.6 pM, less than about 7.5 pM, less than about 7.4 pM, less than about 7.3 pM, less than
about 7.2 pM, less than about 7.1 pM, less than about 7 pM, less than about 6.9 pm, less
than about 6.8 pM, less than about 6.7 pM, less than about 6.6 pM, less than about 6.5 pM,
less than about 6.4 pM, less than about 6.3 pM, less than about 6.2 pM, less than about 6.1
pM, less than about 6 pM, less than about 5.9 pm, less than about 5.8 pM, less than about
5.7 pM, less than about 5.6 pM, less than about 5.5 pM, less than about 5.4 pM, less than
about 5.3 pM, less than about 5.2 pM, less than about 5.1 pM, or less than about 5 pM, for
human C1q.
In some embodiments, dissociation constants of humanized anti-C1q antibodies
of the present disclosure are less than 125 nM, less than 120 nM, less than 115 nM, less
than 110 nM, less than 100 nM, less than 90 nM, less than 80 nM, less than 70 nM, less
than 60 nM, less than 50 nM, less than 40 nM, less than 30 nM, less than 20 nM, less than
10 nM, less than 9 nM, less than 8 nM, less than 7 nM, less than 6 nM, less than 5 nM, less

than 4 nM, less than 3 nM, less than 2 nM, less than 1 nM, less than 0.5 nM, less than 0.1
nM, or less than 0.05 nM for mouse C1q.
Antibody dissociation constants for antigens other than C1q may be least 5-fold,
at least 10-fold, at least 100-fold, at least 1,000-fold, at least 10,000-fold, or at least
100,000-fold higher that the dissociation constants for C1q. For example, the dissociation
constant of a humanized anti-C1q antibody of the present disclosure may be at least 1,000-
fold higher for C1s than for C1q. Dissociation constants may be determined through any
analytical technique, including any biochemical or biophysical technique such as ELISA,
surface plasmon resonance (SPR), bio-layer interferometry (see, e.g., Octet System by
ForteBio), isothermal titration calorimetry (ITC), differential scanning calorimetry (DSC),
circular dichroism (CD), stopped-flow analysis, and colorimetric or fluorescent protein
melting analyses. Dissociation constants (KD) of the anti-C1q antibodies for C1q may be
determined, e.g., using full-length antibodies or antibody fragments, such as Fab fragments.
One exemplary way of determining binding affinity of humanized antibodies to
C1q is by measuring binding affinity of monofunctional Fab fragments of the antibody. To
obtain monofunctional Fab fragments, an antibody (for example, IgG) can be cleaved with
papain or expressed recombinantly. The affinity of an Fab fragment of an antibody can be
determined by surface plasmon resonance (Biacore3000™ surface plasmon resonance
(SPR) system, Biacore.TM., INC, Piscataway N.J.) equipped with pre-immobilized
streptavidin sensor chips (SA) using HBS-EP running buffer (0.01M HEPES, pH 7.4, 0.15
NaCl, 3 mM EDTA, 0.005% v/v Surfactant P20). Biotinylated human C1q (or any other
C1q) can be diluted into HBS-EP buffer to a concentration of less than 0.5 μg/mL and
injected across the individual chip channels using variable contact times, to achieve two
ranges of antigen density, either 50-200 response units (RU) for detailed kinetic studies or
800-1,000 RU for screening assays. Regeneration studies have shown that 25 mM NaOH in
25% v/v ethanol effectively removes the bound Fab while keeping the activity of C1q on
the chip for over 200 injections. Typically, serial dilutions (spanning concentrations of 0.1-
10.times. estimated KD) of purified Fab samples are injected for 1 min at 100 μL/minute
and dissociation times of up to 2 hours are allowed. The concentrations of the Fab proteins
are determined by ELISA and/or SDS-PAGE electrophoresis using a Fab of known
concentration (as determined by amino acid analysis) as a standard. Kinetic association
rates (kon) and dissociation rates (koff) are obtained simultaneously by fitting the data
globally to a 1:1 Langmuir binding model (Karlsson, R. Roos, H. Fagerstam, L. Petersson,

B. (1994). Methods Enzymology 6. 99-110) using the BIAevaluation program. Equilibrium
dissociation constant (K_{D}) values are calculated as k_{off}/kₒₙ. This protocol is suitable for use
in determining binding affinity of an antibody to any C1q, including human C1q, C1q of
another mammal (such as mouse C1q, rat C1q, primate C1q), as well as different forms of
C1q. Binding affinity of an antibody is generally measured at 25°C, but can also be
measured at 37°C.
The humanized antibodies of the present disclosure may bind to C1q antigens
derived from any organism having a complement system, including any mammalian
organism such as human, mouse, rat, rabbit, monkey, dog, cat, cow, horse, camel, sheep,
goat, or pig. In some embodiments, the anti-C1q antibodies bind specifically to epitopes on
human C1q. In some embodiments, the anti-C1q antibodies specifically bind to epitopes on
both human and mouse C1q. In some embodiments, the anti-C1q antibodies specifically
bind to epitopes on human, mouse, and rat C1q.
In some embodiments, provided herein is a humanized anti-C1q antibody that
binds to an epitope of C1q that is the same as or overlaps with the C1q epitope bound by
another antibody of this disclosure. In certain embodiments, provided herein is a
humanized anti-C1q antibody that binds to an epitope of C1q that is the same as or overlaps
with the C1q epitope bound by anti-C1q antibody M1 produced by the hybridoma cell line
with ATCC Accession Number PTA-120399. In some embodiments, the humanized anti-
C1q antibody competes with another antibody of this disclosure for binding to C1q. In
certain embodiments, the anti-C1q antibody competes with anti-C1q antibody M1 produced
by the hybridoma cell line with ATCC Accession Number PTA-120399 or anti-C1q
binding fragments thereof.
Methods that may be used to determine which C1q epitope of a humanized anti-
C1q antibody binds to, or whether two antibodies bind to the same or an overlapping
epitope, may include, without limitation, X-ray crystallography, NMR spectroscopy,
Alanine-Scanning Mutagenesis, the screening of peptide libraries that include C1q-derived
peptides with overlapping C1q sequences, and competition assays. Competition assays are
especially useful to determine whether two antibodies bind the same epitope by recognizing
identical or sterically overlapping epitopes or whether one antibody competitively inhibits
binding of another antibody to the antigen. These assays are known in the art. Typically,
an antigen or antigen expressing cells are immobilized on a multi-well plate and the ability

of unlabeled antibodies to block the binding of labeled antibodies is measured. Common
labels for such competition assays are radioactive labels or enzyme labels.
Competitive antibodies encompassed herein are humanized antibodies that
inhibit (i.e., prevent or interfere with in comparison to a control) or reduce the binding of
any anti-C1q antibody of this disclosure (such as M1 or an antigen-binding fragment of
M1) to C1q by at least 50%, 60%, 70%, 80%, 90% and 95% at 1 μM or less. For example,
the concentration competing antibody in the competition assay may be at or below the K_{D}
of antibody M1 or an antigen-binding fragment of M1. Competition between binding
members may be readily assayed in vitro for example using ELISA and/or by monitoring
the interaction of the antibodies with C1q in solution. The exact means for conducting the
analysis is not critical. C1q may be immobilized to a 96-well plate or may be placed in a
homogenous solution. In specific embodiments, the ability of unlabeled candidate
antibody(ies) to block the binding of the labeled anti-C1q antibody, e.g. M1, can be
measured using radioactive, enzyme or other labels. In the reverse assay, the ability of
unlabeled antibodies to interfere with the interaction of a labeled anti-C1q antibody with
C1q wherein said labeled anti-C1q antibody, e.g., M1, and C1q are already bound is
determined. The readout is through measurement of bound label. C1q and the candidate
antibody(ies) may be added in any order or at the same time.
In some embodiments, the humanized anti-C1q antibody inhibits the interaction
between C1q and an autoantibody.
In some embodiments, a humanized anti-C1q antibody of the present disclosure
binds essentially the same C1q epitope as antibody M1 produced by the hybridoma cell line
with ATCC Accession Number PTA-120399 or anti-C1q binding fragments thereof.
In some embodiments, the humanized anti-C1q antibody is an antibody, or an
antigen-binding fragment thereof, comprising a heavy chain variable domain comprising an
amino acid sequence selected from SEQ ID NOs: 1-4, or an amino acid sequence with at
least about 70%, at least about 75%, at least about 80% at least about 85% at least about
90%, or at least about 95% homology to the amino acid sequence selected from SEQ ID
NOs: 1-4. In some embodiments, the humanized anti-C1q antibody is an antibody, or an
antigen-binding fragment thereof, comprising a light chain variable domain comprising an
amino acid sequence selected from SEQ ID NOs: 5-8, or an amino acid sequence with at
least about 70%, at least about 75%, at least about 80% at least about 85% at least about
90%, or at least about 95% homology to the amino acid sequence selected from SEQ ID

NOs: 5-8. In some embodiments, the humanized anti-C1q antibody is an antibody, or an
antigen-binding fragment thereof, comprising a heavy chain variable domain comprising an
amino acid sequence selected from SEQ ID NOs: 1-4, or an amino acid sequence with at
least about 70%, at least about 75%, at least about 80% at least about 85% at least about
90%, or at least about 95% homology to the amino acid sequence selected from SEQ ID
NOs: 1-4; and/or a light chain variable domain comprising an amino acid sequence selected
from SEQ ID NOs: 5-8, or an amino acid sequence with at least about 70%, at least about
75%, at least about 80% at least about 85% at least about 90%, or at least about 95%
homology to the amino acid sequence selected from SEQ ID NOs: 5-8.
In some embodiments, the humanized anti-C1q antibody, or an antigen-binding
fragment thereof, comprises a heavy chain variable domain comprising the amino acid
sequence of SEQ ID NO: 1, or an amino acid sequence with at least about 70%, at least
about 75%, at least about 80% at least about 85% at least about 90%, or at least about 95%
homology to the amino acid sequence of SEQ ID NO: 1; and a light chain variable domain
comprising the amino acid sequence of SEQ ID NO: 5, or an amino acid sequence with at
least about 70%, at least about 75%, at least about 80% at least about 85% at least about
90%, or at least about 95% homology to the amino acid sequence of SEQ ID NO: 5. In
some embodiments, the humanized anti-C1q antibody, or an antigen-binding fragment
thereof, comprises a heavy chain variable domain comprising the amino acid sequence of
SEQ ID NO: 1, or an amino acid sequence with at least about 70%, at least about 75%, at
least about 80% at least about 85% at least about 90%, or at least about 95% homology to
the amino acid sequence of SEQ ID NO: 1; and a light chain variable domain comprising
the amino acid sequence of SEQ ID NO: 6, or an amino acid sequence with at least about
70%, at least about 75%, at least about 80% at least about 85% at least about 90%, or at
least about 95% homology to the amino acid sequence of SEQ ID NO: 6. In some
embodiments, the humanized anti-C1q antibody, or an antigen-binding fragment thereof,
comprises a heavy chain variable domain comprising the amino acid sequence of SEQ ID
NO: 1, or an amino acid sequence with at least about 70%, at least about 75%, at least
about 80% at least about 85% at least about 90%, or at least about 95% homology to the
amino acid sequence of SEQ ID NO: 1; and a light chain variable domain comprising the
amino acid sequence of SEQ ID NO: 7, or an amino acid sequence with at least about 70%,
at least about 75%, at least about 80% at least about 85% at least about 90%, or at least
about 95% homology to the amino acid sequence of SEQ ID NO: 7. In some embodiments,

the humanized anti-C1q antibody, or an antigen-binding fragment thereof, comprises a
heavy chain variable domain comprising the amino acid sequence of SEQ ID NO: 1, or an
amino acid sequence with at least about 70%, at least about 75%, at least about 80% at least
about 85% at least about 90%, or at least about 95% homology to the amino acid sequence
of SEQ ID NO: 1; and a light chain variable domain comprising the amino acid sequence of
SEQ ID NO: 8, or an amino acid sequence with at least about 70%, at least about 75%, at
least about 80% at least about 85% at least about 90%, or at least about 95% homology to
the amino acid sequence of SEQ ID NO: 8.
In some embodiments, the humanized anti-C1q antibody, or an antigen-binding
fragment thereof, comprises a heavy chain variable domain comprising the amino acid
sequence of SEQ ID NO: 2, or an amino acid sequence with at least about 70%, at least
about 75%, at least about 80% at least about 85% at least about 90%, or at least about 95%
homology to the amino acid sequence of SEQ ID NO: 2; and a light chain variable domain
comprising the amino acid sequence of SEQ ID NO: 5, or an amino acid sequence with at
least about 70%, at least about 75%, at least about 80% at least about 85% at least about
90%, or at least about 95% homology to the amino acid sequence of SEQ ID NO: 5.  In
some embodiments, the humanized anti-C1q antibody, or an antigen-binding fragment
thereof, comprises a heavy chain variable domain comprising the amino acid sequence of
SEQ ID NO: 2, or an amino acid sequence with at least about 70%, at least about 75%, at
least about 80% at least about 85% at least about 90%, or at least about 95% homology to
the amino acid sequence of SEQ ID NO: 2; and a light chain variable domain comprising
the amino acid sequence of SEQ ID NO: 6, or an amino acid sequence with at least about
70%, at least about 75%, at least about 80% at least about 85% at least about 90%, or at
least about 95% homology to the amino acid sequence of SEQ ID NO: 6.  In some
embodiments, the humanized anti-C1q antibody, or an antigen-binding fragment thereof,
comprises a heavy chain variable domain comprising the amino acid sequence of SEQ ID
NO: 2, or an amino acid sequence with at least about 70%, at least about 75%, at least
about 80% at least about 85% at least about 90%, or at least about 95% homology to the
amino acid sequence of SEQ ID NO: 2; and a light chain variable domain comprising the
amino acid sequence of SEQ ID NO: 7, or an amino acid sequence with at least about 70%,
at least about 75%, at least about 80% at least about 85% at least about 90%, or at least
about 95% homology to the amino acid sequence of SEQ ID NO: 7.  In some embodiments,
the humanized anti-C1q antibody, or an antigen-binding fragment thereof, comprises a

heavy chain variable domain comprising the amino acid sequence of SEQ ID NO: 2, or an
amino acid sequence with at least about 70%, at least about 75%, at least about 80% at least
about 85% at least about 90%, or at least about 95% homology to the amino acid sequence
of SEQ ID NO: 2; and a light chain variable domain comprising the amino acid sequence of
SEQ ID NO: 8, or an amino acid sequence with at least about 70%, at least about 75%, at
least about 80% at least about 85% at least about 90%, or at least about 95% homology to
the amino acid sequence of SEQ ID NO: 8.
In some embodiments, the humanized anti-C1q antibody, or an antigen-binding
fragment thereof, comprises a heavy chain variable domain comprising the amino acid
sequence of SEQ ID NO: 3, or an amino acid sequence with at least about 70%, at least
about 75%, at least about 80% at least about 85% at least about 90%, or at least about 95%
homology to the amino acid sequence of SEQ ID NO: 3; and a light chain variable domain
comprising the amino acid sequence of SEQ ID NO: 5, or an amino acid sequence with at
least about 70%, at least about 75%, at least about 80% at least about 85% at least about
90%, or at least about 95% homology to the amino acid sequence of SEQ ID NO: 5.  In
some embodiments, the humanized anti-C1q antibody, or an antigen-binding fragment
thereof, comprises a heavy chain variable domain comprising the amino acid sequence of
SEQ ID NO: 3, or an amino acid sequence with at least about 70%, at least about 75%, at
least about 80% at least about 85% at least about 90%, or at least about 95% homology to
the amino acid sequence of SEQ ID NO: 3; and a light chain variable domain comprising
the amino acid sequence of SEQ ID NO: 6, or an amino acid sequence with at least about
70%, at least about 75%, at least about 80% at least about 85% at least about 90%, or at
least about 95% homology to the amino acid sequence of SEQ ID NO: 6.  In some
embodiments, the humanized anti-C1q antibody, or an antigen-binding fragment thereof,
comprises a heavy chain variable domain comprising the amino acid sequence of SEQ ID
NO: 3, or an amino acid sequence with at least about 70%, at least about 75%, at least
about 80% at least about 85% at least about 90%, or at least about 95% homology to the
amino acid sequence of SEQ ID NO: 3; and a light chain variable domain comprising the
amino acid sequence of SEQ ID NO: 7, or an amino acid sequence with at least about 70%,
at least about 75%, at least about 80% at least about 85% at least about 90%, or at least
about 95% homology to the amino acid sequence of SEQ ID NO: 7.  In some embodiments,
the humanized anti-C1q antibody, or an antigen-binding fragment thereof, comprises a
heavy chain variable domain comprising the amino acid sequence of SEQ ID NO: 3, or an

amino acid sequence with at least about 70%, at least about 75%, at least about 80% at least
about 85% at least about 90%, or at least about 95% homology to the amino acid sequence
of SEQ ID NO: 3; and a light chain variable domain comprising the amino acid sequence of
SEQ ID NO: 8, or an amino acid sequence with at least about 70%, at least about 75%, at
least about 80% at least about 85% at least about 90%, or at least about 95% homology to
the amino acid sequence of SEQ ID NO: 8.
In some embodiments, the humanized anti-C1q antibody, or an antigen-binding
fragment thereof, comprises a heavy chain variable domain comprising the amino acid
sequence of SEQ ID NO: 4, or an amino acid sequence with at least about 70%, at least
about 75%, at least about 80% at least about 85% at least about 90%, or at least about 95%
homology to the amino acid sequence of SEQ ID NO: 4; and a light chain variable domain
comprising the amino acid sequence of SEQ ID NO: 5, or an amino acid sequence with at
least about 70%, at least about 75%, at least about 80% at least about 85% at least about
90%, or at least about 95% homology to the amino acid sequence of SEQ ID NO: 5. In
some embodiments, the humanized anti-C1q antibody, or an antigen-binding fragment
thereof, comprises a heavy chain variable domain comprising the amino acid sequence of
SEQ ID NO: 4, or an amino acid sequence with at least about 70%, at least about 75%, at
least about 80% at least about 85% at least about 90%, or at least about 95% homology to
the amino acid sequence of SEQ ID NO: 4; and a light chain variable domain comprising
the amino acid sequence of SEQ ID NO: 6, or an amino acid sequence with at least about
70%, at least about 75%, at least about 80% at least about 85% at least about 90%, or at
least about 95% homology to the amino acid sequence of SEQ ID NO: 6. In some
embodiments, the humanized anti-C1q antibody, or an antigen-binding fragment thereof,
comprises a heavy chain variable domain comprising the amino acid sequence of SEQ ID
NO: 4, or an amino acid sequence with at least about 70%, at least about 75%, at least
about 80% at least about 85% at least about 90%, or at least about 95% homology to the
amino acid sequence of SEQ ID NO: 4; and a light chain variable domain comprising the
amino acid sequence of SEQ ID NO: 7, or an amino acid sequence with at least about 70%,
at least about 75%, at least about 80% at least about 85% at least about 90%, or at least
about 95% homology to the amino acid sequence of SEQ ID NO: 7. In some embodiments,
the humanized anti-C1q antibody, or an antigen-binding fragment thereof, comprises a
heavy chain variable domain comprising the amino acid sequence of SEQ ID NO: 4, or an
amino acid sequence with at least about 70%, at least about 75%, at least about 80% at least

about 85% at least about 90%, or at least about 95% homology to the amino acid sequence
of SEQ ID NO: 4; and a light chain variable domain comprising the amino acid sequence of
SEQ ID NO: 8, or an amino acid sequence with at least about 70%, at least about 75%, at
least about 80% at least about 85% at least about 90%, or at least about 95%homology to
the amino acid sequence of SEQ ID NO: 8.
In some embodiments, humanized anti-C1q antibodies of the present disclosure
may comprise at least one HVR selected from HVR-L1, HVR-L2, and HVR-L3 of the light
chain variable domains of monoclonal antibody M1 produced by the hybridoma cell line
having ATCC Accession Number PTA-120399, or progeny thereof. In some embodiments,
humanized anti-C1q antibodies of the present disclosure may comprise at least one HVR
selected from HVR-H1, HVR-H2, and HVR-H3 of the heavy chain variable domains of
monoclonal antibody M1 produced by the hybridoma cell line having ATCC Accession
Number PTA-120399, or progeny thereof. In some embodiments, humanized anti-C1q
antibodies of the present disclosure may comprise at least one HVR selected from HVR-L1,
HVR-L2, and HVR-L3 of the light chain variable domains and at least one HVR selected
from HVR-H1, HVR-H2, and HVR-H3 of the heavy chain variable domains of monoclonal
antibody M1 produced by the hybridoma cell line having ATCC Accession Number PTA-
120399, or progeny thereof.
In some embodiments, humanized anti-C1q antibodies of the present disclosure
may bind to a C1q protein and binds to one or more amino acids of the C1q protein within
amino acid residues selected from (a) amino acid residues 196-226 of SEQ ID NO: 9 (SEQ
ID NO:12), or amino acid residues of a C1q protein chain A (C1qA) corresponding to
amino acid residues 196-226 (GLFQVVSGGMVLQLQQGDQVWVEKDPKKGHI) of
SEQ ID NO: 9 (SEQ ID NO:12); (b) amino acid residues 196-221 of SEQ ID NO: 9 (SEQ
ID NO:13), or amino acid residues of a C1qA corresponding to amino acid residues 196-
221 (GLFQVVSGGMVLQLQQGDQVWVEKDP) of SEQ ID. NO: 9 (SEQ ID NO:13); (c)
amino acid residues 202-221 of SEQ ID NO: 9 (SEQ ID NO:14), or amino acid residues of
a C1qA corresponding to amino acid residues 202-221
(SGGMVLQLQQGDQVWVEKDP) of SEQ ID NO: 9 (SEQ ID NO:14); (d) amino acid
residues 202-219 of SEQ ID NO: 9 (SEQ ID NO:15), or amino acid residues of a C1qA
corresponding to amino acid residues 202-219 (SGGMVLQLQQGDQVWVEK) of SEQ ID
NO: 9 (SEQ ID NO:15); and (e) amino acid residues Lys 219 and/or Ser 202 of SEQ ID

NO: 9, or amino acid residues of a C1qA corresponding Lys 219 and/or Ser 202 of SEQ ID
NO: 9.
In some embodiments, the humanized anti-C1q antibodies may further binds to
one or more amino acids of the C1q protein within amino acid residues selected from: (a)
amino acid residues 218-240 of SEQ ID NO: 11 (SEQ ID NO:16) or amino acid residues of
a C1q protein chain C (C1qC) corresponding to amino acid residues 218-240
(WLAVNDYYDMVGI QGSDSVFSGF) of SEQ ID NO: 11 (SEQ ID NO:16); (b) amino
acid residues 225-240 of SEQ ID NO: 11 (SEQ ID NO:17) or amino acid residues of a
C1qC corresponding to amino acid residues 225-240 (YDMVGI QGSDSVFSGF) of SEQ
ID NO: 11 (SEQ ID NO:17); (c) amino acid residues 225-232 of SEQ ID NO: 11 (SEQ ID
NO:18) or amino acid residues of a C1qC corresponding to amino acid residues 225-232
(YDMVGIQG) of SEQ ID NO: 11 (SEQ ID NO:18); (d) amino acid residue Tyr 225 of
SEQ ID NO: 11 or an amino acid residue of a C1qC corresponding to amino acid residue
Tyr 225 of SEQ ID NO: 11; (e) amino acid residues 174-196 of SEQ ID NO: 11 (SEQ ID
NO:19) or amino acid residues of a C1qC corresponding to amino acid residues 174-196
(HTANLCVLLYRSGVKVVTFCGHT) of SEQ ID NO: 11 (SEQ ID NO:19); (f) amino
acid residues 184-192 of SEQ ID NO: 11 (SEQ ID NO:20) or amino acid residues of a
C1qC corresponding to amino acid residues 184-192 (RSGVKVVTF) of SEQ ID NO: 11
(SEQ ID NO:20); (g) amino acid residues 185-187 of SEQ ID NO: 11 or amino acid
residues of a C1qC corresponding to amino acid residues 185-187 (SGV) of SEQ ID NO:
11; (h) amino acid residue Ser 185 of SEQ ID NO: 11 or an amino acid residue of a C1qC
corresponding to amino acid residue Ser 185 of SEQ ID NO: 11.
In certain embodiments, humanized anti-C1q antibodies of the present
disclosure may bind to amino acid residue Lys 219 and Ser 202 of the human C1qA as
shown in SEQ ID NO: 9 or amino acids of a human C1qA corresponding to Lys 219 and
Ser 202 as shown in SEQ ID NO: 9, and amino acid residue Tyr 225 of the human C1qC as
shown in SEQ ID NO: 11 or an amino acid residue of a human C1qC corresponding to Tyr
225 as shown in SEQ ID NO: 11.  In certain embodiments, the anti-C1q antibody binds to
amino acid residue Lys 219 of the human C1qA as shown in SEQ ID NO: 9 or an amino
acid residue of a human C1qA corresponding to Lys 219 as shown in SEQ ID NO: 9, and
amino acid residue Ser 185 of the human C1qC as shown in SEQ ID NO: 11 or an amino
acid residue of a human C1qC corresponding to Ser 185 as shown in SEQ ID NO: 11.

In some embodiments, humanized anti-C1q antibodies of the present disclosure
may bind to a C1q protein and binds to one or more amino acids of the C1q protein within
amino acid residues selected from: (a) amino acid residues 218-240 of SEQ ID NO: 11
(SEQ ID NO:16) or amino acid residues of a C1qC corresponding to amino acid residues
218-240 (WLAVNDYYDMVGI QGSDSVFSGF) of SEQ ID NO: 11 (SEQ ID NO:16); (b)
amino acid residues 225-240 of SEQ ID NO: 11 (SEQ ID NO:17) or amino acid residues of
a C1qC corresponding to amino acid residues 225-240 (YDMVGI QGSDSVFSGF) of SEQ
ID NO: 11 (SEQ ID NO:17); (c) amino acid residues 225-232 of SEQ ID NO: 11 (SEQ ID
NO:18) or amino acid residues of a C1qC corresponding to amino acid residues 225-232
(YDMVGIQG) of SEQ ID NO: 11 (SEQ ID NO:18); (d) amino acid residue Tyr 225 of
SEQ ID NO: 11 or an amino acid residue of a C1qC corresponding to amino acid residue
Tyr 225 of SEQ ID NO: 11; (e) amino acid residues 174-196 of SEQ ID NO: 11 (SEQ ID
NO:19) or amino acid residues of a C1qC corresponding to amino acid residues 174-196
(HTANLCVLLYRSGVKVVTFCGHT) of SEQ ID NO: 11 (SEQ ID NO:19); (f) amino
acid residues 184-192 of SEQ ID NO: 11 (SEQ ID NO:20) or amino acid residues of a
C1qC corresponding to amino acid residues 184-192 (RSGVKVVTF) of SEQ ID NO: 11
(SEQ ID NO:20); (g) amino acid residues 185-187 of SEQ ID NO: 11 or amino acid
residues of a C1qC corresponding to amino acid residues 185-187 (SGV) of SEQ ID NO:
11; (h) amino acid residue Ser 185 of SEQ ID NO: 11 or an amino acid residue of a C1qC
corresponding to amino acid residue Ser 185 of SEQ ID NO: 11.
In some embodiments, a humanized anti-C1q antibody of the present disclosure
inhibits the interaction between C1q and C1s. In some embodiments, the humanized anti-
C1q antibody inhibits the interaction between C1q and C1r. In some embodiments the
humanized anti-C1q antibody inhibits the interaction between C1q and C1s and between
C1q and C1r. In some embodiments, the humanized anti-C1q antibody inhibits the
interaction between C1q and another antibody, such as an autoantibody. In some
embodiments, the humanized anti-C1q antibody inhibits the respective interactions, at a
stoichiometry of less than 2.5:1; 2.0:1; 1.5:1; or 1.0:1. In some embodiments, the
humanized C1q antibody inhibits an interaction, such as the C1q-C1s interaction, at
approximately equimolar concentrations of C1q and the anti-C1q antibody. In other
embodiments, the anti-C1q antibody binds to C1q with a stoichiometry of less than 20:1;
less than 19.5:1; less than19:1; less than 18.5:1; less than 18:1; less than 17.5:1; less than
17:1; less than 16.5:1; less than 16:1; less than 15.5:1; less than 15:1; less than 14.5:1; less

than 14:1; less than 13.5:1; less than 13:1; less than 12.5:1; less than 12:1; less than 11.5:1;
less than 11:1; less than 10.5:1; less than 10:1; less than 9.5:1; less than 9:1; less than 8.5:1;
less than 8:1; less than 7.5:1; less than 7:1; less than 6.5:1; less than 6:1; less than 5.5:1;
less than 5:1; less than 4.5:1; less than 4:1; less than 3.5:1; less than 3:1; less than 2.5:1;
less than 2.0:1; less than 1.5:1; or less than 1.0:1. In certain embodiments, the humanized
anti-C1q antibody binds C1q with a binding stoichiometry that ranges from 20:1 to 1.0:1 or
less than1.0:1. In certain embodiments, the humanized anti-C1q antibody binds C1q with a
binding stoichiometry that ranges from 6:1 to 1.0:1 or less than1.0:1. In certain
embodiments, the humanized anti-C1q antibody binds C1q with a binding stoichiometry
that ranges from 2.5:1 to 1.0:1 or less than1.0:1. In some embodiments, an anti-C1q
antibody of the present disclosure having a binding stoichiometry for C1q of 1.0:1 yeilds
approximately 50% inhibition of C1F hemolysis, as deptermined for example by CH50
assays of the present disclosure. In some embodiments, the humanized anti-C1q antibody
inhibits the interaction between C1q and C1r, or between C1q and C1s, or between C1q and
both C1r and C1s. In some embodiments, the humanized anti-C1q antibody inhibits the
interaction between C1q and C1r, between C1q and C1s, and/or between C1q and both C1r
and C1s. In some embodiments, the humanized anti-C1q antibody binds to the C1q A-
chain. In other embodiments, the humanized anti-C1q antibody binds to the C1q B-chain.
In other embodiments, the humanized anti-C1q antibody binds to the C1q C-chain. In some
embodiments, the humanized anti-C1q antibody binds to the C1q A-chain, the C1q B-chain
and/or the C1q C-chain. In some embodiments, the humanized anti-C1q antibody binds to
the globular domain of the C1q A-chain, B-chain, and/or C-chain. In other embodiments,
the humanized anti-C1q antibody binds to the collagen-like domain of the C1q A-chain, the
C1q B-chain, and/or the C1q C-chain.
Where humanized antibodies of this disclosure inhibit the interaction between
two or more complement factors, such as the interaction of C1q and C1s, or the interaction
between C1q and C1r, the interaction occurring in the presence of the antibody may be
reduced by at least 10%, at least 20%, at least 30%, at least 40%, at least 50%, at least 60%,
at least 70%, at least 80%, at least 90%, at least 95%, or at least 99% relative to a control
wherein the antibodies of this disclosure are absent. In certain embodiments, the
interaction occurring in the presence of the humanized antibody is reduced by an amount
that ranges from at least 30% to at least 99% relative to a control wherein the humanized
antibodies of this disclosure are absent.

In some embodiments, humanized anti-C1q antibodies of the present disclosure
inhibit C4-cleavage by at least 20%, at least 30%, at least 40%, at least 50%, at least 60%,
at least 70%, at least 80%, at least 90%, at least 95%, or at least 99%, or by an amount that
ranges from at least 30% to at least 99%, relative to a control wherein the antibodies of this
disclosure are absent. Methods for measuring C4-cleavage are well known in the art. The
EC50 values for antibodies of this disclosure with respect C4-cleavage may be less than 3
μg/ml; 2.5 μg/ml; 2.0 μg/ml; 1.5 μg/ml; 1.0 μg/ml; 0.5 μg/ml; 0.25 μg/ml; 0.1 μg/ml; 0.05
μg/ml. In some embodiments, the antibodies of this disclosure inhibit C4-cleavage at
approximately equimolar concentrations of C1q and the respective anti-C1q antibody.
In some embodiments, humanized anti-C1q antibodies of the present disclosure
inhibit autoantibody-dependent and complement-dependent cytotoxicity (CDC) by at least
20%, at least 30%, at least 40%, at least 50%, at least 60%, at least 70%, at least 80%, at
least 90%, at least 95%, or at least 99%, or by an amount that ranges from at least 30% to at
least 99%, relative to a control wherein the antibodies of this disclosure are absent. The
EC50 values for antibodies of this disclosure with respect to inhibition of autoantibody-
dependent and complement-dependent cytotoxicity may be less than 3 μg/ml; 2.5 μg/ml;
2.0 μg/ml; 1.5 μg/ml; 1.0 μg/ml; 0.5 μg/ml; 0.25 μg/ml; 0.1 μg/ml; 0.05 μg/ml.
In some embodiments, humanized anti-C1q antibodies of the present disclosure
inhibit complement-dependent cell-mediated cytotoxicity (CDCC) by at least 20%, at least
30%, at least 40%, at least 50%, at least 60%, at least 70%, at least 80%, at least 90%, at
least 95%, or at least 99%, or by an amount that ranges from at least 30% to at least 99%,
relative to a control wherein the antibodies of this disclosure are absent. Methods for
measuring CDCC are well known in the art. The EC50 values for antibodies of this
disclosure with respect CDCC inhibition may be l less than 3 μg/ml; 2.5 μg/ml; 2.0 μg/ml;
1.5 μg/ml; 1.0 μg/ml; 0.5 μg/ml; 0.25 μg/ml; 0.1 μg/ml; 0.05 μg/ml. In some
embodiments, the antibodies of this disclosure inhibit CDCC but not antibody-dependent
cellular cytotoxicity (ADCC).
In some embodiments, humanized anti-C1q antibodies of the present disclosure
inhibit C1F hemolysis (also referred to as CH50 hemolysis) by at least 20%, at least 30%,
at least 40%, at least 50%, at least 60%, at least 70%, at least 80%, at least 90%, at least
95%, or at least 99%, or by an amount that ranges from at least 30% to at least 99%,relative
to a control wherein the antibodies of this disclosure are absent or wherein control
antibodies are used that do not bind to a complement factor or another antibody such as an

autoantibody (see, e.g., Examples section below). Methods for measuring C1F hemolysis
are well known in the art (see, e.g., Examples section below). The EC50 values for
humanized antibodies of this disclosure with respect to C1F hemolysis may be less than 3
μg/ml; 2.5 μg/ml; 2.0 μg/ml; 1.5 μg/ml; 1.0 μg/ml; 0.5 μg/ml; 0.25 μg/ml; 0.1 μg/ml; 0.05
μg/ml. In some embodiments, humanized anti-C1q antibodies of this disclosure neutralize
at least 50% of C1F hemolysis at a dose of less than 200 ng/ml, less than100 ng/ml, less
than 50 ng/ml, or less than 20 ng/ml. In some embodiments, humanized antibodies of this
disclosure neutralize C1F hemolysis at approximately equimolar concentrations of C1q and
the anti-C1q antibody. In some embodiments, humanized anti-C1q antibodies of this
disclosure neutralize hemolysis in a human C1F hemolysis assay. In some embodiments,
humanized anti-C1q antibodies of this disclosure neutralize hemolysis in a human and rat
C1F hemolysis assay (see, e.g., see, e.g., Examples section below).
In some embodiments, the alternative pathway may amplify CDC initiated by
C1q binding and subsequent C1s activation; in at least some of these embodiments, the
antibodies of this disclosure inhibit the alternative pathway by at least 30%, at least 40%, at
least 50%, at least 60%, at least 70%, at least 80%, at least 90%, at least 95%, or at least
99%, or by an amount that ranges from at least 30% to at least 99%, relative to a control
wherein the antibodies of this disclosure were absent.
In some embodiments, humanized anti-C1q antibodies of the present disclosure
prevent synaptic loss in a cellular in vitro model or an in vivo model of synaptic loss, such
as an in vivo mouse model. In vivo mouse models may include Tg2576, a mouse amyloid
precursor protein (APP) transgenic model of Alzheimer's disease, R6/2 NT-CAG150, a
transgenic model for Huntington's disease, or SMAΔ7, a mouse model for Spinal Muscular
Atrophy, or DBA/2J, a genetic mouse model of glaucoma. In general, any
neurodegenerative disease model may be used that displays synapse loss.
Methods for measuring synaptic loss in vitro or in vivo are well known in the
art. In vitro lesion formation may be reduced by at least 30%, at least 40%, at least 50%, at
least 60%, at least 70%, at least 80%, at least 90%, or at least 95%, or by an amount that
ranges from at least 30% to at least 95%, relative to a control experiment in which
antibodies of this disclosure are absent. The EC50 values for antibodies of this disclosure
with respect to the prevention of in vitro lesion formation may be less than 3 μg/ml; 2.5
μg/ml; 2.0 μg/ml; 1.5 μg/ml; 1.0 μg/ml; 0.5 μg/ml; 0.25 μg/ml; 0.1 μg/ml; 0.05 μg/ml. In
vivo synaptic loss may be reduced by at least 5%, at least 10%, at least 15%, at least 20%,

at least 35%, at least 40%, or at least 50%, or by an amount that ranges from at least 5% to
at least 50%, relative to a control experiment in which antibodies of this disclosure are
absent.
In some embodiments, humanized anti-C1q antibodies of the present disclosure
prevent lesion formation in an ex vivo spinal cord slice model of NMO or in an in vivo
mouse model of NMO. Methods for measuring lesion formation ex vivo or in vivo are well
known in the art. Ex vivo lesion formation may be reduced at least by a relative score of
0.5, 1.0, 1.5, 2.0, 2.5, 3.0, 3.5, or 4.0. The EC50 values for antibodies of this disclosure with
respect to the prevention of ex vivo lesion formation may be less than 3μg/ml; less than 2.5
μg/ml; less than 2.0 μg/ml; less than 1.5 μg/ml; less than 1.0 μg/ml; less than 0.5 μg/ml;
less than 0.25 μg/ml; less than 0.1 μg/ml; or less than 0.05 μg/ml. In vivo lesion formation
may be reduced by at least 5%, at least 10%, at least 15%, at least 20%, at least 35%, at
least 40%, or at least 50%, or by an amount that ranges from at least 5% to at least 50%, in
terms of loss of staining (% of area). Staining may be assessed, without limitation, by
APQ4 staining, GFAP staining, or MBP staining.
The present disclosure provides humanized anti-C1q antibodies. The
humanized antibodies of the present disclosure may have one or more of the following
characteristics. The antibodies of this disclosure may be polyclonal antibodies, monoclonal
antibodies, chimeric antibodies, human antibodies, antibody fragments, bispecific and
polyspecific antibodies, multivalent antibodies, or heteroconjugate antibodies. Antibody
fragments of this disclosure may be functional fragments that bind the same epitope as any
of the humanized anti-C1q antibodies of this disclosure. In some embodiments, the
antibody fragments of this disclosure specifically bind to and neutralize a biological activity
of C1q. In some embodiments, the antibody fragments are miniaturized versions of the
humanized anti-C1q antibodies or antibody fragments of this disclosure that have the same
epitope of the corresponding full-length antibody, but have much smaller molecule weight.
Such miniaturized anti-C1q antibody fragments may have better brain penetration ability
and a shorter half-life, which is advantageous for imaging and diagnostic utilities (see e.g.,
Lütje S et al., Bioconjug Chem. 2014 Feb 19;25(2):335-41; Tavaré R et al., Proc Natl Acad
Sci U S A. 2014 Jan 21;111(3):1108-13; and Wiehr S et al., Prostate. 2014 May;74(7):743-
55). Accordingly, in some embodiments, humanized anti-C1q antibody fragments of this
disclosure have better brain penetration as compared to their corresponding full-length
antibodies and/or have a shorter half-life as compared to their corresponding full-length

antibodies. In some embodiments, humanized anti-C1q antibodies of the present disclosure
are bispecific antibodies recognizing a first antigen and a second antigen. In some
embodiments, the first antigen is a C1q antigen. In some embodiments, the second antigen
is an antigen facilitating transport across the blood-brain-barrier, including without
limitation, transferrin receptor (TR), insulin receptor (HIR), insulin-like growth factor
receptor (IGFR), low-density lipoprotein receptor related proteins 1 and 2 (LPR-1 and 2),
diphtheria toxin receptor, CRM197, a llama single domain antibody, TMEM 30(A), a
protein transduction domain, TAT, Syn-B, penetratin, a poly-arginine peptide, an angiopep
peptide, and ANG1005.
Humanized anti-C1q antibodies of the present disclosure may further contain
engineered effector functions, amino acid sequence modifications or other antibody
modifications known in the art; e.g., the constant region of the anti-C1q antibodies
described herein may be modified to impair complement activation. For example, and
without wishing to be bound by theory, unlike the Fc region of human IgG1, IgG2, and
IgG3, the Fc region of human IgG4 does not bind to C1q. Accordingly, in some
embodiments, humanized anti-C1q antibodies of this disclosure may further comprise the
Fc region of human IgG4. In some embodiments, humanized anti-C1q antibodies of this
disclosure comprise one or more amino acid substitutions within the Fc region that, for
example, prevent arm switching and/or reduces or otherwise inhibits the ability of Fc region
from interacting with Fc receptors expressed on cells (see e.g., Angal S et al., Mol
Immunol. 1993 Jan;30(1):105-8; and Morgan A et al., Immunology 1995 86 319-324). In
some embodiments humanized anti-C1q antibodies of this disclosure comprise an Fc region
that comprises an amino acid substitution at position 241 or 248 according to Kabat
numbering convention. In some embodiments, the Fc region comprises a serine to proline
amino acid substitution at position 241 that prevent arm switching. In some embodiments,
the Fc region comprises a serine to proline amino acid substitution at position 241
according to Kabat numbering convention. In some embodiments, the Fc region comprises
a serine to proline amino acid substitution at position 248 that reduces or otherwise inhibits
the ability of Fc region from interacting with an Fc receptor. In some embodiments, the Fc
region comprises a leucine to glutamate amino acid substitution at position 248 according
to Kabat numbering convention. In some embodiments humanized anti-C1q antibodies of
this disclosure comprise an Fc region comprising the amino acid sequence of SEQ ID NO:
37.

Additional humanized anti-C1q antibodies, e.g., humanized antibodies that
specifically bind to a C1q protein of the present disclosure, may be identified, screened,
and/or characterized for their physical/chemical properties and/or biological activities by
various assays known in the art.
Antibody preparation
Anti-C1q antibodies of the present disclosure may be produced using any
methods described herein or known in the art. Monoclonal antibodies (e.g., humanized
antibodies) of the of the present disclosure can be produced using a variety of known
techniques, such as the standard somatic cell hybridization technique described by Kohler
and Milstein, Nature 256: 495 (1975). Although somatic cell hybridization procedures are
preferred, in principle, other techniques for producing monoclonal antibodies also can be
employed, e.g., viral or oncogenic transformation of B lymphocytes and phage display
technique using libraries of human antibody genes.
One method for generating hybridomas which produce monoclonal antibodies of
the present disclosure is the murine system. Hybridoma production in the mouse is well-
known in the art, including immunization protocols and techniques for isolating and fusing
immunized splenocytes.
Polyclonal antibodies can be prepared by immunizing a suitable subject with a
polypeptide immunogen. The polypeptide antibody titer in the immunized subject can be
monitored over time by standard techniques, such as with an enzyme linked immunosorbent
assay (ELISA) using immobilized polypeptide. If desired, the antibody directed against the
antigen can be isolated from the mammal (e.g., from the blood) and further purified by
well-known techniques, such as protein A chromatography to obtain the IgG fraction. At
an appropriate time after immunization, e.g., when the antibody titers are highest, antibody-
producing cells can be obtained from the subject and used to prepare monoclonal antibodies
by standard techniques, such as the hybridoma technique originally described by Kohler
and Milstein (1975) Nature 256:495-497) (see also Brown et al. (1981) J. Immunol.
127:539-46; Brown et al. (1980) J. Biol. Chem. 255:4980-83; Yeh et al. (1976) Proc. Natl.
Acad. Sci. 76:2927-31; and Yeh et al. (1982) Int. J. Cancer 29:269-75), the more recent
human B cell hybridoma technique (Kozbor et al. (1983) Immunol. Today 4:72), the EBV-
hybridoma technique (Cole et al. (1985) Monoclonal Antibodies and Cancer Therapy, Alan
R. Liss, Inc., pp. 77-96) or trioma techniques. The technology for producing monoclonal
antibody hybridomas is well-known (see generally Kenneth, R. H. in Monoclonal

Antibodies: A New Dimension In Biological Analyses, Plenum Publishing Corp., New
York, New York (1980); Lerner, E. A. (1981) Yale J. Biol. Med. 54:387-402; Gefter, M. L.
et al. (1977) Somatic Cell Genet. 3:231-36). Briefly, an immortal cell line (typically a
myeloma) is fused to lymphocytes (typically splenocytes) from a mammal immunized with
an immunogen as described above, and the culture supernatants of the resulting hybridoma
cells are screened to identify a hybridoma producing a monoclonal antibody that binds to
the polypeptide antigen, preferably specifically.
Any of the many well-known protocols used for fusing lymphocytes and
immortalized cell lines can be applied for the purpose of generating an anti-PD-1, PD-L1,
or PD-L2_monoclonal antibody (see, e.g., Galfre, G. et al. (1977) Nature 266:55052; Gefter
et al. (1977) supra; Lerner (1981) supra; Kenneth (1980) supra). Moreover, the ordinary
skilled worker will appreciate that there are many variations of such methods which also
would be useful. Typically, the immortal cell line (e.g., a myeloma cell line) is derived
from the same mammalian species as the lymphocytes. For example, murine hybridomas
can be made by fusing lymphocytes from a mouse immunized with an immunogenic
preparation of the present disclosure with an immortalized mouse cell line. Preferred
immortal cell lines are mouse myeloma cell lines that are sensitive to culture medium
containing hypoxanthine, aminopterin and thymidine ("HAT medium"). Any of a number
of myeloma cell lines can be used as a fusion partner according to standard techniques, e.g.,
the P3-NS1/1-Ag4-1, P3-x63-Ag8.653 or Sp2/O-Ag14 myeloma lines. These myeloma
lines are available from the American Type Culture Collection (ATCC), Rockville, Md.
Typically, HAT-sensitive mouse myeloma cells are fused to mouse splenocytes using
polyethylene glycol ("PEG"). Hybridoma cells resulting from the fusion are then selected
using HAT medium, which kills unfused and unproductively fused myeloma cells (unfused
splenocytes die after several days because they are not transformed). Hybridoma cells
producing a monoclonal antibody of the present disclosure are detected by screening the
hybridoma culture supernatants for antibodies that bind a given polypeptide, e.g., using a
standard ELISA assay.
As an alternative to preparing monoclonal antibody-secreting hybridomas, a
monoclonal specific for a desired polypeptide (e.g., C1q) can be identified and isolated by
screening a recombinant combinatorial immunoglobulin library (e.g., an antibody phage
display library) with the appropriate polypeptide to thereby isolate immunoglobulin library
members that bind the polypeptide. Kits for generating and screening phage display

libraries are commercially available (e.g., the Pharmacia Recombinant Phage Antibody
System, Catalog No. 27-9400-01; and the Stratagene SurfZAP™ Phage Display Kit,
Catalog No. 240612). Additionally, examples of methods and reagents particularly
amenable for use in generating and screening an antibody display library can be found in,
for example, Ladner et al. U.S. Patent No. 5,223,409; Kang et al. International Publication
No. WO 92/18619; Dower et al. International Publication No. WO 91/17271; Winter et al.
International Publication WO 92/20791; Markland et al. International Publication No. WO
92/15679; Breitling et al. International Publication WO 93/01288; McCafferty et al.
International Publication No. WO 92/01047; Garrard et al. International Publication No.
WO 92/09690; Ladner et al. International Publication No. WO 90/02809; Fuchs et al.
(1991) Biotechnology (NY) 9:1369-1372; Hay et al. (1992) Hum. Antibod. Hybridomas
3:81-85; Huse et al. (1989) Science 246:1275-1281; Griffiths et al. (1993) EMBO J.
12:725-734; Hawkins et al. (1992) J. Mol. Biol. 226:889-896; Clarkson et al. (1991) Nature
352:624-628; Gram et al. (1992) Proc. Natl. Acad. Sci. USA 89:3576-3580; Garrard et al.
(1991) Biotechnology (NY) 9:1373-1377; Hoogenboom et al. (1991) Nucleic Acids Res.
19:4133-4137; Barbas et al. (1991) Proc. Natl. Acad. Sci. USA 88:7978-7982; and
McCafferty et al. (1990) Nature 348:552-554.
Additionally, recombinant anti-C1q antibodies, such as humanized and chimeric
monoclonal antibodies, which can be made using standard recombinant DNA techniques,
can be generated. Such humanized and chimeric monoclonal antibodies can be produced
by recombinant DNA techniques known in the art, for example using methods described in
Robinson et al. International Patent Publication PCT/US86/02269; Akira et al. European
Patent Application 184,187; Taniguchi, M. European Patent Application 171,496; Morrison
et al. European Patent Application 173,494; Neuberger et al. PCT Application WO
86/01533; Cabilly et al. U.S. Patent No. 4,816,567; Cabilly et al. European Patent
Application 125,023; Better et al. (1988) Science 240:1041-1043; Liu et al. (1987) Proc.
Natl. Acad. Sci. USA 84:3439-3443; Liu et al. (1987) J. Immunol. 139:3521-3526; Sun et
al. (1987) Proc. Natl. Acad. Sci. 84:214-218; Nishimura et al. (1987) Cancer Res. 47:999-
1005; Wood et al. (1985) Nature 314:446-449; and Shaw et al. (1988) J. Natl. Cancer Inst.
80:1553-1559); Morrison, S. L. (1985) Science 229:1202-1207; Oi et al. (1986)
Biotechniques 4:214; Winter U.S. Patent 5,225,539; Jones et al. (1986) Nature 321:552-
525; Verhoeyan et al. (1988) Science 239:1534; and Beidler et al. (1988) J. Immunol.
141:4053-4060.

In addition, humanized antibodies can be made according to standard protocols
such as those disclosed in US patent 5,565,332. In another embodiment, antibody chains or
specific binding pair members can be produced by recombination between vectors
comprising nucleic acid molecules encoding a fusion of a polypeptide chain of a specific
binding pair member and a component of a replicable generic display package and vectors
containing nucleic acid molecules encoding a second polypeptide chain of a single binding
pair member using techniques known in the art, e.g., as described in US patents 5,565,332,
5,871,907, or 5,733,743. The use of intracellular antibodies to inhibit protein function in a
cell is also known in the art (see e.g., Carlson, J. R. (1988) Mol. Cell. Biol. 8:2638-2646;
Biocca, S. et al. (1990) EMBO J. 9:101-108; Werge, T. M. et al. (1990) FEBS Lett.
274:193-198; Carlson, J. R. (1993) Proc. Natl. Acad. Sci. USA 90:7427-7428; Marasco, W.
A. et al. (1993) Proc. Natl. Acad. Sci. USA 90:7889-7893; Biocca, S. et al. (1994)
Biotechnology (NY) 12:396-399; Chen, S-Y. et al. (1994) Hum. Gene Ther. 5:595-601;
Duan, L et al. (1994) Proc. Natl. Acad. Sci. USA 91:5075-5079; Chen, S-Y. et al. (1994)
Proc. Natl. Acad. Sci. USA 91:5932-5936; Beerli, R. R. et al. (1994) J. Biol. Chem.
269:23931-23936; Beerli, R. R. et al. (1994) Biochem. Biophys. Res. Commun. 204:666-
672; Mhashilkar, A. M. et al. (1995) EMBO J. 14:1542-1551; Richardson, J. H. et al.
(1995) Proc. Natl. Acad. Sci. USA 92:3137-3141; PCT Publication No. WO 94/02610 by
Marasco et al.; and PCT Publication No. WO 95/03832 by Duan et al.).
In another embodiment, human monoclonal anti-C1q antibodies can be generated
using transgenic or transchromosomal mice carrying parts of the human immune system
rather than the mouse system. In one embodiment, transgenic mice, referred to herein as
"HuMAb mice" which contain a human immunoglobulin gene miniloci that encodes
unrearranged human heavy (μ and γ) and κ light chain immunoglobulin sequences, together
with targeted mutations that inactivate the endogenous μ and κ chain loci (Lonberg, N. et
al. (1994) Nature 368(6474): 856 859). Accordingly, the mice exhibit reduced expression
of mouse IgM or κ, and in response to immunization, the introduced human heavy and light
chain transgenes undergo class switching and somatic mutation to generate high affinity
human IgGκ monoclonal antibodies (Lonberg, N. et al. (1994), supra; reviewed in Lonberg,
N. (1994) Handbook of Experimental Pharmacology 113:49 101; Lonberg, N. and Huszar,
D. (1995) Intern. Rev. Immunol. Vol. 13: 65 93, and Harding, F. and Lonberg, N. (1995)
Ann. N. Y Acad. Sci 764:536 546). The preparation of HuMAb mice is described in
Taylor, L. et al. (1992) Nucleic Acids Research 20:6287 6295; Chen, J. et al. (1993)

International Immunology 5: 647 656; Tuaillon et al. (1993) Proc. Natl. Acad. Sci USA
90:3720 3724; Choi et al. (1993) Nature Genetics 4:117 123; Chen, J. et al. (1993) EMBO
J. 12: 821 830; Tuaillon et al. (1994) J. Immunol. 152:2912 2920; Lonberg et al., (1994)
Nature 368(6474): 856 859; Lonberg, N. (1994) Handbook of Experimental Pharmacology
113:49 101; Taylor, L. et al. (1994) International Immunology 6: 579 591; Lonberg, N. and
Huszar, D. (1995) Intern. Rev. Immunol. Vol. 13: 65 93; Harding, F. and Lonberg, N.
(1995) Ann. N.Y. Acad. Sci 764:536 546; Fishwild, D. et al. (1996) Nature Biotechnology
14: 845 851. See further, U.S. Pat. Nos. 5,545,806; 5,569,825; 5,625,126; 5,633,425;
5,789,650; 5,877,397; 5,661,016; 5,814,318; 5,874,299; and 5,770,429; all to Lonberg and
Kay, and GenPharm International; U.S. Pat. No. 5,545,807 to Surani et al.; International
Publication Nos. WO 98/24884, published on Jun. 11, 1998; WO 94/25585, published Nov.
10, 1994; WO 93/1227, published Jun. 24, 1993; WO 92/22645, published Dec. 23, 1992;
WO 92/03918, published Mar. 19, 1992.
Yet another aspect of the present disclosure relates to anti-C1q antibodies that are
obtainable by a process comprising, immunizing an animal with an immunogenic C1q
polypeptide, respectively, or an immunogenic portion thereof; and then isolating from the
animal antibodies that specifically bind to the polypeptide.
In still another aspect of the present disclosure, partial or known antibody
sequences can be used to generate and/or express new antibodies. Antibodies interact with
target antigens predominantly through amino acid residues that are located in the six heavy
and light chain complementarity determining regions (CDRs). For this reason, the amino
acid sequences within CDRs are more diverse between individual antibodies than
sequences outside of CDRs. Because CDR sequences are responsible for most antibody-
antigen interactions, it is possible to express recombinant antibodies that mimic the
properties of specific naturally occurring antibodies by constructing expression vectors that
include CDR sequences from the specific naturally occurring antibody grafted onto
framework sequences from a different antibody with different properties (see, e.g.,
Riechmann, L. et al., 1998, Nature 332:323 327; Jones, P. et al., 1986, Nature 321:522 525;
and Queen, C. et al., 1989, Proc. Natl. Acad. See. U.S.A. 86:10029 10033). Such
framework sequences can be obtained from public DNA databases that include germline or
non-germline antibody gene sequences. These germline sequences will differ from mature
antibody gene sequences because they will not include completely assembled variable
genes, which are formed by V(D)J joining during B cell maturation. Germline gene

sequences will also differ from the sequences of a high affinity secondary repertoire
antibody at individual evenly across the variable region. For example, somatic mutations
are relatively infrequent in the amino-terminal portion of framework region. For example,
somatic mutations are relatively infrequent in the amino terminal portion of framework
region 1 and in the carboxy-terminal portion of framework region 4. Furthermore, many
somatic mutations do not significantly alter the binding properties of the antibody. For this
reason, it is not necessary to obtain the entire DNA sequence of a particular antibody in
order to recreate an intact recombinant antibody having binding properties similar to those
of the original antibody (see PCT/US99/05535 filed on Mar. 12, 1999). Partial heavy and
light chain sequence spanning the CDR regions is typically sufficient for this purpose. The
partial sequence is used to determine which germline and/or non-germline variable and
joining gene segments contributed to the recombined antibody variable genes. The
germline and/or non-germline sequence is then used to fill in missing portions of the
variable regions. Heavy and light chain leader sequences are cleaved during protein
maturation and do not contribute to the properties of the final antibody. To add missing
sequences, cloned cDNA sequences can be combined with synthetic oligonucleotides by
ligation or PCR amplification. Alternatively, the entire variable region can be synthesized
as a set of short, overlapping, oligonucleotides and combined by PCR amplification to
create an entirely synthetic variable region clone. This process has certain advantages such
as elimination or inclusion or particular restriction sites, or optimization of particular
codons. The process can also be used to screen libraries of particular immunoglobulin
encoding sequences in one species (e.g., human) to design cognate immunoglobulin
encoding sequences from known antibody sequence in another species (e.g., mouse) (see,
for example, the Examples section below).
The nucleotide sequences of heavy and light chain transcripts from a hybridoma
may be used to design an overlapping set of synthetic oligonucleotides to create synthetic V
sequences with identical amino acid coding capacities as the natural sequences. The
synthetic heavy and kappa chain sequences can differ from the natural sequences in three
ways: strings of repeated nucleotide bases are interrupted to facilitate oligonucleotide
synthesis and PCR amplification; optimal translation initiation sites are incorporated
according to Kozak's rules (Kozak, 1991, J. Biol. Chem. 266L19867019870); and, HindIII
sites are engineered upstream of the translation initiation sites.

For both the heavy and light chain variable regions, the optimized coding, and
corresponding non-coding, strand sequences are broken down into 30-50 nucleotide
approximately the midpoint of the corresponding non-coding oligonucleotide.  Thus, for
each chain, the oligonucleotides can be assembled into overlapping double stranded sets
that span segments of 150-400 nucleotides.  The pools are then used as templates to
produce PCR amplification products of 150-400 nucleotides.  Typically, a single variable
region oligonucleotide set will be broken down into two pools which are separately
amplified to generate two overlapping PCR products.  These overlapping products are then
combined by PCR amplification to form the complete variable region.  It may also be
desirable to include an overlapping fragment of the heavy or light chain constant region in
the PCR amplification to generate fragments that can easily be cloned into the expression
vector constructs.
The reconstructed heavy and light chain variable regions are then combined with
cloned promoter, leader sequence, translation initiation, leader sequence, constant region,
3' untranslated, polyadenylation, and transcription termination, sequences to form
expression vector constructs.  The heavy and light chain expression constructs can be
combined into a single vector, co-transfected, serially transfected, or separately transfected
into host cells which are then fused to form a host cell expressing both chains.
Plasmids for this use are known in the art and include the plasmids provided in the
Examples section below.  Fully human and chimeric antibodies of the present disclosure
also include IgG1, IgG2, IgG3, IgG4, IgE, IgA, IgM, and IgD antibodies, and variants and
mutants thereof.  Similar plasmids can be constructed for expression of other heavy chain
isotypes, or for expression of antibodies comprising lambda light chains.
Thus, in one aspect of the present disclosure, the structural features of known,
non-human or human antibodies (e.g., a mouse anti-human anti-C1q antibody, such as the
monoclonal antibody M1 produced by the hybridoma cell line having ATCC Accession
Number PTA-120399) are used to create structurally related human anti-human C1q
antibodies that retain at least one functional property of the antibodies of the present
disclosure, such as binding to a C1q protein.  Another functional property includes
inhibiting binding of the monoclonal antibody M1 to C1q in a competition ELISA assay.
In some embodiments, the structurally related anti-human C1q antibodies have a
comparable binding affinity to the antigen as compared to the monoclonal antibody M1 as
measured by the IC50 value as described in the Examples section below. In some

embodiments, the structurally related anti-human C1q antibodies have a higher affinity to
the antigen as compared to the monoclonal antibody M1 as measured by the IC50 value as
described in the Examples section below. In addition, one or more CDR or variable regions
of an anti-C1q antibody (e.g., monoclonal antibody M1 produced by the hybridoma cell
line having ATCC Accession Number PTA-120399) can be combined recombinantly with
known human framework regions and CDRs to create additional, recombinantly-
engineered, human anti-C1q antibodies of the present disclosure.
Since it is well-known in the art that antibody heavy and light chain CDR3
domains play a particularly important role in the binding specificity/affinity of an antibody
for an antigen, the recombinant antibodies of the present disclosure prepared as set forth
above may, in some embodiments, comprise the heavy and light chain CDR3s of variable
regions of the monoclonal antibody M1 produced by the hybridoma cell line having ATCC
Accession Number PTA-120399. In some embodiments, the antibodies further can
comprise the CDR2s of variable regions of the monoclonal antibody M1. In some
embodiments, the antibodies further can comprise the CDR1s of variable regions of the
monoclonal antibody M1. In some embodiments, the antibodies can further comprise any
combinations of the CDRs.
In some embodiments, the CDR1, 2, and/or 3 regions of the engineered antibodies
described above may comprise the exact amino acid sequence(s) as those of variable
regions of the monoclonal antibody M1 produced by the hybridoma cell line having ATCC
Accession Number PTA-120399. However, the ordinarily skilled artisan will appreciate
that some deviation from the exact CDR sequences may be possible while still retaining the
ability of the antibody to bind C1q effectively (e.g., conservative sequence modifications).
Accordingly, in another embodiment, the engineered antibody may be composed of one or
more CDRs that are, for example, 50%, 60%, 70%, 80%, 85%, 90%, 91%, 92%, 93%, 94%,
95%, 96%, 97%, 98%, 99%, or 99.5% identical to one or more CDRs of the monoclonal
antibody M1.
Antibody fragments
In certain embodiments there are advantages to using anti-C1q antibody
fragments, rather than whole anti-C1q antibodies. Smaller fragment sizes allow for rapid
clearance.
Various techniques have been developed for the production of antibody
fragments. Traditionally, these fragments were derived via proteolytic digestion of intact

antibodies (see, e.g., Morimoto et al., J. Biochem. Biophys. Method. 24:107-117 (1992);
and Brennan et al., Science 229:81 (1985)). However, these fragments can now be
produced directly by recombinant host-cells, for example, using nucleic acids encoding
anti-C1q antibodies of the present disclosure. Fab, Fv and scFv antibody fragments can all
be expressed in and secreted from E. coli, thus allowing the straightforward production of
large amounts of these fragments. A anti-C1q antibody fragments can also be isolated from
the antibody phage libraries as discussed above. Alternatively, Fab'-SH fragments can be
directly recovered from E. coli and chemically coupled to form F(ab')2 fragments (Carter et
al., Bio/Technology 10:163-167 (1992)). According to another approach, F(ab')2 fragments
can be isolated directly from recombinant host-cell culture. Production of Fab and F(ab')2
antibody fragments with increased in vivo half-lives are described in U.S. Patent No.
5,869,046. In other embodiments, the antibody of choice is a single chain Fv fragment
(scFv). See WO 93/16185; U.S. Patent No. 5,571,894 and U.S. Patent No. 5,587,458. The
anti-C1q, anti-C1r, or anti-C1q antibody fragment may also be a "linear antibody," e.g., as
described in U.S. Patent 5,641,870. Such linear antibody fragments may be monospecific
or bispecific.
Bispecific and polyspecific antibodies
In some embodiments, antibodies of the present disclosure encompass bispecific
antibodies and polyspecific antibodies.
Bispecific antibodies (BsAbs) are antibodies that have binding specificities for
at least two different epitopes, including those on the same or another protein (e.g., one or
more C1q proteins of the present disclosure). Alternatively, one part of a BsAb can be
armed to bind to the target C1q antigen, and another can be combined with an arm that
binds to a second protein. Such antibodies can be derived from full length antibodies or
antibody fragments (e.g., F(ab')2 bispecific antibodies).
Methods for making bispecific antibodies are known in the art. Traditional
production of full length bispecific antibodies is based on the coexpression of two
immunoglobulin heavy-chain/light chain pairs, where the two chains have different
specificities. Millstein et al., Nature, 305:537-539 (1983). Because of the random
assortment of immunoglobulin heavy and light chains, these hybridomas (quadromas)
produce a potential mixture of 10 different antibody molecules, of which only one has the
correct bispecific structure. Purification of the correct molecule, which is usually done by
affinity chromatography steps, is rather cumbersome, and the product yields are low.

Similar procedures are disclosed in WO 93/08829 and in Traunecker et al., EMBO J.,
10:3655-3659 (1991).
According to a different approach, antibody variable domains with the desired
binding specificities (antibody-antigen combining sites) are fused to immunoglobulin
constant domain sequences. The fusion may be with an immunoglobulin heavy chain
constant domain, comprising at least part of the hinge, CH2, and CH3 regions. In some
embodiments, the first heavy-chain constant region (CH1) containing the site necessary for
light chain binding is present in at least one of the fusions. DNAs encoding the
immunoglobulin heavy chain fusions and, if desired, the immunoglobulin light chain, are
inserted into separate expression vectors, and are co-transfected into a suitable host
organism. This provides great flexibility in adjusting the mutual proportions of the three
polypeptide fragments in embodiments when unequal ratios of the three polypeptide chains
used in the construction provide the optimum yields. It is, however, possible to insert the
coding sequences for two or all three polypeptide chains in one expression vector when the
expression of at least two polypeptide chains in equal ratios results in high yields or when
the ratios are of no particular significance.
In some embodiments of this approach, the bispecific antibodies are composed
of a hybrid immunoglobulin heavy chain with a first binding specificity in one arm, and a
hybrid immunoglobulin heavy chain-light chain pair (providing a second binding
specificity) in the other arm. It was found that this asymmetric structure facilitates the
separation of the desired bispecific compound from unwanted immunoglobulin chain
combinations, as the presence of an immunoglobulin light chain in only half of the
bispecific molecules provides an easy way of separation. This approach is disclosed in WO
94/04690. For further details of generating bispecific antibodies, see, for example, Suresh
et al., Methods in Enzymology 121: 210 (1986).
According to another approach described in WO 96/27011 or U.S. Patent No.
5,731,168, the interface between a pair of antibody molecules can be engineered to
maximize the percentage of heterodimers which are recovered from recombinant-cell
culture. The interface may comprise at least a part of the CH3 region of an antibody
constant domain. In this method, one or more small amino acid side chains from the
interface of the first antibody molecule are replaced with larger side chains (e.g., tyrosine or
tryptophan). Compensatory "cavities" of identical or similar size to the large side chains(s)
are created on the interface of the second antibody molecule by replacing large amino acid

side chains with smaller ones (e.g., alanine or threonine). This provides a mechanism for
increasing the yield of the heterodimer over other unwanted end-products such as
homodimers.
Techniques for generating bispecific antibodies from antibody fragments have
been described in the literature. For example, bispecific antibodies can be prepared using
chemical linkage. Brennan et al., Science 229:81 (1985) describe a procedure wherein
intact antibodies are proteolytically cleaved to generate F(ab')2 fragments. These fragments
are reduced in the presence of the dithiol complexing agent sodium arsenite to stabilize
vicinal dithiols and prevent intermolecular disulfide formation. The Fab' fragments
generated are then converted to thionitrobenzoate (TNB) derivatives. One of the Fab'-TNB
derivatives is then reconverted to the Fab'-TNB derivative to form the bispecific antibody.
The bispecific antibodies produced can be used as agents for the selective immobilization
of enzymes.
Fab' fragments may be directly recovered from E. coli and chemically coupled
to form bispecific antibodies. Shalaby et al., J. Exp. Med. 175: 217-225 (1992) describes
the production of fully humanized bispecific antibody F(ab')2 molecules. Each Fab'
fragment was separately secreted from E. coli and subjected to directed chemical coupling
in vitro to form the bispecific antibody. The bispecific antibody thus formed was able to
bind to cells overexpressing the ErbB2 receptor and normal human T-cells, as well as
trigger the lytic activity of human cytotoxic lymphocytes against human breast tumor
targets.
Various techniques for making and isolating bivalent antibody fragments
directly from recombinant-cell culture have also been described. For example, bivalent
heterodimers have been produced using leucine zippers. Kostelny et al., J. Immunol.,
148(5):1547-1553 (1992). The leucine zipper peptides from the Fos and Jun proteins were
linked to the Fab' portions of two different antibodies by gene fusion. The antibody
homodimers were reduced at the hinge region to form monomers and then re-oxidized to
form the antibody heterodimers. The "diabody" technology described by Hollinger et al.,
Proc. Nat'l Acad. Sci. USA, 90: 6444-6448 (1993) has provided an alternative mechanism
for making bispecific/bivalent antibody fragments. The fragments comprise a heavy-chain
variable domain (VH) connected to a light-chain variable domain (VL) by a linker which is
too short to allow pairing between the two domains on the same chain. Accordingly, the
VH and VL domains of one fragment are forced to pair with the complementary VL and VH

domains of another fragment, thereby forming two antigen-binding sites. Another strategy
for making bispecific/bivalent antibody fragments by the use of single-chain Fv (sFv)
dimers has also been reported. *See* Gruber et al., *J. Immunol.*, 152:5368 (1994).
**[0180]** Antibodies with more than two valencies are also contemplated. For example,
trispecific antibodies can be prepared. Tutt et al., *J. Immunol.* 147:60 (1991).
**[0181]** Exemplary bispecific antibodies may bind to two different antigens. In some
embodiments a bispecific antibody binds to a first antigen, C1q, and a second antigen
facilitating transport across the blood-brain barrier. Numerous antigens are known in the
art that facilitate transport across the blood-brain barrier (*see, e.g.*, Gabathuler R.,
Approaches to transport therapeutic drugs across the blood-brain barrier to treat brain
diseases, Neurobiol. Dis. 37 (2010) 48-57). Such second antigens include, without
limitation, transferrin receptor (TR), insulin receptor (HIR), Insulin-like growth factor
receptor (IGFR), low-density lipoprotein receptor related proteins 1 and 2 (LPR-1 and 2),
diphtheria toxin receptor, including CRM197 (a non-toxic mutant of diphtheria toxin),
llama single domain antibodies such as TMEM 30(A) (Flippase), protein transduction
domains such as TAT, Syn-B, or penetratin, poly-arginine or generally positively charged
peptides, and Angiopep peptides such as ANG1005 (*see, e.g.*, Gabathuler, 2010).
*Multivalent antibodies*
**[0182]** In some embodiments, antibodies of the present disclosure encompass
multivalent antibodies. A multivalent antibody may be internalized (and/or catabolized)
faster than a bivalent antibody by a cell expressing an antigen to which the antibodies bind.
The anti-C1q antibodies of the present disclosure or antibody fragments thereof can be
multivalent antibodies (which are other than of the IgM class) with three or more antigen
binding sites (*e.g.*, tetravalent antibodies), which can be readily produced by recombinant
expression of nucleic acid encoding the polypeptide chains of the antibody. The
multivalent antibody can comprise a dimerization domain and three or more antigen
binding sites. In some embodiments, the dimerization domain comprises an Fc region or a
hinge region. In this scenario, the antibody will comprise an Fc region and three or more
antigen binding sites amino-terminal to the Fc region. In some embodiments, the
multivalent antibody herein contains three to about eight, and in some embodiments four,
antigen binding sites. The multivalent antibody contains at least one polypeptide chain
(and in some embodiments two polypeptide chains), wherein the polypeptide chain or
chains comprise two or more variable domains. For instance, the polypeptide chain or

chains may comprise VD1-(X1)n-VD2-(X2)n-Fc, wherein VD1 is a first variable domain,
VD2 is a second variable domain, Fc is one polypeptide chain of an Fc region, X1 and X2
represent an amino acid or polypeptide, and n is 0 or 1. Similarly, the polypeptide chain or
chains may comprise V_{H}-C_{H}1-flexible linker-V_{H}-C_{H}1-Fc region chain; or V_{H}-C_{H}1-V_{H}-C_{H}1-
Fc region chain. The multivalent antibody herein may further comprise at least two (and in
some embodiments four) light chain variable domain polypeptides. The multivalent
antibody herein may, for instance, comprise from about two to about eight light chain
variable domain polypeptides. The light chain variable domain polypeptides contemplated
here comprise a light chain variable domain and, optionally, further comprise a CL domain.
*Heteroconjugate antibodies*
**[0183]** Heteroconjugate antibodies are also within the scope of the present disclosure.
Heteroconjugate antibodies are composed of two covalently joined antibodies (*e.g.*, anti-
C1q antibodies of the present disclosure or antibody fragments thereof). For example, one
of the antibodies in the heteroconjugate can be coupled to avidin, the other to biotin. Such
antibodies have, for example, been proposed to target immune system cells to unwanted
cells, U.S. Patent No. 4,676,980, and have been used to treat HIV infection. International
Publication Nos. WO 91/00360, WO 92/200373 and EP 0308936. It is contemplated that
the antibodies may be prepared *in vitro* using known methods in synthetic protein
chemistry, including those involving crosslinking agents. For example, immunotoxins may
be constructed using a disulfide exchange reaction or by forming a thioether bond.
Examples of suitable reagents for this purpose include iminothiolate and methyl-4-
mercaptobutyrimidate and those disclosed, for example, in U.S. Patent No. 4,676,980.
Heteroconjugate antibodies may be made using any convenient cross-linking methods.
Suitable cross-linking agents are well known in the art, and are disclosed in U.S. Patent No.
4,676,980, along with a number of cross-linking techniques.
*Effector function engineering*
**[0184]** In some embodiments, it may be desirable to modify a humanized anti-C1q
antibody of the present disclosure to modify effector function and/or to increase serum half-
life of the antibody. For example, the Fc receptor binding site on the constant region may
be modified or mutated to remove or reduce binding affinity to certain Fc receptors, such as
FcγRI, FcγRII, and/or FcγRIII. In some embodiments, the effector function is impaired by
removing N-glycosylation of the Fc region (*e.g.*, in the CH 2 domain of IgG) of the
antibody. In some embodiments, the effector function is impaired by modifying regions

such as 233-236, 297, and/or 327-331 of human IgG as described in PCT WO 99/58572
and Armour et al., Molecular Immunology 40: 585-593 (2003); Reddy et al., J. Immunology
164:1925-1933 (2000).
The constant region of the anti-complement antibodies described herein may
also be modified to impair complement activation. For example, complement activation of
IgG antibodies following binding of the C1 component of complement may be reduced by
mutating amino acid residues in the constant region in a C1 binding motif (e.g., C1q
binding motif). It has been reported that Ala mutation for each of D270, K322, P329, P331
of human IgG1 significantly reduced the ability of the antibody to bind to C1q and
activating complement. For murine IgG2b, C1q binding motif constitutes residues E318,
K320, and K322. Idusogie et al. (2000) J. Immunology 164:4178-4184; Duncan et al.
(1988) Nature 322: 738-740. As the Cls binding motif E318, K320, and K322 identified
for murine IgG2b is believed to be common for other antibody isotypes (Duncan et al.
(1988) Nature 322:738-740), C1q binding activity for IgG2b can be abolished by replacing
any one of the three specified residues with a residue having an inappropriate functionality
on its side chain. It is not necessary to replace the ionic residues only with Ala to abolish
Clq binding. It is also possible to use other alkyl-substituted non-ionic residues, such as
Gly, Ile, Leu, or Val, or such aromatic non-polar residues as Phe, Tyr, Trp, and Pro in place
of any one of the three residues in order to abolish Clq binding. In addition, it is also
possible to use such polar non-ionic residues as Ser, Thr, Cys, and Met in place of residues
320 and 322, but not 318, in order to abolish Cls binding activity. In addition, removal of
carbohydrate modifications of the Fc region necessary for complement binding can prevent
complement activation Glycosylation of a conserved asparagine (Asn-297) on the CH2
domain of IgG heavy chains is essential for antibody effector functions (Jefferis et al.
(1998) Immunol Rev 163:59–76). Modification of the Fc glycan alters IgG conformation
and reduces the Fc affinity for binding of complement protein C1q and effector cell
receptor FcR (Alhorn et al. (2008) PLos ONE 2008;3:e1413). Complete removal of the Fc
glycan abolishes CDC and ADCC. Deglycosylation can be performed using glycosidase
enzymes for example  Endoglycosidase S (EndoS), a 108kDa enzyme encoded by the gene
endoS of Streptococcus pyogenes that selectively digests asparagine-linked glycans on the
heavy chain of all IgG subclasses, without action on other immunoglobulin classes or other
glycoproteins (Collin et al. (2001) EMBO J 2001;20:3046–3055).

To increase the serum half-life of the antibody, one may incorporate a salvage
receptor binding epitope into the antibody (especially an antibody fragment) as described in
U.S. Patent 5,739,277, for example. As used herein, the term "salvage receptor binding
epitope" refers to an epitope of the Fc region of an IgG molecule (e.g., IgG1, IgG2, IgG3, or
IgG4) that is responsible for increasing the in vivo serum half-life of the IgG molecule.
Other amino acid sequence modifications
Amino acid sequence modifications of humanized anti-C1q antibodies of the
present disclosure, or antibody fragments thereof, are also contemplated. For example, it
may be desirable to improve the binding affinity and/or other biological properties of the
antibodies or antibody fragments. Amino acid sequence variants of the antibodies or
antibody fragments are prepared by introducing appropriate nucleotide changes into the
nucleic acid encoding the antibodies or antibody fragments, or by peptide synthesis. Such
modifications include, for example, deletions from, and/or insertions into and/or
substitutions of, residues within the amino acid sequences of the antibody. Any
combination of deletion, insertion, and substitution is made to arrive at the final construct,
provided that the final construct possesses the desired characteristics (i.e., the ability to
bind or physically interact with a C1q protein of the present disclosure). The amino acid
changes also may alter post-translational processes of the antibody, such as changing the
number or position of glycosylation sites.
A useful method for identification of certain residues or regions of the anti-C1q
antibody that are preferred locations for mutagenesis is called "alanine scanning
mutagenesis" as described by Cunningham and Wells in Science, 244:1081-1085 (1989).
Here, a residue or group of target residues are identified (e.g., charged residues such as arg,
asp, his, lys, and glu) and replaced by a neutral or negatively charged amino acid (most
preferably alanine or polyalanine) to affect the interaction of the amino acids with the target
antigen. Those amino acid locations demonstrating functional sensitivity to the
substitutions then are refined by introducing further or other variants at, or for, the sites of
substitution. Thus, while the site for introducing an amino acid sequence variation is
predetermined, the nature of the mutation per se need not be predetermined. For example,
to analyze the performance of a mutation at a given site, alanine scanning or random
mutagenesis is conducted at the target codon or region and the expressed antibody variants
are screened for the desired activity.

Amino acid sequence insertions include amino- ("N") and/or carboxy- ("C")
terminal fusions ranging in length from one residue to polypeptides containing a hundred or
more residues, as well as intrasequence insertions of single or multiple amino acid residues.
Examples of terminal insertions include an antibody with an N-terminal methionyl residue
or the antibody fused to a cytotoxic polypeptide. Other insertional variants of the antibody
molecule include the fusion to the N- or C-terminus of the antibody to an enzyme or a
polypeptide which increases the serum half-life of the antibody.
Another type of variant is an amino acid substitution variant. These variants
have at least one amino acid residue in the antibody molecule replaced by a different
residue. The sites of greatest interest for substitutional mutagenesis include the
hypervariable regions, but FR alterations are also contemplated. Conservative substitutions
are shown in the Table A below under the heading of "preferred substitutions". If such
substitutions result in a change in biological activity, then more substantial changes,
denominated "exemplary substitutions" in Table A, or as further described below in
reference to amino acid classes, may be introduced and the products screened.
TABLE A: Amino Acid Substitutions
Original Residue | Exemplary Substitutions | Preferred Substitutions
Ala (A) | val; leu; ile | val
Arg (R) | lys; gln; asn | lys
Asn (N) | gln; his; asp, lys; arg | gln
Asp (D) | glu; asn | glu
Cys (C) | ser; ala | ser
Gln (Q) | asn; glu | asn
Glu (E) | asp; gln | asp
Gly (G) | ala | ala
His (H) | asn; gln; lys; arg | arg
Ile (I) | leu; val; met; ala; phe; norleucine | leu
Leu (L) | norleucine; ile; val; met; ala; phe | ile
Lys (K) | arg; gln; asn | arg
Met (M) | leu; phe; ile | leu
Phe (F) | leu; val; ile; ala; tyr | tyr
Pro (P) | ala | ala
Ser (S) | thr | thr
Thr (T) | ser | ser
Trp (W) | tyr; phe | tyr
Tyr (Y) | trp; phe; thr; ser | phe
Val (V) | ile; leu; met; phe; ala; norleucine | leu

Substantial modifications in the biological properties of the antibody are
accomplished by selecting substitutions that differ significantly in their effect on
maintaining (a) the structure of the polypeptide backbone in the area of the substitution, for
example, as a sheet or helical conformation, (b) the charge or hydrophobicity of the
molecule at the target site, or (c) the bulk of the side chain.  Naturally occurring residues
are divided into groups based on common side-chain properties:
(1) hydrophobic:  norleucine, met, ala, val, leu, ile;
(2) neutral hydrophilic:  cys, ser, thr;
(3) acidic:  asp, glu;
(4) basic:  asn, gln, his, lys, arg;
(5) residues that influence chain orientation:  gly, pro; and
(6) aromatic:  trp, tyr, phe.
Non-conservative substitutions entail exchanging a member of one of these
classes for another class.
Any cysteine residue not involved in maintaining the proper conformation of the
antibody also may be substituted, generally with serine, to improve the oxidative stability
of the molecule and prevent aberrant crosslinking.  Conversely, cysteine bond(s) may be
added to the antibody to improve its stability (particularly where the antibody is an
antibody fragment, such as an Fv fragment).
In some embodiments, the substitutional variant involves substituting one or
more hypervariable region residues of a parent antibody (e.g. a humanized or human anti-
C1q antibody).  Generally, the resulting variant(s) selected for further development will
have improved biological properties relative to the parent antibody from which they are
generated.  A convenient way for generating such substitutional variants involves affinity
maturation using phage display.  Briefly, several hypervariable region sites (e.g., 6-7 sites)
are mutated to generate all possible amino substitutions at each site.  The antibody variants
thus generated are displayed in a monovalent fashion from filamentous phage particles as
fusions to the gene III product of M13 packaged within each particle.  The phage-displayed
variants are then screened for their biological activity (e.g., binding affinity) as herein
disclosed. In order to identify candidate hypervariable region sites for modification, alanine
scanning mutagenesis can be performed to identify hypervariable region residues
contributing significantly to antigen binding.  Alternatively, or additionally, it may be
beneficial to analyze a crystal structure of the antigen-antibody complex to identify contact

points between the antibody and the antigen (*e.g.*, a C1q protein of the present disclosure).
Such contact residues and neighboring residues are candidates for substitution according to
the techniques elaborated herein. Once such variants are generated, the panel of variants is
subjected to screening as described herein and antibodies with superior properties in one or
more relevant assays may be selected for further development.
**[0195]** Another type of amino acid variant of the antibody alters the original
glycosylation pattern of the antibody. By altering is meant deleting one or more
carbohydrate moieties found in the antibody, and/or adding one or more glycosylation sites
that are not present in the antibody.
**[0196]** Glycosylation of antibodies is typically either N-linked or O-linked. N-linked
refers to the attachment of the carbohydrate moiety to the side chain of an asparagine
residue. The tripeptide sequences asparagine-X-serine and asparagine-X-threonine, where
X is any amino acid except proline, are the recognition sequences for enzymatic attachment
of the carbohydrate moiety to the asparagine side chain. Thus, the presence of either of
these tripeptide sequences in a polypeptide creates a potential glycosylation site. O-linked
glycosylation refers to the attachment of one of the sugars N-aceylgalactosamine, galactose,
or xylose to a hydroxyamino acid, most commonly serine or threonine, although 5-
hydroxyproline or 5-hydroxylysine may also be used.
**[0197]** Addition of glycosylation sites to the antibody is conveniently accomplished by
altering the amino acid sequence such that it contains one or more of the above-described
tripeptide sequences (for N-linked glycosylation sites). The alteration may also be made by
the addition of, or substitution by, one or more serine or threonine residues to the sequence
of the original antibody (for O-linked glycosylation sites).
**[0198]** Nucleic acid molecules encoding amino acid sequence variants of the anti-IgE
antibody are prepared by a variety of methods known in the art. These methods include,
but are not limited to, isolation from a natural source (in the case of naturally occurring
amino acid sequence variants) or preparation by oligonucleotide-mediated (or site-directed)
mutagenesis, PCR mutagenesis, and cassette mutagenesis of an earlier prepared variant or a
non-variant version of the antibodies (*e.g.*, anti-C1q antibody of the present disclosure) or
antibody fragments.
*Other antibody modifications*
**[0199]** In some embodiments, humanized anti-C1q antibodies of the present disclosure,
or antibody fragments thereof, may be further modified to contain additional non-

proteinaceous moieties that are known in the art and readily available. In some
embodiments, the moieties suitable for derivatization of the antibody are water-soluble
polymers. Non-limiting examples of water-soluble polymers include, but are not limited to,
polyethylene glycol (PEG), copolymers of ethylene glycol/propylene glycol,
carboxymethylcellulose, dextran, polyvinyl alcohol, polyvinyl pyrrolidone, poly-1, 3-
dioxolane, poly-1,3,6-trioxane, ethylene/maleic anhydride copolymer, polyaminoacids
(either homopolymers or random copolymers), and dextran or poly(n-vinyl
pyrrolidone)polyethylene glycol, polypropylene glycol homopolymers, polypropylene
oxide/ethylene oxide co-polymers, polyoxyethylated polyols (e.g., glycerol), polyvinyl
alcohol, and mixtures thereof. Polyethylene glycol propionaldehyde may have advantages
in manufacturing due to its stability in water. The polymer may be of any molecular
weight, and may be branched or unbranched. The number of polymers attached to the
antibody may vary, and if more than one polymer is attached, they can be the same or
different molecules. In general, the number and/or type of polymers used for derivatization
can be determined based on considerations including, but not limited to, the particular
properties or functions of the antibody to be improved, whether the antibody derivative will
be used in a therapy under defined conditions, etc. Such techniques and other suitable
formulations are disclosed in Remington: The Science and Practice of Pharmacy, 20th Ed.,
Alfonso Gennaro, Ed., Philadelphia College of Pharmacy and Science (2000).
Nucleic acids, vectors, and host cells
Humanized anti-C1q antibodies of the present disclosure may be produced using
recombinant methods and compositions, e.g., as described in U.S. Patent No. 4,816,567. In
some embodiments, isolated nucleic acids having a nucleotide sequence encoding any of
the anti-C1q antibodies of the present disclosure are provided. Such nucleic acids may
encode an amino acid sequence containing the VL and/or an amino acid sequence
containing the VH of the anti-C1q antibody (e.g., the light and/or heavy chains of the
antibody). In some embodiments, one or more vectors (e.g., expression vectors) containing
such nucleic acids are provided. In some embodiments, a host cell containing such nucleic
acid is also provided. In some embodiments, the host cell contains (e.g., has been
transduced with): (1) a vector containing a nucleic acid that encodes an amino acid
sequence containing the VL of the antibody and an amino acid sequence containing the VH
of the antibody, or (2) a first vector containing a nucleic acid that encodes an amino acid
sequence containing the VL of the antibody and a second vector containing a nucleic acid

that encodes an amino acid sequence containing the VH of the antibody. In some
embodiments, the host cell is eukaryotic, e.g., a Chinese Hamster Ovary (CHO) cell or
lymphoid cell (e.g., Y0, NS0, Sp20 cell).
Methods of making an anti-C1q antibody of the present disclosure are provided.
In some embodiments, the method includes culturing a host cell of the present disclosure
containing a nucleic acid encoding the anti-C1q antibody, under conditions suitable for
expression of the antibody. In some embodiments, the antibody is subsequently recovered
from the host cell (or host cell culture medium).
For recombinant production of a humanized anti-C1q antibody of the present
disclosure, a nucleic acid encoding the anti-C1q antibody is isolated and inserted into one
or more vectors for further cloning and/or expression in a host cell. Such nucleic acid may
be readily isolated and sequenced using conventional procedures (e.g., by using
oligonucleotide probes that are capable of binding specifically to genes encoding the heavy
and light chains of the antibody).
Suitable vectors containing a nucleic acid sequence encoding any of the anti-
C1q antibodies of the present disclosure, or fragments thereof polypeptides (including
antibodies) described herein include, without limitation, cloning vectors and expression
vectors. Suitable cloning vectors can be constructed according to standard techniques, or
may be selected from a large number of cloning vectors available in the art. While the
cloning vector selected may vary according to the host cell intended to be used, useful
cloning vectors generally have the ability to self-replicate, may possess a single target for a
particular restriction endonuclease, and/or may carry genes for a marker that can be used in
selecting clones containing the vector. Suitable examples include plasmids and bacterial
viruses, e.g., pUC18, pUC19, Bluescript (e.g., pBS SK+) and its derivatives, mpl8, mpl9,
pBR322, pMB9, ColE1, pCR1, RP4, phage DNAs, and shuttle vectors such as pSA3 and
pAT28. These and many other cloning vectors are available from commercial vendors such
as BioRad, Strategene, and Invitrogen.
Expression vectors generally are replicable polynucleotide constructs that
contain a nucleic acid of the present disclosure. The expression vector may replicable in
the host cells either as episomes or as an integral part of the chromosomal DNA. Suitable
expression vectors include but are not limited to plasmids, viral vectors, including
adenoviruses, adeno-associated viruses, retroviruses, cosmids, and expression vector(s)
disclosed in PCT Publication No. WO 87/04462. Vector components may generally

include, but are not limited to, one or more of the following: a signal sequence; an origin of
replication; one or more marker genes; suitable transcriptional controlling elements (such
as promoters, enhancers and terminator). For expression (i.e., translation), one or more
translational controlling elements are also usually required, such as ribosome binding sites,
translation initiation sites, and stop codons.
The vectors containing the nucleic acids of interest can be introduced into the
host cell by any of a number of appropriate means, including electroporation, transfection
employing calcium chloride, rubidium chloride, calcium phosphate, DEAE-dextran, or
other substances; microprojectile bombardment; lipofection; and infection (e.g., where the
vector is an infectious agent such as vaccinia virus). The choice of introducing vectors or
polynucleotides will often depend on features of the host cell. In some embodiments, the
vector contains a nucleic acid containing one or more amino acid sequences encoding an
anti-C1q antibody of the present disclosure.
Suitable host cells for cloning or expression of antibody-encoding vectors
include prokaryotic or eukaryotic cells. For example, anti-C1q antibodies of the present
disclosure may be produced in bacteria, in particular when glycosylation and Fc effector
function are not needed. For expression of antibody fragments and polypeptides in bacteria
(e.g., U.S. Patent Nos. 5,648,237, 5,789,199, and 5,840,523; and Charlton, Methods in
Molecular Biology, Vol. 248 (B.K.C. Lo, ed., Humana Press, Totowa, NJ, 2003), pp. 245-
254, describing expression of antibody fragments in E. coli.). After expression, the
antibody may be isolated from the bacterial cell paste in a soluble fraction and can be
further purified.
In addition to prokaryotes, eukaryotic microorganisms, such as filamentous
fungi or yeast, are also suitable cloning or expression hosts for antibody-encoding vectors,
including fungi and yeast strains whose glycosylation pathways have been "humanized,"
resulting in the production of an antibody with a partially or fully human glycosylation
pattern (e.g., Gerngross, Nat. Biotech. 22:1409-1414 (2004); and Li et al., Nat. Biotech.
24:210-215 (2006)).
Suitable host cells for the expression of glycosylated antibody can also be
derived from multicellular organisms (invertebrates and vertebrates). Examples of
invertebrate cells include plant and insect cells. Numerous baculoviral strains have been
identified which may be used in conjunction with insect cells, particularly for transfection
of Spodoptera frugiperda cells. Plant cell cultures can also be utilized as hosts (e.g., U.S.

Patent Nos. 5,959,177, 6,040,498, 6,420,548, 7,125,978, and 6,417,429, describing
PLANTIBODIES™ technology for producing antibodies in transgenic plants.).
Vertebrate cells may also be used as hosts. For example, mammalian cell lines
that are adapted to grow in suspension may be useful. Other examples of useful
mammalian host cell lines are monkey kidney CV1 line transformed by SV40 (COS-7);
human embryonic kidney line (293 or 293 cells as described, e.g., in Graham et al., J. Gen
Virol. 36:59 (1977)); baby hamster kidney cells (BHK); mouse sertoli cells (TM4 cells as
described, e.g., in Mather, Biol. Reprod. 23:243-251 (1980)); monkey kidney cells (CV1);
African green monkey kidney cells (VERO-76); human cervical carcinoma cells (HELA);
canine kidney cells (MDCK; buffalo rat liver cells (BRL 3A); human lung cells (W138);
human liver cells (Hep G2); mouse mammary tumor (MMT 060562); TRI cells, as
described, e.g., in Mather et al., Annals N.Y. Acad. Sci. 383:44-68 (1982); MRC 5 cells; and
FS4 cells. Other useful mammalian host cell lines include Chinese hamster ovary (CHO)
cells, including DHFR-CHO cells (Urlaub et al., Proc. Natl. Acad. Sci. USA 77:4216
(1980)); and myeloma cell lines such as Y0, NS0 and Sp2/0. For a review of certain
mammalian host cell lines suitable for antibody production, see, e.g., Yazaki and Wu,
Methods in Molecular Biology, Vol. 248 (B.K.C. Lo, ed., Humana Press, Totowa, NJ), pp.
255-268 (2003).
Pharmaceutical compositions
Humanized anti-C1q antibodies of the present disclosure can be incorporated
into a variety of formulations for therapeutic use (e.g., by administration) or in the
manufacture of a medicament (e.g., for treating or preventing a neurodegenerative disease
or autoimmune disease) by combining the antibodies with appropriate pharmaceutically
acceptable carriers or diluents, and may be formulated into preparations in solid, semi-
solid, liquid or gaseous forms. Examples of such formulations include, without limitation,
tablets, capsules, powders, granules, ointments, solutions, suppositories, injections,
inhalants, gels, microspheres, and aerosols. Pharmaceutical compositions can include,
depending on the formulation desired, pharmaceutically-acceptable, non-toxic carriers of
diluents, which are vehicles commonly used to formulate pharmaceutical compositions for
animal or human administration. The diluent is selected so as not to affect the biological
activity of the combination. Examples of such diluents include, without limitation, distilled
water, buffered water, physiological saline, PBS, Ringer's solution, dextrose solution, and
Hank's solution. A pharmaceutical composition or formulation of the present disclosure

can further include other carriers, adjuvants, or non-toxic, nontherapeutic, nonimmunogenic
stabilizers, excipients and the like. The compositions can also include additional
substances to approximate physiological conditions, such as pH adjusting and buffering
agents, toxicity adjusting agents, wetting agents and detergents.
A pharmaceutical composition of the present disclosure can also include any of
a variety of stabilizing agents, such as an antioxidant for example. When the
pharmaceutical composition includes a polypeptide, the polypeptide can be complexed with
various well-known compounds that enhance the in vivo stability of the polypeptide, or
otherwise enhance its pharmacological properties (e.g., increase the half-life of the
polypeptide, reduce its toxicity, and enhance solubility or uptake). Examples of such
modifications or complexing agents include, without limitation, sulfate, gluconate, citrate
and phosphate. The polypeptides of a composition can also be complexed with molecules
that enhance their in vivo attributes. Such molecules include, without limitation,
carbohydrates, polyamines, amino acids, other peptides, ions (e.g., sodium, potassium,
calcium, magnesium, manganese), and lipids.
Further examples of formulations that are suitable for various types of
administration can be found in Remington's Pharmaceutical Sciences, Mace Publishing
Company, Philadelphia, PA, 17th ed. (1985). For a brief review of methods for drug
delivery, see, Langer, Science 249:1527-1533 (1990).
For oral administration, the active ingredient can be administered in solid
dosage forms, such as capsules, tablets, and powders, or in liquid dosage forms, such as
elixirs, syrups, and suspensions. The active component(s) can be encapsulated in gelatin
capsules together with inactive ingredients and powdered carriers, such as glucose, lactose,
sucrose, mannitol, starch, cellulose or cellulose derivatives, magnesium stearate, stearic
acid, sodium saccharin, talcum, magnesium carbonate. Examples of additional inactive
ingredients that may be added to provide desirable color, taste, stability, buffering capacity,
dispersion or other known desirable features are red iron oxide, silica gel, sodium lauryl
sulfate, titanium dioxide, and edible white ink. Similar diluents can be used to make
compressed tablets. Both tablets and capsules can be manufactured as sustained release
products to provide for continuous release of medication over a period of hours.
Compressed tablets can be sugar coated or film coated to mask any unpleasant taste and
protect the tablet from the atmosphere, or enteric-coated for selective disintegration in the

gastrointestinal tract. Liquid dosage forms for oral administration can contain coloring and
flavoring to increase patient acceptance.
Formulations suitable for parenteral administration include aqueous and non-
aqueous, isotonic sterile injection solutions, which can contain antioxidants, buffers,
bacteriostats, and solutes that render the formulation isotonic with the blood of the intended
recipient, and aqueous and non-aqueous sterile suspensions that can include suspending
agents, solubilizers, thickening agents, stabilizers, and preservatives.
The components used to formulate the pharmaceutical compositions are
preferably of high purity and are substantially free of potentially harmful contaminants
(e.g., at least National Food (NF) grade, generally at least analytical grade, and more
typically at least pharmaceutical grade). Moreover, compositions intended for in vivo use
are usually sterile. To the extent that a given compound must be synthesized prior to use,
the resulting product is typically substantially free of any potentially toxic agents,
particularly any endotoxins, which may be present during the synthesis or purification
process. Compositions for parental administration are also sterile, substantially isotonic
and made under GMP conditions.
Formulations may be optimized for retention and stabilization in the brain or
central nervous system. When the agent is administered into the cranial compartment, it is
desirable for the agent to be retained in the compartment, and not to diffuse or otherwise
cross the blood brain barrier. Stabilization techniques include cross-linking, multimerizing,
or linking to groups such as polyethylene glycol, polyacrylamide, neutral protein carriers,
etc. in order to achieve an increase in molecular weight.
Other strategies for increasing retention include the entrapment of the antibody,
such as a humanized anti-C1q antibody of the present disclosure, in a biodegradable or
bioerodible implant. The rate of release of the therapeutically active agent is controlled by
the rate of transport through the polymeric matrix, and the biodegradation of the implant.
The transport of drug through the polymer barrier will also be affected by compound
solubility, polymer hydrophilicity, extent of polymer cross-linking, expansion of the
polymer upon water absorption so as to make the polymer barrier more permeable to the
drug, geometry of the implant, and the like. The implants are of dimensions commensurate
with the size and shape of the region selected as the site of implantation. Implants may be
particles, sheets, patches, plaques, fibers, microcapsules and the like and may be of any size
or shape compatible with the selected site of insertion.

The implants may be monolithic, i.e. having the active agent homogenously
distributed through the polymeric matrix, or encapsulated, where a reservoir of active agent
is encapsulated by the polymeric matrix. The selection of the polymeric composition to be
employed will vary with the site of administration, the desired period of treatment, patient
tolerance, the nature of the disease to be treated and the like. Characteristics of the
polymers will include biodegradability at the site of implantation, compatibility with the
agent of interest, ease of encapsulation, a half-life in the physiological environment.
Biodegradable polymeric compositions which may be employed may be organic
esters or ethers, which when degraded result in physiologically acceptable degradation
products, including the monomers. Anhydrides, amides, orthoesters or the like, by
themselves or in combination with other monomers, may find use. The polymers will be
condensation polymers. The polymers may be cross-linked or non-cross-linked. Of
particular interest are polymers of hydroxyaliphatic carboxylic acids, either homo- or
copolymers, and polysaccharides. Included among the polyesters of interest are polymers
of D-lactic acid, L-lactic acid, racemic lactic acid, glycolic acid, polycaprolactone, and
combinations thereof. By employing the L-lactate or D-lactate, a slowly biodegrading
polymer is achieved, while degradation is substantially enhanced with the racemate.
Copolymers of glycolic and lactic acid are of particular interest, where the rate of
biodegradation is controlled by the ratio of glycolic to lactic acid. The most rapidly
degraded copolymer has roughly equal amounts of glycolic and lactic acid, where either
homopolymer is more resistant to degradation. The ratio of glycolic acid to lactic acid will
also affect the brittleness of in the implant, where a more flexible implant is desirable for
larger geometries. Among the polysaccharides of interest are calcium alginate, and
functionalized celluloses, particularly carboxymethylcellulose esters characterized by being
water insoluble, a molecular weight of about 5 kD to 500 kD, etc. Biodegradable hydrogels
may also be employed in the implants of the present disclosure. Hydrogels are typically a
copolymer material, characterized by the ability to imbibe a liquid. Exemplary
biodegradable hydrogels which may be employed are described in Heller in: Hydrogels in
Medicine and Pharmacy, N. A. Peppes ed., Vol. III, CRC Press, Boca Raton, Fla., 1987, pp
137-149.
Pharmaceutical dosages
Pharmaceutical compositions of the present disclosure containing a humanized
anti-C1q antibody of the present disclosure may be used (e.g., administered to an individual

in need of treatment with an anti-C1q antibody, such as a human individual) in accordance
with known methods, such as intravenous administration as a bolus or by continuous
infusion over a period of time, by intramuscular, intraperitoneal, intracerobrospinal,
intracranial, intraspinal, subcutaneous, intra-articular, intrasynovial, intrathecal, oral,
topical, or inhalation routes.
Dosages and desired drug concentration of pharmaceutical compositions of the
present disclosure may vary depending on the particular use envisioned. The determination
of the appropriate dosage or route of administration is well within the skill of an ordinary
artisan. Animal experiments provide reliable guidance for the determination of effective
doses for human therapy. Interspecies scaling of effective doses can be performed
following the principles described in Mordenti, J. and Chappell, W. "The Use of
Interspecies Scaling in Toxicokinetics," In Toxicokinetics and New Drug Development,
Yacobi et al., Eds, Pergamon Press, New York 1989, pp.42-46.
For in vivo administration of any of the humanized anti-C1q antibodies of the
present disclosure, normal dosage amounts may vary from about 10 ng/kg up to about 100
mg/kg of an individual's body weight or more per day, depending upon the route of
administration. In some embodiments, the dose amount is about 1 mg/kg/day to 10
mg/kg/day. For repeated administrations over several days or longer, depending on the
severity of the disease, disorder, or condition to be treated, the treatment is sustained until a
desired suppression of symptoms is achieved.
An exemplary dosing regimen may include administering an initial dose of a
humanized anti-C1q antibody, of about 2 mg/kg, followed by a weekly maintenance dose of
about 1 mg/kg every other week. Other dosage regimens may be useful, depending on the
pattern of pharmacokinetic decay that the physician wishes to achieve. For example,
dosing an individual from one to twenty-one times a week is contemplated herein. In
certain embodiments, dosing ranging from about 3 μg/kg to about 2 mg/kg (such as about 3
μg/kg, about 10 μg/kg, about 30 μg/kg, about 100 μg/kg, about 300 μg/kg, about 1 mg/kg,
or about 2 mg/kg) may be used. In certain embodiments, dosing frequency is three times
per day, twice per day, once per day, once every other day, once weekly, once every two
weeks, once every four weeks, once every five weeks, once every six weeks, once every
seven weeks, once every eight weeks, once every nine weeks, once every ten weeks, or
once monthly, once every two months, once every three months, or longer. Progress of the
therapy is easily monitored by conventional techniques and assays. The dosing regimen,

including the humanized anti-C1q antibody administered, can vary over time independently
of the dose used.
Dosages for a particular humanized anti-C1q antibody may be determined
empirically in individuals who have been given one or more administrations of the
humanized anti-C1q antibody. Individuals are given incremental doses of a humanized
anti-C1q antibody. To assess efficacy of a humanized anti-C1q antibody, any clinical
symptom of a neurodegenerative disorder, inflammatory disorder, or autoimmune disorder
can be monitored.
Administration of a humanized anti-C1q antibody of the present disclosure can
be continuous or intermittent, depending, for example, on the recipient's physiological
condition, whether the purpose of the administration is therapeutic or prophylactic, and
other factors known to skilled practitioners. The administration of a humanized anti-C1q
antibody may be essentially continuous over a preselected period of time or may be in a
series of spaced doses.
Guidance regarding particular dosages and methods of delivery is provided in
the literature; see, for example, U.S. Patent Nos. 4,657,760; 5,206,344; or 5,225,212. It is
within the scope of the present disclosure that different formulations will be effective for
different treatments and different disorders, and that administration intended to treat a
specific organ or tissue may necessitate delivery in a manner different from that to another
organ or tissue. Moreover, dosages may be administered by one or more separate
administrations, or by continuous infusion. For repeated administrations over several days
or longer, depending on the condition, the treatment is sustained until a desired suppression
of disease symptoms occurs. However, other dosage regimens may be useful. The
progress of this therapy is easily monitored by conventional techniques and assays.
Therapeutic uses
The present disclosure provides humanized anti-C1q antibodies, and antigen-
binding fragments thereof, which can bind to and neutralize a biologic activity of C1q.
These humanized anti-C1q antibodies are useful for preventing, reducing risk, or treating a
range of diseases associated with complement activation, including, without limitation,
neurodegenerative disorders, inflammatory disorders, and autoimmune disorders.
Accordingly, as disclosed herein, humanized anti-C1q antibodies of the present disclosure
may be used for treating, preventing, or reducing risk of a disease associated with
complement activation, including, without limitation, neurodegenerative disorders,

inflammatory disorders, and autoimmune disorders, in an individual. In some
embodiments, the individual has such a disease. In some embodiments, the individual is a
human.
Neurodegenerative disorders that may be treated with humanized anti-C1q
antibodies of this disclosure include disorders associated with loss of nerve connections or
synapses, including CF1-dependent synapse loss. Such disorders may include, without
limitation, Alzheimer's disease, amyotrophic lateral sclerosis, multiple sclerosis, glaucoma,
myotonic dystrophy, Guillain-Barre' syndrome (GBS), Myasthenia Gravis, Bullous
Pemphigoid, spinal muscular atrophy, Down syndrome, Parkinson's disease, and
Huntington's disease. In some neurodegenerative disorders, synapse loss is dependent on
the complement receptor 3 (CR3)/C3 or complement receptor CR1. In some
neurodegenerative disorders, synapse loss is associated with pathological activity-
dependent synaptic pruning. In some disorders, synapses are phagocytosed by microglia.
Accordingly, the humanized anti-C1q antibodies of the present disclosure may be used to
treat, prevent, or improve one or more symptoms of a neurodegenerative disorder of the
present disclosure. In some embodiments, the present disclosure provides methods of
treating, preventing, or improving one or more symptoms in individuals having a
neurodegenerative disorder of the present disclosure by administering a humanized anti-
C1q antibody of the present disclosure to, for example, inhibit the interaction between C1q
and an autoantibody, the interaction of C1q and C1r, and/or the interaction of C1q and C1s.
Inflammatory or autoimmune diseases that may be treated with humanized anti-
C1q antibodies of this disclosure include, without limitation, rheumatoid arthritis (RA),
acute respiratory distress syndrome (ARDS), remote tissue injury after ischemia and
reperfusion, complement activation during cardiopulmonary bypass surgery,
dermatomyositis, pemphigus, lupus nephritis and resultant glomerulonephritis and
vasculitis, cardiopulmonary bypass, cardioplegia-induced coronary endothelial dysfunction,
type II membranoproliferative glomerulonephritis, IgA nephropathy, acute renal failure,
cryoglobulemia, antiphospholipid syndrome, Chronic open-angle glaucoma, acute closed
angle glaucoma, macular degenerative diseases, age-related macular degeneration (AMD),
(AMD-wet), Geographic atrophy choroidal neovascularization (CNV), uveitis, diabetic
retinopathy, ischemia-related retinopathy, endophthalmitis, intraocular neovascular disease,
diabetic macular edema, pathological myopia, von Hippel-Lindau disease, histoplasmosis
of the eye, Neuromyelitis Optica (NMO), Central Retinal Vein Occlusion (CRVO), corneal

neovascularization, retinal neovascularization, Leber's hereditary optic neuropathy, optic
neuritis, Behcet's retinopathy, ischemic optic neuropathy, retinal vasculitis, ANCA
vasculitis, Purtscher retinopathy, Sjogren's dry eye disease, dry AMD, sarcoidosis,
temporal arteritis, polyarteritis nodosa, multiple sclerosis, as well as allo-transplantation,
hyperacute rejection, hemodialysis, chronic occlusive pulmonary distress syndrome
(COPD), asthma, and aspiration pneumonia. In some embodiments, autoimmune disease
may further include, without limitation, Guillain-Barré syndrome, myasthenia gravis,
Diabetes mellitus type 1, Hashimoto's thyroiditis, Addison's disease, Coeliac disease,
Crohn's disease, pernicious anaemia, Pemphigus vulgaris, vitiligo, autoimmune hemolytic
anemias, paraneoplastic syndromes, a vasculitis disease, hypocomplementemic urticarial
vasculitis (HUV), polymyalgia rheumatica, temporal arteritis, and Wegener's
granulomatosis.
In autoimmune diseases, such as Neuromyelitis Optica (NMO), autoantibodies
activate the complement system. In NMO patients, the classical complement pathway is
triggered by the binding of an autoantibody, such as an AQP4-targeted autoantibody, to its
autoantigen, AQP4. AQP4 thereby activates the classical pathway of complement
activation. In the first step of this activation process complement factor C1q binds to the
autoantibody-autoantigen-immune complex. Autoantibodies may include naturally
occurring antibodies, such as serum antibodies from NMO patients (commonly referred to
as NMO-IgG) or monoclonal antibodies, such as rAb-53.
Accordingly, the humanized anti-C1q antibodies of the present disclosure may
be used to treat, prevent, or improve one or more symptoms of an inflammatory or
autoimmune disease of the present disclosure. In some embodiments, the present
disclosure provides methods of treating, preventing, or improving one or more symptoms in
individuals having an inflammatory or autoimmune disease of the present disclosure by
administering a humanized anti-C1q antibody of the present disclosure to, for example,
inhibit the interaction between C1q and an autoantibody, the interaction of C1q and C1r,
and/or the interaction of C1q and C1s.
Metabolic diseases that may be treated with humanized anti-C1q antibodies
include, without limitation, diabetes, such as type II diabetes, and obesity. In vitro and in
vivo models of metabolic disorders that can be used for the testing of humanized anti-C1q
antibodies are well known in the art. Accordingly, the humanized anti-C1q antibodies of
the present disclosure may be used to treat, prevent, or improve one or more symptoms of a

metabolic disease of the present disclosure. In some embodiments, the present disclosure
provides methods of treating, preventing, or improving one or more symptoms in
individuals having metabolic disease of the present disclosure by administering a
humanized anti-C1q antibody of the present disclosure to, for example, inhibit the
interaction between C1q and an autoantibody, such as an anti-ganglioside autoantibody, the
interaction of C1q and C1r, and/or the interaction of C1q and C1s.
Combination Treatments
The antibodies of the present disclosure may be used, without limitation, in
combination with any additional treatment for neurodegenerative disorders, inflammatory
disorders, and/or autoimmune disorders.
In some embodiments, a humanized anti-C1q antibody of this disclosure is
administered in therapeutically effective amounts in combination with a second anti-
complement factor antibody (e.g., a neutralizing anti-complement factor antibody), such as
an anti-C1s or anti-C1r antibody, or a second anti-C1q antibody. In some embodiments, a
humanized anti-C1q antibody of this disclosure is administered in therapeutically effective
amounts with a second and a third neutralizing anti-complement factor antibody, such as a
second anti-C1q antibody, an anti-C1s antibody, and/or an anti-C1r antibody.
In some embodiments, the humanized anti-C1q antibodies of this disclosure are
administered in combination with an inhibitor of antibody-dependent cellular cytotoxicity
(ADCC). ADCC inhibitors may include, without limitation, soluble NK cell inhibitory
receptors such as the killer cell Ig-like receptors (KIRs), which recognize HLA-A, HLA-B,
or HLA-C and C-type lectin CD94/NKG2A heterodimers, which recognize HLA-E (see,
e.g., López-Botet M., T. Bellón, M. Llano, F. Navarro, P. García & M. de Miguel. (2000),
Paired inhibitory and triggering NK cell receptors for HLA class I molecules. Hum.
Immunol. 61: 7-17; Lanier L.L. (1998) Follow the leader: NK cell receptors for classical
and nonclassical MHC class I. Cell 92: 705-707.), and cadmium (see, e.g.,
Immunopharmacology 1990; Volume 20, Pages 73–8).
In some embodiments, the humanized anti-C1q antibodies of this disclosure are
administered in combination with an inhibitor of the alternative pathway of complement
activation. Such inhibitors may include, without limitation, factor B blocking antibodies,
factor D blocking antibodies, soluble, membrane-bound, tagged or fusion-protein forms of
CD59, DAF, CR1, CR2, Crry or Comstatin-like peptides that block the cleavage of C3,
non-peptide C3aR antagonists such as SB 290157, Cobra venom factor or non-specific

complement inhibitors such as nafamostat mesilate (FUTHAN; FUT-175), aprotinin, K-76
monocarboxylic acid (MX-1) and heparin (*see*, *e.g.*, T.E. Mollnes & M. Kirschfink,
*Molecular Immunology* 43 (2006) 107–121). In some embodiments, the humanized anti-
C1q antibodies of this disclosure are administered in combination with an inhibitor of the
interaction between the autoantibody and its autoantigen. Such inhibitors may include
purified soluble forms of the autoantigen, or antigen mimetics such as peptide or RNA-
derived mimotopes, including mimotopes of the AQP4 antigen. Alternatively, such
inhibitors may include blocking agents that recognize the autoantigen and prevent binding
of the autoantibody without triggering the classical complement pathway. Such blocking
agents may include, *e.g.*, autoantigen-binding RNA aptamers or antibodies lacking
functional C1q binding sites in their Fc domains (*e.g.*, Fab fragments or antibody otherwise
engineered not to bind C1q).
**Diagnostic uses**
**[0237]** The humanized anti-C1q antibodies of the present disclosure, or functional
fragments thereof, also have diagnostic utility. This disclosure therefore provides methods
of using the antibodies of this disclosure, or functional fragments thereof, for diagnostic
purposes, such as the detection of C1q in an individual or in tissue samples derived from an
individual. In some embodiments, the individual is a human. In some embodiments, the
individual is a human patient suffering from a neurodegenerative disorder or an
inflammatory, or autoimmune disease. In some embodiments, the humanized anti-C1q
antibodies of this disclosure are used to detect synapses and synapse loss. For example,
synapse loss may be measured in an individual suffering from a neurodegenerative disorder
such as Alzheimer's disease or glaucoma.
**[0238]** In some embodiments, the diagnostic methods involve the steps of administering
a humanized anti-C1q antibody of this disclosure, or functional fragment thereof, to an
individual and detecting the antibody bound to a synapse of the individual. Antibody-
binding to synapses may be quantified, for example, by non-invasive techniques such as
positron emission tomography (PET), X-ray computed tomography, single-photon emission
computed tomography (SPECT), computed tomography (CT), and computed axial
tomography (CAT).
**[0239]** In some embodiments, the diagnostic methods involve detecting synapses in a
biological sample, such as a biopsy specimen, a tissue, or a cell. A humanized anti-C1q
antibody, or functional fragment thereof, is contacted with the biological sample and

synapse-bound antibody is detected. The detection method may involve quantification of
the synapse-bound antibody. Antibody detection in biological samples may occur with any
method known in the art, including immunofluorescence microscopy,
immunocytochemistry, immunohistochemistry, ELISA, FACS analysis,
immunoprecipitation, or micro-positron emission tomography. In certain embodiments, the
antibody is radiolabeled, for example with ¹⁸F and subsequently detected utilizing micro-
positron emission tomography analysis.
The quantification of synapse-bound antibodies provides a relative measure for
the number of synapses present in the individual. Typically, synapses are quantified
repeatedly over a period of time. The exact periodicity of synapse quantification depends
on many factors, including the nature of the neurodegenerative disease, the stage of disease
progression, treatment modalities and many other factors. Repeat measurements commonly
reveal progressive synapse loss in individuals having a neurodegenerative disorder.
Alternatively, relative synapse counts may be compared in populations of diseased
individuals and healthy control individuals at a single time point. In diseased individuals
undergoing treatment, the treatment's efficacy can be assessed by comparing the rates of
synapse loss in the treated individuals with the rates of synapse loss in a control group.
Control group members have received either no treatment or a control treatment, such as a
placebo control.
Kits
The present disclosure also provides kits containing a humanized anti-C1q
antibody of the present disclosure, or a functional fragment thereof. Kits of the present
disclosure include one or more containers comprising a purified humanized anti-C1q
antibody of this disclosure. In some embodiments, the kits further include instructions for
use in accordance with the methods of this disclosure. In some embodiments, these
instructions comprise a description of administration of the humanized anti-C1q antibody to
treat or diagnose a disease associated with complement activation including, without
limitation a neurodegenerative disorder (e.g., Alzheimer's disease), inflammatory disease,
autoimmune disease, and/or metabolic disorder, according to any methods of this
disclosure. In some embodiments, the instructions comprise a description of how to detect
C1q, for example in an individual, in a tissue sample, or in a cell. The kit may further
comprise a description of selecting an individual suitable for treatment based on identifying
whether that individual has the disease and the stage of the disease.

The instructions generally include information as to dosage, dosing schedule,
and route of administration for the intended treatment. The containers may be unit doses,
bulk packages (e.g., multi-dose packages) or sub-unit doses. Instructions supplied in the
kits of the present disclosure are typically written instructions on a label or package insert
(e.g., a paper sheet included in the kit), but machine-readable instructions (e.g., instructions
carried on a magnetic or optical storage disk) are also acceptable.
The label or package insert indicates that the composition is used for treating,
e.g., a neurodegenerative disease. Instructions may be provided for practicing any of the
methods described herein.
The kits of this disclosure are in suitable packaging. Suitable packaging
includes, but is not limited to, vials, bottles, jars, flexible packaging (e.g., sealed Mylar or
plastic bags), and the like. Also contemplated are packages for use in combination with a
specific device, such as an inhaler, nasal administration device (e.g., an atomizer) or an
infusion device such as a minipump. A kit may have a sterile access port (for example the
container may be an intravenous solution bag or a vial having a stopper pierceable by a
hypodermic injection needle). The container may also have a sterile access port (e.g., the
container may be an intravenous solution bag or a vial having a stopper pierceable by a
hypodermic injection needle). At least one active agent in the composition is an inhibitor
of classical complement pathway. The container may further comprise a second
pharmaceutically active agent.
Kits may optionally provide additional components such as buffers and
interpretive information. Normally, the kit comprises a container and a label or package
insert(s) on or associated with the container.
The present disclosure will be more fully understood by reference to the
following Examples. They should not, however, be construed as limiting any aspect or
scope of the present disclosure in any way. All citations throughout the disclosure are
hereby expressly incorporated by reference.

EXAMPLES
Example 1: Production of Humanized Anti-C1q Antibodies
Introduction
This example describes the generation of fully humanized antibodies from the
murine hybridoma M1 (expressing the mouse anti-human C1q antibody M1). Composite

human antibody variable region genes were generated using synthetic oligonucleotides
encoding combinations of selected human sequence segments. These were then cloned into
vectors encoding human IgG4 (S241P L248E) heavy chain and human kappa light chain.
Humanized antibodies were stably expressed in NS0 (mouse myeloma cell-line) cells,
Protein A purified and tested for binding to human C1q using a competition ELISA assay
against biotinylated murine M1 antibody. Selected antibodies were also tested for binding
to mouse C1q using competition ELISA assay against biotinylated chimeric antibody.
Results
Sequencing of anti-human C1q V regions
RNA was extracted from the hybridoma cell pellet expressing M1 antibody
using an RNAqueousR-4PCR kit (Ambion cat. no. AM1914). Initially, RT-PCR was
performed using degenerate primer pools for murine signal sequences together with
constant region primers for both of IgG and Igκ. Heavy chain V region RNA was amplified
using a set of six degenerate primer pools (HA to HF) and light chain V region mRNA was
amplified using a set of seven degenerate primer pools for the κ cluster (KA to KG).
For the VH region, amplification products of the expected size were found in
IgG primer pools HB and HE. For the Vκ region, amplification products of the expected
size were found in kappa primer pools KC, KE, and KG. The PCR products obtained from
each of the successful amplifications were purified and cloned into a 'TA' cloning vector
(pGEM-T Easy, Promega cat. no. A1360) and sequenced. A total of 14 VH and 24 Vκ
clones were sequenced.
A single functional VH gene was identified in 14 clones from IgG pools HB and
HE. A single functional Vκ gene sequence was identified from 9 clones from primer pool
KC. The 3' coding sequence downstream of the variable region obtained from IgG primer
pools was consistent with the antibody isotype being IgG.
The functional VH and Vκ gene sequences were identical to the hybridoma
sequences with the exception of five amino acids at the beginning of the VH sequence and
two amino acids at the beginning of the Vκ sequence. These differences were most likely
due to the method of sequencing, and were a result of using primers that are degenerate to
the signal sequence rather than primers that are degenerate to 5' end of V regions.
The amino acid sequence of the functional VH is:
QVQLQQPGAELVKPGASVKLSCKSSGYHFTSYWMHWVKQRPGQGLEWIGVIHP
NSGSINYNEKFESKATLTVDKSSSTAYMQLSSLTSEDSAVYYCAGERDSTEVLPM

DYWGQGTSVTVSS (SEQ ID NO: 21). The hyper variable regions (HVRs) of the VH are
depicted in bolded and underlined text.
The amino acid sequence of the functional Vκ is:
DVQITQSPSYLAASPGETITINCRASKSINKYLAWYQEKPGKTNKLLIYSGSTLQSG
IPSRFSGSGSGTDFTLTISSLEPEDFAMYYCQQHNEYPLTFGAGTKLELK (SEQ ID
NO: 22). The hyper variable regions (HVRs) of the Vκ are depicted in bolded and
underlined text.
Construction of chimeric antibody
The VH and Vκ sequences of the murine M1 antibody were PCR amplified
using primers that introduced flanking restriction enzyme sites for cloning into the IgG4
(S241P L248E) heavy and kappa chain expression vectors (FIG. 1). The BamHI, HindIII
and SspI restriction sites were removed from the Vκ sequence in order to clone the gene.
The VH region was cloned using MluI and HindIII sites, and the Vκ region was cloned
using BssHII and BamHI restriction sites. Both constructs were confirmed by sequencing.
Design of composite human variable region sequences
Structural models of the murine M1 antibody V regions were produced using
Swiss PDB and analyzed in order to identify important "constraining" amino acids in the V
regions that were likely to be essential for the binding properties of the antibody. Most
residues contained within the HVRs (using both Kabat and Chothia definitions) together
with a number of framework residues were considered to be important. The VH and Vκ
sequences of M1 contain typical framework residues and the HVR 1, 2 and 3 motifs are
comparable to many murine antibodies.
From the above analysis, it was considered that composite human sequences of
M1 could be created with a wide latitude for alternative residues outside of the HVRs but
with only a narrow menu of possible residues within the HVR sequences. Preliminary
analysis indicated that corresponding sequence segments from several human antibodies
could be combined to create HVRs similar or identical to those in the murine sequences.
For regions outside of and flanking the HVRs, a wide selection of human sequence
segments were identified as possible components of the novel humanized V regions.
CD4⁺ T cell epitope avoidance
Based upon the structural analysis, a large preliminary set of sequence segments
that could be used to create M1 humanized variants were selected and analyzed using
iTope™ technology for in silico analysis of peptide binding to human MHC class II alleles

(Perry, LCA et al. Drugs R D. 2008;9(6):385-96), and using the TCED™ T-cell epitope
database of known antibody sequence-related T cell epitopes (Bryson, CJ et al. BioDrugs.
2010 Feb 1;24(1):1-8). Sequence segments that were identified as significant non-human
germline binders to human MHC class II or that scored significant hits against the TCED™
were discarded. This resulted in a reduced set of segments, and combinations of these were
again analyzed, as above, to ensure that the junctions between segments did not contain
potential T cell epitopes. Selected sequence segments were assembled into complete V
region sequences that were devoid of significant T cell epitopes. Four heavy chain
sequences (VH1-VH4) and four light chain sequences (Vκ1- Vκ4) were then chosen for
gene synthesis, expression in mammalian cells, and testing for activity.
Sequences of humanized heavy chain and light variable domains
Using standard techniques, the amino acid and nucleic acid sequences encoding
the heavy chain variable domain (VH) and kappa light chain variable domain (Vκ) variants
were determined.
The amino acid sequence of heavy chain variable domain variant 1 (VH1) is:


 (SEQ ID NO: 1). The hyper variable regions (HVRs) of VH1 are
depicted in bolded and underlined text.
The amino acid sequence of heavy chain variable domain variant 2 (VH2) is:


 (SEQ ID NO: 2). The hyper variable regions (HVRs) of VH2 are
depicted in bolded and underlined text.
The amino acid sequence of heavy chain variable domain variant 3 (VH3) is:
QVQLVQSGAELKKPGASVKVSCKSSGYHFTSYWMHWVKQAPGQGLEWIGVIHP
NSGSINYNEKFESRVTITVDKSTSTAYMELSSLRSEDTAVYYCAGERDSTEVLPM
DYWGQGTTVTVSS (SEQ ID NO: 3). The hyper variable regions (HVRs) of VH3 are
depicted in bolded and underlined text.
The amino acid sequence of heavy chain variable domain variant 4 (VH4) is:



 (SEQ ID NO: 4). The hyper variable regions (HVRs) of VH4 are
depicted in bolded and underlined text.
In some embodiments, the HVR-H1 of any one of VH1, VH2, VH3, or VH4 has
the sequence GYHFTSYWMH (SEQ ID NO: 23), the HVR-H2 of V any one of VH1,
VH2, VH3, or VH4 has the sequence VIHPNSGSINYNEKFES (SEQ ID NO: 24), and the
HVR-H3 of any one of VH1, VH2, VH3, or VH4 has the sequence ERDSTEVLPMDY
(SEQ ID NO: 25).
The nucleic acid sequence encoding heavy chain variable domain variant 1
(VH1) is:
CAGGTGCAGCTGGTGCAGTCAGGGGCTGAGCTGAAGAAGCCTGGGGCTTCAGT
GAAGGTTTCCTGCAAGTCTTCTGGCTACCATTTCACCAGCTACTGGATGCACTG
GGTGAAGCAGGCCCCTGGACAAGGCCTTGAGTGGATTGGAGTGATTCATCCTA
ATAGTGGTAGTATTAACTACAATGAGAAGTTCGAGAGCAAGGCCACAATTACT
GTAGACAAATCCACCAGCACAGCCTACATGCAACTCAGCAGCCTGACATCTGA
GGACTCGGCGGTCTATTATTGTGCAGGAGAGAGAGATTCTACGGAGGTTCTCCC
TATGGACTACTGGGGTCAAGGAACCTCAGTCACCGTCTCCTCA (SEQ ID NO: 26).
The nucleic acid sequence encoding heavy chain variable domain variant 2
(VH2) is:
CAGGTGCAGCTGGTGCAGTCAGGGGCTGAGCTGAAGAAGCCTGGGGCTTCAGT
GAAGGTTTCCTGCAAGTCTTCTGGCTACCATTTCACCAGCTACTGGATGCACTG
GGTGAAGCAGGCCCCTGGACAAGGCCTTGAGTGGATTGGAGTGATTCATCCTA
ATAGTGGTAGTATTAACTACAATGAGAAGTTCGAGAGCAGAGCCACAATTACT
GTAGACAAATCCACCAGCACAGCCTACATGGAGCTCAGCAGCCTGAGATCTGA
GGACACGGCGGTCTATTATTGTGCAGGAGAGAGAGATTCTACGGAGGTTCTCCC
TATGGACTACTGGGGTCAAGGAACCACGGTCACCGTCTCCTCA (SEQ ID NO: 27).
The nucleic acid sequence encoding heavy chain variable domain variant 3
(VH3) is:
CAGGTGCAGCTGGTGCAGTCAGGGGCTGAGCTGAAGAAGCCTGGGGCTTCAGT
GAAGGTTTCCTGCAAGTCTTCTGGCTACCATTTCACCAGCTACTGGATGCACTG
GGTGAAGCAGGCCCCTGGACAAGGCCTTGAGTGGATTGGAGTGATTCATCCTA
ATAGTGGTAGTATTAACTACAATGAGAAGTTCGAGAGCAGAGTCACAATTACT
GTAGACAAATCCACCAGCACAGCCTACATGGAGCTCAGCAGCCTGAGATCTGA
GGACACGGCGGTCTATTATTGTGCAGGAGAGAGAGATTCTACGGAGGTTCTCCC

TATGGACTACTGGGGTCAAGGAACCACGGTCACCGTCTCCTCAG (SEQ ID NO:
28).
The nucleic acid sequence encoding heavy chain variable domain variant 4
(VH4) is:
CAGGTGCAGCTGGTGCAGTCAGGGGCTGAGCTGAAGAAGCCTGGGGCTTCAGT
GAAGGTTTCCTGCAAGTCTTCTGGCTACCATTTCACCAGCTACTGGATGCACTG
GGTGCGACAGGCCCCTGGACAAGGCCTTGAGTGGATTGGAGTGATTCATCCTA
ATAGTGGTAGTATTAACTACAATGAGAAGTTCGAGAGCAGAGTCACAATTACT
GTAGACAAATCCACCAGCACAGCCTACATGGAGCTCAGCAGCCTGAGATCTGA
GGACACGGCGGTCTATTATTGTGCAGGAGAGAGAGATTCTACGGAGGTTCTCCC
TATGGACTACTGGGGTCAAGGAACCACGGTCACCGTCTCCTCA (SEQ ID NO: 29).
The amino acid sequence of kappa light chain variable domain variant 1 (Vκ1)
is:

 (SEQ ID
NO: 5). The hyper variable regions (HVRs) of Vκ1 are depicted in bolded and underlined
text.
The amino acid sequence of kappa light chain variable domain variant 2 (Vκ2)
is:

 (SEQ ID
NO: 6). The hyper variable regions (HVRs) of Vκ2 are depicted in bolded and underlined
text.
The amino acid sequence of kappa light chain variable domain variant 3 (Vκ3)
is:
DVQITQSPSSLSASLGERATINCRASKSINKYLAWYQQKPGKAPKLLIYSGSTLQSG
IPARFSGSGSGTDFTLTISSLEPEDFAMYYCQQHNEYPLTFGQGTKLEIK (SEQ ID
NO: 7). The hyper variable regions (HVRs) of Vκ3 are depicted in bolded and underlined
text.
The amino acid sequence of kappa light chain variable domain variant 4 (Vκ4)
is:

 (SEQ ID

NO: 8). The hyper variable regions (HVRs) of Vκ4 are depicted in bolded and underlined
text.
In some embodiments, the HVR-L1 of any one of Vκ1, Vκ2, Vκ3, or Vκ4 has
the sequence has the sequence RASKSINKYLA (SEQ ID NO: 30), the HVR-L2 of any one
of Vκ1, Vκ2, Vκ3, or Vκ4 has the sequence SGSTLQS (SEQ ID NO: 31), and the HVR-L3
of any one of Vκ1, Vκ2, Vκ3, or Vκ4 has the sequence QQHNEYPLT (SEQ ID NO: 32).
The nucleic acid sequence encoding kappa light chain variable domain variant 1
(Vκ1) is:
GATGTCCAGATCACACAGTCTCCATCTTATCTTGCTGCATCTCTCGGAGAAAGA
GCTACTATTAATTGCAGGGCAAGTAAGAGCATTAACAAATACTTAGCCTGGTAT
CAACAGAAACCTGGGAAAACTAATAAGCTCCTTATCTACTCTGGCTCCACTTTG
CAATCTGGAATTCCAGCAAGGTTCAGTGGCAGTGGATCTGGTACAGATTTCACT
CTCACCATCAGTAGCCTGGAGCCTGAAGATTTTGCAATGTATTACTGTCAACAA
CATAATGAATACCCGCTCACGTTCGGTCAGGGGACCAAGCTGGAGATCAAA
(SEQ ID NO: 33).
The nucleic acid sequence encoding kappa light chain variable domain variant 2
(Vκ2) is:
GATGTCCAGATCACACAGTCTCCATCTTCCCTTTCTGCATCTCTCGGAGAAAGA
GCTACTATTAATTGCAGGGCAAGTAAGAGCATTAACAAATACTTAGCCTGGTAT
CAACAGAAACCTGGGAAAGCTAATAAGCTCCTTATCTACTCTGGCTCCACTTTG
CAATCTGGAATTCCAGCAAGGTTCAGTGGCAGTGGATCTGGTACAGATTTCACT
CTCACCATCAGTAGCCTGGAGCCTGAAGATTTTGCAATGTATTACTGTCAACAA
CATAATGAATACCCGCTCACGTTCGGTCAGGGGACCAAGCTGGAGATCAAA
(SEQ ID NO: 34).
The nucleic acid sequence encoding kappa light chain variable domain variant 3
(Vκ3) is:
GATGTCCAGATCACACAGTCTCCATCTTCCCTTTCTGCATCTCTCGGAGAAAGA
GCTACTATTAATTGCAGGGCAAGTAAGAGCATTAACAAATACTTAGCCTGGTAT
CAACAGAAACCTGGGAAAGCTCCTAAGCTCCTTATCTACTCTGGCTCCACTTTG
CAATCTGGAATTCCAGCAAGGTTCAGTGGCAGTGGATCTGGTACAGATTTCACT
CTCACCATCAGTAGCCTGGAGCCTGAAGATTTTGCAATGTATTACTGTCAACAA
CATAATGAATACCCGCTCACGTTCGGTCAGGGGACCAAGCTGGAGATCAAA
(SEQ ID NO: 35).

The nucleic acid sequence encoding kappa light chain variable domain variant 4 (Vκ4) is:
GATATTCAGCTCACACAGTCTCCATCTTCCCTTTCTGCATCTCTCGGAGAAAGA
GCTACTATTAATTGCAGGGCAAGTAAGAGCATTAACAAATACTTAGCCTGGTAT
CAACAGAAACCTGGGAAAGCTCCTAAGCTCCTTATCTACTCTGGCTCCACTTTG
CAATCTGGAATTCCAGCAAGGTTCAGTGGCAGTGGATCTGGTACAGATTTCACT
CTCACCATCAGTAGCCTGGAGCCTGAAGATTTTGCAATGTATTACTGTCAACAA
CATAATGAATACCCGCTCACGTTCGGTCAGGGGACCAAGCTGGAGATCAAA
(SEQ ID NO: 36).
Sequences of human IgG4 (S241P L248E) heavy chain constant domain
Using standard techniques, the amino acid and nucleic acid sequences encoding the human IgG4 (S241P L248E) heavy chain constant domain (i.e., CH1, CH2, CH3, and hinge region,) were determined.
The amino acid sequence of human IgG4 (S241P L248E) heavy chain constant domain is:
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
EGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK (SEQ ID NO: 37). The S241P mutation and the L248E mutation are depicted in bolded and underlined text.
The amino acid sequence of the human IgG4 (S241P L248E) CH1 is:
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRV (SEQ ID NO: 38).
The amino acid sequence of the human IgG4 (S241P L248E) hinge region is:
ESKYGPPCPPCP (SEQ ID NO: 39). The S241P mutation is depicted in bolded and underlined text.
The amino acid sequence of the human IgG4 (S241P L248E) CH2 is:
APEFEGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAK
(SEQ ID NO: 40). The L248E mutation is depicted in bolded and underlined text.

The amino acid sequence of the human IgG4 (S241P L248E) CH3 is:
GQPREPQVYTLPPSQEEMTKNQVSLTCLVKGFYPSDIAVEWESNGQPENNYKTTPP
VLDSDGSFFLYSRLTVDKSRWQEGNVFSCSVMHEALHNHYTQKSLSLSLGK (SEQ
ID NO: 41).
The nucleic acid sequence encoding human IgG4 (S241P L248E) heavy chain
constant domain is:
GTAAGCTTTCTGGGGCAGGCCGGGCCTGACTTTGGCTGGGGGCAGGGAGGGGG
CTAAGGTGACGCAGGTGGCGCCAGCCAGGTGCACACCCAATGCCCATGAGCCC
AGACACTGGACCCTGCATGGACCATCGCGGATAGACAAGAACCGAGGGGCCTC
TGCGCCCTGGGCCCAGCTCTGTCCCACACCGCGGTCACATGGCACCACCTCTCT
TGCAGCTTCCACCAAGGGCCCATCCGTCTTCCCCCTGGCGCCCTGCTCCAGGAG
CACCTCCGAGAGCACAGCCGCCCTGGGCTGCCTGGTCAAGGACTACTTCCCCGA
ACCGGTGACGGTGTCGTGGAACTCAGGCGCCCTGACCAGCGGCGTGCACACCT
TCCCGGCTGTCCTACAGTCCTCAGGACTCTACTCCCTCAGCAGCGTGGTGACCG
TGCCCTCCAGCAGCTTGGGCACGAAGACCTACACCTGCAATGTAGATCACAAG
CCCAGCAACACCAAGGTGGACAAGAGAGTTGGTGAGAGGCCAGCACAGGGAG
GGAGGGTGTCTGCTGGAAGCCAGGCTCAGCCCTCCTGCCTGGACGCACCCCGG
CTGTGCAGCCCCAGCCCAGGGCAGCAAGGCAGGCCCCATCTGTCTCCTCACCTG
GAGGCCTCTGACCACCCCACTCATGCTCAGGGAGAGGGTCTTCTGGATTTTTCC
ACCAGGCTCCGGGCAGCCACAGGCTGGATGCCCCTACCCCAGGCCCTGCGCAT
ACAGGGGCAGGTGCTGCGCTCAGACCTGCCAAGAGCCATATCCGGGAGGACCC
TGCCCCTGACCTAAGCCCACCCCAAAGGCCAAACTCTCCACTCCCTCAGCTCAG
ACACCTTCTCTCCTCCCAGATCTGAGTAACTCCCAATCTTCTCTCTGCAGAGTCC
AAATATGGTCCCCCATGCCCACCATGCCCAGGTAAGCCAACCCAGGCCTCGCCC
TCCAGCTCAAGGCGGGACAGGTGCCCTAGAGTAGCCTGCATCCAGGGACAGGC
CCCAGCCGGGTGCTGACGCATCCACCTCCATCTCTTCCTCAGCACCTGAGTTCG
AGGGGGGACCATCAGTCTTCCTGTTCCCCCCAAAACCCAAGGACACTCTCATGA
TCTCCCGGACCCCTGAGGTCACGTGCGTGGTGGTGGACGTGAGCCAGGAAGAC
CCCGAGGTCCAGTTCAACTGGTACGTGGATGGCGTGGAGGTGCATAATGCCAA
GACAAAGCCGCGGGAGGAGCAGTTCAACAGCACGTACCGTGTGGTCAGCGTCC
TCACCGTCCTGCACCAGGACTGGCTGAACGGCAAGGAGTACAAGTGCAAGGTC
TCCAACAAAGGCCTCCCGTCCTCCATCGAGAAAACCATCTCCAAAGCCAAAGG
TGGGACCCACGGGGTGCGAGGGCCACATGGACAGAGGTCAGCTCGGCCCACCC

TCTGCCCTGGGAGTGACCGCTGTGCCAACCTCTGTCCCTACAGGGCAGCCCCGA
GAGCCACAGGTGTACACCCTGCCCCCATCCCAGGAGGAGATGACCAAGAACCA
GGTCAGCCTGACCTGCCTGGTCAAAGGCTTCTACCCCAGCGACATCGCCGTGGA
GTGGGAGAGCAATGGGCAGCCGGAGAACAACTACAAGACCACGCCTCCCGTGC
TGGACTCCGACGGCTCCTTCTTCCTCTACAGCAGGCTAACCGTGGACAAGAGCA
GGTGGCAGGAGGGGAATGTCTTCTCATGCTCCGTGATGCATGAGGCTCTGCACA
ACCACTACACACAGAAGAGCCTCTCCCTGTCTCTGGGTAAA (SEQ ID NO: 42).
The nucleic acid sequence encoding the human IgG4 (S241P L248E) heavy
chain CH1 is:
CTTCCACCAAGGGCCCATCCGTCTTCCCCCTGGCGCCCTGCTCCAGGAGCACCT
CCGAGAGCACAGCCGCCCTGGGCTGCCTGGTCAAGGACTACTTCCCCGAACCG
GTGACGGTGTCGTGGAACTCAGGCGCCCTGACCAGCGGCGTGCACACCTTCCCG
GCTGTCCTACAGTCCTCAGGACTCTACTCCCTCAGCAGCGTGGTGACCGTGCCC
TCCAGCAGCTTGGGCACGAAGACCTACACCTGCAATGTAGATCACAAGCCCAG
CAACACCAAGGTGGACAAGAGAGTTG (SEQ ID NO: 43).
The nucleic acid sequence encoding the human IgG4 (S241P L248E) heavy
chain hinge is:
AGTCCAAATATGGTCCCCCATGCCCACCATGCCCAG (SEQ ID NO: 44).
The nucleic acid sequence encoding the human IgG4 (S241P L248E) heavy
chain CH2 is:
CACCTGAGTTCGAGGGGGGACCATCAGTCTTCCTGTTCCCCCCAAAACCCAAGG
ACACTCTCATGATCTCCCGGACCCCTGAGGTCACGTGCGTGGTGGTGGACGTGA
GCCAGGAAGACCCCGAGGTCCAGTTCAACTGGTACGTGGATGGCGTGGAGGTG
CATAATGCCAAGACAAAGCCGCGGGAGGAGCAGTTCAACAGCACGTACCGTGT
GGTCAGCGTCCTCACCGTCCTGCACCAGGACTGGCTGAACGGCAAGGAGTACA
AGTGCAAGGTCTCCAACAAAGGCCTCCCGTCCTCCATCGAGAAAACCATCTCCA
AAGCCAAAG (SEQ ID NO: 45).
The nucleic acid sequence encoding the human IgG4 (S241P L248E) heavy
chain CH3 is:
GGCAGCCCCGAGAGCCACAGGTGTACACCCTGCCCCCATCCCAGGAGGAGATG
ACCAAGAACCAGGTCAGCCTGACCTGCCTGGTCAAAGGCTTCTACCCCAGCGA
CATCGCCGTGGAGTGGGAGAGCAATGGGCAGCCGGAGAACAACTACAAGACC
ACGCCTCCCGTGCTGGACTCCGACGGCTCCTTCTTCCTCTACAGCAGGCTAACC

GTGGACAAGAGCAGGTGGCAGGAGGGGAATGTCTTCTCATGCTCCGTGATGCA
TGAGGCTCTGCACAACCACTACACACAGAAGAGCCTCTCCCTGTCTCTGGGTAA
A (SEQ ID NO: 46).
FIG. 2 shows an alignment of the amino acid sequences of the heavy chain
variable region (VH) of M1 and the amino acid sequences of the humanized VH variants
VH1-VH4, and of the amino acid sequences of the kappa light chain variable region (Vκ)
of M1 and the amino acid sequences of the humanized Vκ variants Vκ1-Vκ4.
Construction of composite human antibody variants
All variant VH and Vκ region genes for M1 were synthesized using a series of
overlapping oligonucleotides that were annealed, ligated and PCR amplified to give full
length synthetic V regions with any restriction sites removed. The assembled variants were
then cloned directly into the pANT expression vector system for IgG4 (S241 L248E) heavy
chain and kappa light chain (FIG. 1). The VH region was cloned using MluI and HindIII
sites, and the Vκ region was cloned using BssHII and BamHI restriction sites. All
constructs were confirmed by sequencing.
Expression and purification of antibodies
A total of 16 fully humanized antibodies were stably transfected into NS0 cells
via electroporation. In addition, the chimeric antibody M1 along with two controls –
chimeric VH (ChVH) with variant Vκ1 and variant VH1 with chimeric Vκ (ChVκ) - were
included. Stable transfectants were selected using 200 nM methotrexate (Sigma Cat. No.
M8407). Methotrexate-resistant colonies for each construct were tested for IgG expression
levels using an IgG4 ELISA, and the best expressing lines were selected, expanded and
frozen under liquid nitrogen. Successful transfection and stable clone selection was
achieved for all of the 16 humanized M1 variants as well as chimeric M1, ChVH/Vκ1 and
VH1/ChVκ antibodies. The identity of each cell line was confirmed by DNA sequencing of
the variable domains from genomic DNA.
Antibodies were purified from cell culture supernatants on a Protein A
sepharose column (GE Healthcare cat. co. 110034-93), buffer exchanged into PBS, pH 7.4
and quantified by OD280nm using an extinction coefficient based on the predicted amino acid
sequences. Chimeric and humanized variant IgGs were analyzed by reducing SDS-PAGE.
Bands corresponding to the predicted sizes of the VH and Vκ chains were observed with no
evidence of any aggregation, degradation or other unusual features (FIG. 3).

Competition ELISA against human C1q antigen
The binding of humanized M1 variants to human C1q was analyzed by
competition ELISA.  A three-fold dilution series of test antibody (5 μg/ml to 0.002 μg/ml)
was premixed with a constant concentration of biotinylated mouse M1 antibody (0.02
μg/ml, final concentration) before incubating for 1 hour at 37°C with shaking on plates pre-
coated with human C1q at 1.0μg/ml. The binding of mouse M1 antibody was detected with
streptavidin-peroxidase conjugate (Sigma-Aldrich cat. no. S5512) and TMB (3,3′,5,5′-
tetramethylbenzidine) substrate (Thermo Scientific cat. no. 34029). The reaction was
stopped with 1M HCl, absorbance read at 450 nm on a Dynex Technologies MRX TC II
plate reader and the binding curves plotted.
FIG. 4 shows that all humanized M1 variants generated have similar binding
profiles to the chimeric M1 antibody. The binding curves were used to calculate IC50 values
(concentration of test antibody that inhibits labelled competitor binding by 50%) for each
antibody normalized to the IC50 of chimeric M1 and antibody yields from NS0 transfectants
were also compared (Table 1).
TABLE 1: Relative IC50 values for binding to human C1q and protein expression
levels
Antibody | Relative IC50 | Expression levels (μg/ml)
Chimeric M1 | 1.00 | 12.6
ChV1/Vκ1 | 1.09 | 7.0
VH1/ChVκ | 0.92 | 11.9
VH1/Vκ1 | 0.90 | 14.0
VH1/Vκ2 | 0.84 | 14.5
VH1/Vκ3 | 0.91 | 28.9
VH1/Vκ4 | 0.80 | 22.6
VH2/Vκ1 | 1.15 | 1.4
VH2/Vκ2 | 1.12 | 3.8
VH2/Vκ3 | 0.75 | 21.3
VH2/Vκ4 | 0.72 | 6.1
VH3/Vκ1 | 0.65 | 16.9
VH3/Vκ2 | 0.82 | 8.7
VH3/Vκ3 | 0.63 | 19.8
VH3/Vκ4 | 0.83 | 32.2
VH4/Vκ1 | 1.03 | 8.5
VH4/Vκ2 | 0.84 | 1.6
VH4/Vκ3 | 0.77 | 18.3
VH4/Vκ4 | 0.92 | 2.4

Table 1 shows calculated relative IC50 values for humanized M1 variants
binding to human C1q and protein expression levels of corresponding NS0 cell line.

The normalized IC50 data for all variants tested were in the range of 0.63 to 1.15
indicating that the binding efficiencies of all of the fully humanized M1 antibodies to
human C1q were comparable to that of chimeric M1. Furthermore, most humanized
variants achieved an increase in expression level compared to the chimeric antibody.
Competition ELISA against mouse C1q antigen
The binding of humanized M1 variants to mouse C1q was analyzed by
competition ELISA on four selected antibodies, VH1/Vκ1, VH3/Vκ3, VH3/Vκ4 and
VH4/Vκ3. An irrelevant IgG4 (S241P L248E) antibody was also included as a binding
control. A three-fold dilution series of test antibody (100 μg/ml to 0.046 μg/ml) was
premixed with a constant concentration of biotinylated chimeric M1 antibody (0.03 μg/ml,
final concentration) before incubating for 1 hour at 37°C with shaking on plates pre-coated
with mouse C1q at 5.0μg/ml. The binding of chimeric M1 antibody was detected with
streptavidin-peroxidase conjugate (Sigma-Aldrich cat. no. S5512) and TMB substrate
(Thermo Scientific cat. no. 34029). The reaction was stopped with 1M HCl, absorbance
read at 450 nm on a Dynex Technologies MRX TC II plate reader and the binding curves
plotted.
FIG. 5 shows that humanized M1 variants generated have similar binding
profiles to the chimeric M1 antibody. The binding curves were used to calculate IC50 values
for each antibody normalized to the IC50 of chimeric M1 (Table 2).
TABLE 2: Relative IC50 values for binding to mouse C1q
Antibody | Relative IC50
Chimeric M1 | 1.00
VH1/Vκ1 | 1.62
VH3/Vκ3 | 1.50
VH3/Vκ4 | 1.91
VH4/Vκ3 | 1.84

Table 2 shows calculated relative IC50 values for humanized M1 variants
binding to mouse C1q.
Conclusion
The V region genes from the murine antibody M1 were cloned into vectors to
generate a chimeric antibody comprising the murine V regions combined with the human
IgG4 (S241P L248E) heavy chain constant region and κ light chain constant regions.
Additionally, a series of four humanized VH regions for IgG4 (S241P L248E) and four
humanized Vκ regions were designed and constructed.

The chimeric antibody and the combinations of humanized V region genes (16
antibodies in total) were expressed in NS0 cells, purified and tested for binding to human
C1q in a competition ELISA assay. The binding data (Table 1) were used to rank the
humanized M1 variants in comparison with the chimeric M1 antibody. No significant
differences in quality of the heavy and light chain bands were detected by SDS-PAGE.
Example 2: Kinetic Characterization of Humanized Anti-C1q Antibody VH3/Vκ3
Introduction
Immunological biosensors, for example Biacore™ surface plasmon resonance
(SPR) instruments, that measure the binding and dissociation of antigen-antibody
complexes in real time allow the elucidation of binding kinetics. The rate of dissociation
and its subsequent optimization is especially important for biopharmaceutical antibody
development.
The Biacore uses SPR to monitor the interaction between a surface bound
molecule 'ligand' and its binding partner in solution 'analyte', in real time. SPR is an
electron charge-density wave phenomenon, which arises at the surface of a metallic layer
when light is reflected at the layer under conditions of total internal reflectance. The surface
plasmons that are generated are sensitive to any changes in the refractive index of the
medium on the opposite side of the metallic layer from the reflected light. Protein-protein
interactions occurring at the surface affect the refractive index of the medium and can
therefore be detected. Binding of molecules to the ligand modified sensor surface generates
a response, which is proportional to the bound mass allowing small changes in the amount
of bound analyte to be detected (down to low picogram levels). The technique can be used
to measure affinity constants (K_{D}) over the range 10⁻⁵ M to 10⁻¹² M, association rate
constants (kₐ) between 10³ M⁻¹s⁻¹ and 10⁷ M⁻¹s⁻¹, and dissociation rate constants (k_{d})
between 10⁻¹ s⁻¹and 10⁻⁶ s⁻¹.
The technique requires only small amounts of material and both of the
interacting biomolecules can be used in a label-free form. Experimental design is important,
however, as some features of the technology, and the fact that one of the proteins must be
surfaced attached, can give rise to misleading results (Huber and Mueller, Curr Pharm Des.
2006;12(31):3999-4021; and Lakey and Raggett, Curr Opin Struct Biol., 1998. 8(1): p.
119-123).
This example describes the kinetic characterization of the interaction between
the humanized anti-C1q antibody VH3/Vκ3 (both Fab fragment and full-length IgG) and

the C1q protein utilizing the Biacore T200 surface plasmon resonance instrument for the
high resolution.
Materials and Methods
Samples
The reagents used in this example are listed in Table 3.
TABLE 3: Samples
Sample | Volume at concentration (mg/ml)
IgG-VH3/Vκ3 | 400 μl at 1.2 mg/ml
Fab-VH3/Vκ3 | 450 μl at 0.3 mg/ml
Mouse C1q | 4 x 50 μl at 1.0 mg/ml
Human C1q | 10 x 50 μl at 1.0 mg/ml

Mouse and human C1q were stored at −80°C, except once defrosted they were
stored at + 4°C. Fab and IgG VH3/Vκ3 were stored at +4°C. Once diluted, C1q solutions
were kept on ice and used within 24 hours.
Equipment
A Biacore T200 instrument (SN: CN 12231) running Biacore T200 Evaluation
Software V1.1 (Uppsala, Sweden) was used.
Materials
The following materials were obtained from Biacore as follows:
Biacore Preventative Maintenance Kit 2: BR-1006-51, Lot No. 164110
Series S CM5 Sensor Chips: BR-1006-68, Lot No. 10102398
Amine Coupling Kit: BR-1000-50, Lot No. 2027942/41
10 mM Acetate pH 5.0: BR-1003-51, Lot No. 21702813
HBS-EP Running buffer: BR-1006-69, Lot No. 2027942/59
BSA was obtained from Sigma (A3294).
Procedures
All experiments were developed with Biacore 'wizard' software. The following
Biacore methods were used:
Immobilization
Kinetics/Affinity
Desorb and Sanitize
Results
VH3/Vκ3 Fab preparation
The Fab fragment of the anti-C1q humanized antibody VH3/Vκ3 was prepared
using a Fab Micro Preparation Kit following the manufacturer's protocol. The starting
concentration of the full-length IgG VH3/Vκ3 was 1.88 mg/ml. To obtain sufficient amount

of the Fab fragments, 6 reactions of 225 μg each of full-length IgG were digested, pooled,
and purified. The purified Fab and the full-length IgG were further purified by size
exclusion chromatography using a Superdex 200 Increase 10/300 GL column (GE
Healthcare Cat. No. 28-9909-44) with 1x PBS as running buffer. Samples were quantified
by OD280nm using an extinction coefficient (EC(0.1%)) based on the predicted amino acid
sequence (Ec(0.1%) = 1.45 for IgG VH3/Vκ3 and 1.4 for Fab VH3/Vκ3). Both samples were
analyzed by non-reducing and reducing SDS-PAGE (FIG. 6). FIG. 6 depicts a Coomassie
Blue-stained SDS-PAGE gel of gel filtration-purified antibodies. 1 μg of each sample was
loaded on a NuPage 4-12% Bis-Tris gel (Invitrogen Cat. No. NP0322BOX) and run at
200V for 35min. Size marker (M) is prestained protein standard Fermentas PageRuler Plus
(Cat. No. SM1811). Lane 1 shows the reduced VH3/Vκ3 Fab; lane 2 shows the non-
reduced VH3/Vκ3 Fab; lane 3 shows the reduced VH3/Vκ3 IgG; and lane 4 shows the non-
reduced VH3/Vκ3 IgG.
Antigen preparation
The samples of mouse C1q (mC1q) and human C1q (hC1q) antigens were stored
at
-80°C, and upon initial defrosting, multiple aliquots were prepared, re-frozen and stored at -
80°C. Further dilutions of the analytes into HBS-EP (10 mM HEPES pH 7.4 and 150 mM
NaCl, 3 mM EDTA and 0.05 % (v/v) P20) were performed for the kinetic runs.
Instrument preparation
Before running any samples, and during the study, a system check (Biacore
Preventative Maintenance Kit 2) was performed. All the systems tested passed (Reagent
pump, Refractometer, Injections, Noise, Mixing and Buffer Selector) indicating that the
instrument was performing to criteria set by the manufacturer.
System preparation
Upon insertion of a CM5 chip the system was primed and then normalized with
BIA normalizing solution (Biacore Preventative Maintenance Kit 2). All samples were run
at 25°C with a sample rack incubated at 10°C. The chip was added to the system with HBS-
EP used as the running buffer; prior to immobilization the chip surface was primed with
two injections of 50 mM NaOH.
Immobilization conditions
Due to concerns about stability, two chips were prepared; one with hC1q and
mC1q (Chip A11) as ligands and one with IgG and Fab (Chip A13) as ligands.

Immobilization for m/h C1q was carried out at a protein concentration of 5 μg/ml in 10 mM
acetate buffer pH 5.5, whereas immobilization for IgG and Fab was carried out at a protein
concentration of 0.5-2 μg/ml in 10 mM acetate buffer pH 5.0, both on a CM5 Series S
sensor chip (Biacore). The final response levels for Chips A11 and A13 used in the kinetic
analysis are shown in Table 4.
TABLE 4: Final response level (RU)
| Fc1 | Fc2 | Fc3 | Fc4
A11 | Blank | hC1q
808.3 | mC1q
801.3 | mC1q
824.1
A13 | Blank | Fab
10.4 | IgG
12.8 | IgG
51.9

Table 4 depicts the final immobilization levels achieved from Chips A11 and
A13 for each flow cell (Fc).
For kinetic experiments the amount of immobilized/captured ligand needs to be
limited to avoid mass transfer effects at the surface of the chip and the surface should
ideally have an analyte maximum binding response (Rmax) of 100-150 response uints (RUs).
The amount of ligand to immobilize is therefore calculated using Equation 1:
Analyte Binding Capacity(RU) = analyte Mw / ligand Mw × immobilised ligand(RU) × Sm
An average MW of 410 kDa (literature and reagent manufacturers) for both
mC1q and hC1q, 150 kDa for IgG (estimated value for antibodies), and 50 kDa for the Fab
(estimated) were employed. Aiming for 100 RU for Rmax and the stoichiometry (Sm) as 1,
an ideal target amount of C1q to immobilize would be ~ 820 RUs. Due to concerns
associated with avidity for the Fab and IgG immobilized surfaces, the amount of
immobilized ligand was kept as low as possible (limit of 10 RUs in Biacore immobilization
software).
Non-specific binding (NSB) control
Non-specific binding can be due to either the analyte or analyte contaminants,
interacting with the ligand (non-specific and difficult to detect), the capture protein, or the
sensor chip surface. When analyzing the response of the blank Fc1 surface after a relatively
high concentration (500 nM), 300 second injection of both mC1q and hC1q, significant
non-specific binding (NSB) was observed. An additional blocking step, a post-ligand
immobilization of 50 μg/ml BSA (10 mM acetate pH 4.25) was therefore included (Moore
et al., MAbs. 2010 Mar-Apr;2(2):181-9). The BSA blocking step was also repeated on the

reference channel (Fc1); for both, immobilization levels were ~8000 RU. No NSB was
observed using Fab, or IgG at 500 nM on the CM5 surface, and at the concentration used in
the kinetic runs, no NSB to the BSA blocked surface was seen.
Regeneration scouting
Where required, either a single injection or two sequential 30 second injections
of
1 M NaCl / 50 mM NaOH were used to regenerate all surfaces. A 180 second wait step was
introduced after the last regeneration injection to allow the surface to stabilize before
starting the next binding cycle.
Surface performance
The performance of the surface was analyzed by repeated control injections of
analyte at the start, interspersed and at the end of a kinetic run. Stable binding was typically
observed throughout the kinetic run, highlighting the suitability of the system for kinetic
analysis.

Mass transfer control
Mass transport limitation occurs when the rate of association contains a
significant component associated with the rate of transport of the analyte to and from the
chip surface. Where mass transfer is found to be significant, the resulting kinetic analysis
could be inaccurate. Lowering the density of immobilized ligand, or increasing the flow
rate, can reduce mass transport limitations. From previous experience of using low density
surfaces and similar MW antigens a flow rate of 40 μL/min was selected for this study.
Linked reactions control
The linked reaction control experiment is used to assess the ligand-analyte
interaction to check for deviations from a 1-to-1 binding model. The analyte is injected
over the surface for different periods of time (contact times) and the dissociation rate is
analyzed to determine if it varies with the contact time. If such a relationship is observed, it
indicates that a second interaction event is taking place after the initial binding event that
results in a stabilized complex at the surface.
Avidity associated with a single hC1q binding two antibodies could be expected
if the antibody was employed as the ligand. A linked reaction control was therefore
performed to provide supporting evidence for more complex data analysis models.

Kinetic analysis
A 1:1 binding model was initially assumed for all ligand/complex interactions
(see Equation 2). Due to ligand activity and drift in the baseline (BSA blocked surfaces)
the parameter Rmax was set to local as opposed to global analysis for selected kinetic
analyses. If necessary, additional models, such as Heterogeneous Ligand (see Equation 3)
and Bivalent Binding (see Equation 4), were also assessed for goodness of fit.
Equation 2: 1-to-1 binding:
ka
A + B ⇄ AB
kd
Equation 3: Heterogeneous ligand:
ka1                    ka2
A + B1 ⇄ AB1        A + B2 ⇄ AB2
kd1                    kd2
Equation 4: Bivalent (Avidity):
ka1                    ka2
2B + A ⇄ BA    +    B ⇄ BBA
kd1                    kd2
Antibody characterization
Both C1q complexes were immobilized based on concerns over avidity (i.e., two
immobilized antibodies binding C1q), and the very high levels of NSB observed using
mC1q and to a lesser extent hC1q to the CM5 surface (charge mediated estimated C1q: pI
8-9). Single cycle kinetics (SCK) were used initially to derive estimated kinetic constants
due to uncertainty over the stability of the C1q complex and the regeneration conditions.
Full kinetic analysis was performed after SCK. The ligand stability control injections
performed during the kinetic run indicated either the regeneration conditions used were
inactivating the ligand, or the ligand itself was unstable at 25°C during the 48 hours
required for kinetic analysis. The lower affinity displayed by mC1q for Fab allowed kinetic
analysis to be performed without surface regeneration.
Due to stability issues both Fab and IgG were used as ligands for kinetic
analysis, with the amount of ligand minimized to avoid potential avidity.
Kinetic data was obtained at a flow rate of 40 μL/min to minimize any potential
mass transfer effects. Two repeats of the blank (no antigen) and a single concentration of
the analyte were programmed into the kinetic run in order to check the stability of both the

surface and analyte over the kinetic cycles. For the initial kinetic runs 3.33-fold dilutions of
analyte were run. For kinetic analysis and on subsequent runs a 2-fold dilution range was
selected.
The association phase was monitored for 500 seconds to allow some of the
higher concentrations of analyte to reach steady state. In order to observe a sufficient signal
decrease (≥10 %) during the dissociation phase of the kinetic cycle, dissociation was
measured for up to 10,800 seconds to allow sufficient dissociation to occur for accurate
assessment of the kinetics. The signal from the reference channel Fc1 was subtracted from
that of Fc2, Fc3, and Fc4.
The kinetic parameters for the interaction of mC1q and the Fab fragment of
VH3/Vκ3 in various assay formats are shown in Table 5. The KD value for mC1q when
used as an analyte was 123 nM, and the KD value for mC1q when used as a ligand was 677
nM. The difference in the KD values reported could be due to the mode of interaction or the
effect of chemically coupling a multi-subunit mC1q to a surface, resulting in changes to the
secondary or tertiary structure of the protein.
TABLE 5: Kinetic analysis with mouse C1q
Ligand | Analyte | ka (1/Ms) | SE (ka) | kd (1/S) | SE (kd) | Rmax (RU) | SE (Rmax) | Chi2 (RU2) | KD (nM)
mC1q | Fab VH3/Vκ3 | 6.23x10^3 | 1.4X10^2 | 4.6x10^-3 | 1.7x10^-5 | 37.0 | 0.6 | 0.8 | 747.9
 | | 6.9x10^3 | 2.4X10^2 | 4.1x10^-3 | 1.0x10^-4 | 33.4 | 0.5 | 0.9 | 606.6
 | Mean | 6.5x10^3 | | 4.3x10^-3 | | 35.2 | | | 677.3
 | Std | 5.0x10^2 | | 3.1x10^-4 | | 2.6 | | | 99.9
Fab VH3/Vκ3 | mC1q | 4.5x10^5 | 2.6X10^4 | 5.6x10^-2 | 3.4x10^-3 | 88.6 | 0.6 | 1.9 | 125.6
 | | 1.7x10^5 | 1.8X10^3 | 9.1x10^-3 | 5.4x10^-5 | 50.0 | 0.3 | 5.6 | 121.2
 | Mean | 3.1x10^5 | | 3.2x10^-2 | | 69.3 | | | 123.4
 | Std | 2.0x10^5 | | 3.3x10^-2 | | 27.3 | | | 3.1

Table 5 depicts the kinetic parameters for the 1-to-1 interactions of mC1q and
Fab as determined using the Biacore T200. The Chi2 values show how well the association
and dissociation data fits the proposed binding model. The lower the value the better the fit.
The associated SE values for the rate constants represent the uncertainty associated with
fitting the data to the model described, and do not represent the total uncertainty for the true
kinetic values. The mean response data represents the average kinetic values and the
associated SD from 2 independent analyses.
The kinetic parameters for both the IgG and Fab interactions with hC1q were in
the low picomolar range (Table 6 for 1-to-1 model and Table 7 for the heterogeneous

model). In order to avoid avidity, hC1q was initially used as the ligand, however this
limited the analysis to single cycle kinetics and the use of more complex models, for
example, heterogeneous ligand binding models (**Equation 3**), that may represent the
different forms of hC1q structure that were associated with chemical coupling of a multi-
subunit protein to a surface. When hC1q was used as the analyte both IgG and Fab were
immobilized at the lowest concentrations possible to avoid avidity. The data was analyzed
using a 1-to-1 model and a more complex bivalent analyte model (**Equation 4**). Complex
model fitting did not significantly improve the fitting metrics and a linked reaction control
did not show a time-dependent dissociation phase. The results indicate that, at the lower
ligand density, the binding was predominately not due to avidity. Using hC1q as the
analyte, the KD value for the full-length VH3/Vκ3 IgG was 5.8 pM and the KD value for
the VH3/Vκ3 Fab was 8.6. It should be noted that the dissociation rate was too slow to
measure accurately with this technique. Longer dissociation times were limited by the
stability of the system (BSA blocking layer) and the low levels of binding used to avoid
avidity. These results correlate well with results obtained using hC1q as a ligand.

**TABLE 6: Kinetic analysis with human C1q**

Ligand | Analyte | ka (1/Ms) | SE (ka) | kd (1/S) | SE (kd) | Rmax (RU) | SE (Rmax) | Chi2 (RU2) | KD (pM)
hC1q | Fab VH3/Vκ3 | 5.2x10^4 | 82 | 4.6x10^-8 | 1.3x10^-7 | 154.9 | 0.033 | 14.8 | 0.87
 | IgG VH3/Vκ3 | 3.9x10^4 | 96 | 3.1x10^-7 | 5.3x10^-8 | 387.9 | 0.11 | 206 | 7.9
Fab VH3/Vκ3 | hC1q | 1.1x10^6 | 190 | 9.1x10^-6 | 1.4x10^-8 | 13.5 | 0.0015 | 0.0773 | 8.6
IgG VH3/Vκ3 | hC1q | 9.6x10^5 | 360 | 6.4x10^-6 | 2.9x10^-8 | 6.5 | 1.5x10^-3 | 0.076 | 6.7
 | | 1.1x10^6 | 190 | 5.1x10^-6 | 1.4x10^-8 | 17.4 | 0.002 | 0.143 | 4.9
 | Mean | 1.0x10^6 | | 5.8x10^-6 | | | | | 5.8
 | Std | 6.2x10^4 | | 9.1x10^-7 | | | | | 1.3

**[0336]** Table 6 depicts the kinetic parameters for the 1-to-1 interactions of hC1q with
Fab, or IgG as determined using the Biacore T200. Legend as Table 5. Data highlighted in
red indicate poor fitting criteria, these data sets have therefore been analyzed using a more
complex model (Table 7).

**TABLE 7: Kinetic analysis for heterogeneous ligand interaction**

Ligand | Analyte | ka1 (1/Ms) | kd1 (1/S) | ka2 (1/Ms) | kd2 (1/S) | Rmax1 (RU) | Rmax2 (RU) | Chi2 (RU2) | KD1 (nM) | KD2 (nM)
hC1q | Fab VH3/Vκ3 | 1.3x10^5 | 2.3x10^-5 | 1.8x10^4 | 6.3x10^-8 | 80.3 | 78.7 | 0.1 | 175.0 | 3.4
 | IgG | 2.0x10^5 | 4.6x10^-6 | 1.6x10^4 | 1.1x10^-7 | 152.6 | 247.1 | 2.41 | 22.7 | 7.3

Ligand | Analyte | ka1 (1/Ms) | kd1 (1/S) | ka2 (1/Ms) | kd2 (1/S) | Rmax1 (RU) | Rmax2 (RU) | Chi2 (RU2) | KD1 (nM) | KD2 (nM)
 | VH3/Vκ3 | | | | | | | | |

Table 7 depicts the kinetic parameters for the heterogeneous ligand interactions
of hC1q and Fab, or IgG as determined using the Biacore T200. Legend as Table 5. Data fit
to a heterogeneous ligand model to represent the expected heterogeneity of immobilizing a
multi-subunit protein on a surface.
Conclusion
The interaction of hC1q and mC1q with full-length IgG and the Fab fragment of
VH3/Vκ3 5was analyzed using both species as ligands. Issues with the stability and
chemical coupling of C1q complexes to the CM5 dextran surface required the development
of an IgG and Fab surface; the results indicate that the binding mode observed with this
surface was mainly 1-to-1, i.e., the kinetic profile did not show signs of avidity. Both the
full-length IgG and Fab fragment of VH3/Vκ3 displayed tight binding in the low picomolar
range for hC1q (5.8 and 8.6 pM, respectively), whereas a lower affinity was observed for
mC1q binding to the Fab fragment of VH3/Vκ3 (123 nM).
Example 3: Humanized Anti-C1q Antibodies Inhibit Complement-Mediated
Hemolysis
Humanized anti-C1q antibodies were tested in human and rodent hemolytic
assays (CH50) for their ability to neutralize C1q and block its activation of the downstream
complement cascade.
The humanized anti-C1q antibodies utilized in this example were produced as
described in Example 1. The following humanized antibodies from Example 1 were
utilized: the antibody VH1/Vκ1 (2B12), the antibody VH3/Vκ3 (5H7), the antibody
VH3/Vκ4 (3F1), and the antibody VH4/Vκ3 (1D3). The mouse monoclonal antibody M1
(ANN-005) and the chimeric M1antibody (3E2) were also utilized as controls.
CH50 assays were conducted essentially as described in Current Protocols in
Immunology (1994) Supplement 9 Unit 13.1. In brief, 5 microliters (μl) of human serum
(Cedarlane, Burlington, NC) or 0.625 μl of Wistar rat serum was diluted to 50 μl of GVB
buffer (Cedarlane, Burlington, NC) and added to 50 μl of the humanized antibodies (1 μg)
diluted in GVB buffer. The antibody:serum mixture was pre-incubated for 30 minutes on
ice and then added to 100 μl of EA cells (2x10^8/ml) for both rat and human assays. The EA

cells were generated exactly as specified in Current Protocols using Sheeps blood in
Alsever's (Cedarlane Cat #CL2581) and hemolysin (Cedalane Cat #CL9000). The EA
cells, serum and antibody mixture was incubated for 30 minutes at 37°C. and then placed on
ice. Next 1.2 ml of 0.15 M NaCl was added to the mixture and the OD412 of the sample was
read in a spectrophotometer to determine the amount of cell lysis. The percent inhibition of
the test antibodies was determined relative to a control mouse IgG1 antibody (Abcam
ab18447).
Four C1q-binding antibodies (2B12, 5H7, 3F1, and 1D3) were tested for their
C1q neutralizing activity in the human CH50 hemolysis assay in a dose-response format
(FIG. 7A). Each of the antibodies was tested at doses of 3.9 ng, 15.9 ng, 62.5 ng, and 260
ng, which correspond to an effective dosing range that results in the anti-C1q antibody
binding to C1q with a stoichiometry that ranges from approximately 10:1 to approximately
1:1. The murine anti-C1q antibody M1 (ANN-005) and the chimeric M1 antibody (3E2)
were used as references. The VH3/Vκ3 antibody (5H7) inhibited CH50 hemolysis in a
dose-dependent manner to degree that was comparable to both the murine M1 antibody and
the chimeric M1 antibody (FIG. 7A). Moreover, approximately 60 ng of the VH3/Vκ3
antibody (5H7), the VH4/Vκ3 antibody (1D3), and the VH1/Vκ1 antibody (2B12) was
required to inhibit 50% of the hemolysis observed (FIG. 7A). Approximately 250 ng of the
antibody VH3/Vκ4 (3F1) was required to inhibit approximately 95% of the hemolysis
observed (FIG. 7A).
Four C1q-binding antibodies (2B12, 5H7, 3F1, and 1D3) were also tested for
their C1q neutralizing activity in the rat CH50 assays (FIG. 7B). Each of the antibodies
was tested at doses of 3.9 ng, 15.9 ng, 62.5 ng, and 260 ng, which correspond to an
effective dosing range that results in the anti-C1q antibody binding to C1q with a
stoichiometry that ranges from approximately 10:1 to approximately 1:1. Testing was
conducted in dose-response formats. The murine anti-C1q antibody M1 (ANN-005) and
the chimeric M1 antibody (3E2) were used as references. The VH1/Vκ1 antibody (2B12)
inhibited CH50 hemolysis in a dose-dependent manner to degree that was comparable to
both the murine M1 antibody and the chimeric M1 antibody (FIG. 7B). Moreover,
approximately 60 ng of the VH1/Vκ1 antibody (2B12), the antibody VH3/Vκ4 (3F1), the
VH3/Vκ3 antibody (5H7), and the VH4/Vκ3 antibody (1D3) was required to inhibit
approximately 50% to approximately 80% of the hemolysis observed (FIG. 7B).

Although the foregoing invention has been described in some detail by way of
illustration and example for purposes of clarity of understanding, the descriptions and
examples should not be construed as limiting the scope of the invention. The disclosures of
all patent and scientific literature cited herein are expressly incorporated in their entirety by
reference.
Example 4: Intravenous dosing study in monkey to evaluate pharmacokinetics of
humanized anti-C1q antibody, pharmacodynamic effects on serum C1q levels and ex-
vivo complement mediated hemolysis
Cynomolgus monkeys were dosed with humanized anti-C1q antibody VH3/Vκ3
(5H7) via single intravenous bolus injection (I.V.) at 15 and 100 mg/Kg dose (N=2 per
dose, 1 male and 1 female monkey per dose).
Blood samples were collected at the following time points - Day 1: pre-study,
0.5, 2, 4, 8, 12, 24, 72, 96 and 120 hours post dose and on Days: 7, 9, 12, 15, 18 and 21.
Blood samples were allowed to clot, serum was separated by centrifugation and then stored
frozen at -80C until analysis.
Determination of serum levels of VH3/Vκ3 (5H7) from monkey samples: Serum
anti-C1q antibody levels were measured using a direct ELISA with hC1q used as the
capture analyte, followed by detection of human 5H7 antibody. Black 96 well ELISA plates
(Corning, Cat# 3925) were coated with human C1q (Complement Technology A099) at 2
μg/mL. After overnight incubation at 4C, plates were washed thrice with Dulbecco's
phosphate buffered saline (DPBS) (Thermo Scientific 28372) and blocked overnight at 4°C
with DPBS containing 3% BSA. Next day, blocking solution was removed and 5H7
standards or individual serum samples at a range of dilutions (2000 to 2000000-fold) were
added to the plates at 50 μL per sample in assay buffer, DPBS containing 0.3% BSA and
0.1% tween (KPL Inc. 51-12-10). Samples were incubated at room temperature, shaking at
300 rpm for 1 hr. Then, 50 μL of goat anti-human FC antibody conjugated with alkaline
phosphatase (Jackson Immuno research, 109-055-098) was added at a concentration of 0.5
μg/mL in assay buffer. After incubation for 1 hr at room temperature, plates were washed
three times in DPBS containing 0.05% tween. Each wash was for a duration of 10 minutes
with shaking at 300 rpm on a plate shaker. Plates were then tapped dry and developed using
alkaline phosphatase substrate incubation for 20 minutes (Life Technologies, #T2214).
Luminescence counts were read on a Perkin Elmer envision reader. Standard curves were

fit using 4PL logistic fit and unknown signal counts were converted μg/mL concentration
and plotted using Graphpad Prism.
Determination of serum C1q levels from monkey samples: Serum levels of C1q
were determined using two distinct hC1q specific ELISA assays. In both ELISA assays,
JL1, an antibody that binds to the collagen tail of C1q was used as the capture antibody
(Abcam ab71940). In the first assay, the murine version of VH3/Vκ3 (5H7) or M1, which
binds to the same site as 5H7, was used as detection antibody to isolate Free C1q levels. In
the second assay, JL1 was used as the detection antibody to measure C1q which is both free
and bound to ANX in the serum samples.
Black 96 well ELISA plates (Corning, Cat# 3925) were coated with JL1 at 1
μg/mL. After overnight incubation at 4C, plates were washed thrice with Dulbecco's
phosphate buffered saline (DPBS) (Thermo Scientific 28372) and blocked overnight with
DPBS containing 3% BSA at 4°C. Next day, blocking solution was removed and C1q
standards or individual serum samples were run at dilutions in the range of 1000x to
10000x, in assay buffer DPBS with 0.3% BSA and 0.1% tween, at 50 μL per sample.
Following incubation at room temperature for 1 hr, 50 uL of respective alkaline
phosphatase conjugated antibodies M1 or JL1 were added, at a final concentration of 200-
400 ng/mL in assay buffer. Samples were incubated overnight with shaking at 4C. Next
day, plates were washed three times in DPBS containing 0.05% tween. Each wash was for a
duration of 10 minutes with shaking at 300 rpm on a plate shaker. Plates were then tapped
dry and developed using alkaline phosphatase substrate incubation for 20 minutes.
Luminescence counts were read on a Perkin Elmer envision reader. Standard curves were
fit using 4PL logistic fit and unknown signal counts were converted to concentration,
dilution correction and then plotted using Graphpad Prism.
Determination of ex-vivo hemolysis activity in monkey serum samples: The
hemolysis assays were similar to that in example 3 with the following modification.
Monkey serum samples from the study were diluted 1:50 in GVB ++ buffer solution
(Complement Technology Cat# B100) and mixed with an equal volume of antibody
sensitized sheep red blood cells at 17 million cells/mL (Complement Technology Cat#
B201). Samples were incubated for 1 hr at 37C. Control wells were set up to determine
baseline (buffer only without any serum) and 100% hemolysis (using deionized water).
Samples were then spun down and supernatants were transferred to clear ELISA plates and
absorbance read at 415 nm. The absorbance for all samples were baseline subtracted and

normalized to 100% hemolysis (deionized water). At the 1:50 dilution, serum samples
showed 50-70% of hemolysis observed with water. % Hemolysis was plotted for each
individual monkey following baseline normalization.
A dose dependent increase in serum 5H7 levels was observed following IV
dosing with a maximal exposure of ~250 ug/mL at the 15 mg/Kg dose and ~ 2000 ug/mL at
the 100 mg/Kg dose. Sustained serum levels of 5H7 were evident over the 20 days of
sampling at the 100 mg/Kg dose, while serum 5H7 levels declined to levels below limit of
detection after 4 days at the 15 mg/Kg dose (FIG. 8). Serum C1q levels (JL1-M1 assay)
were reduced > 90% over 5 days at the 15 mg/Kg dose and recovered back to baseline
between 5-11 days after onset of dosing (FIG. 9A). In contrast, the 100 mg/Kg dose led to
a sustained reduction of serum C1q levels up to 20 days after onset of dosing (FIG. 9A). A
similar pattern of reduction and time course of serum C1q was observed with the JL1-JL1
assay (FIG. 9B). The observation of robust and sustained reduction in serum C1q in 2
independent ELISA assays, one with a detection antibody that binds to the same site on
C1q as 5H7 and the other with detection antibody against an independent site on C1q,
suggests that serum C1q levels are cleared following treatment with 5H7. Consistent with
the reduction in serum C1q levels, a sustained reduction of ex-vivo hemolysis was observed
at the 100 mg/Kg dose up to 20 days after onset of dosing (FIG. 10). At the 15 mg/Kg dose
of ANX, hemolysis was reduced > 90% over 5 days and recovered back to baseline
between 5-11 days after onset of dosing (FIG. 10).
These results demonstrate that the anti-C1q antibody VH3/Vκ3 (5H7) shows a robust
pharmacokinetic exposure and time course along with sustained reduction of serum C1q
levels and hemolysis in cynomolgus monkeys.

The present invention furthermore relates to the following items:
1.	A humanized antibody that specifically binds to a C1q protein, wherein the antibody
	comprises a heavy chain variable region and a human heavy chain constant region,
	wherein the heavy chain variable region comprises an Fab region and the heavy
	chain constant region comprises an Fc region, wherein the Fab region specifically
	binds to the C1q protein, and wherein the Fc region is incapable of binding the C1q
	protein.
2.	The antibody of item 1, wherein the Fc region is incapable of inducing
	complement activity.
3.	The antibody of item 1 or item 2, wherein the Fc region is incapable of inducing
	antibody-dependent cellular cytotoxicity (ADCC).
4.	The antibody of any one of items 1-3, wherein the human heavy chain constant
	region is a human IgG4 heavy chain constant region.
5.	The antibody of item 4, wherein the human IgG4 heavy chain constant region
	comprises the amino acid sequence of SEQ ID NO: 37, or an amino acid sequence
	with at least about 90% homology to the amino acid sequence of SEQ ID NO: 37.
6.	The antibody of item 4, wherein the human IgG4 heavy chain constant region
	comprises an Fc region, and wherein the Fc region comprises one or more
	modifications.
7.	The antibody of item 6, wherein the Fc region comprises one or more amino acid
	substitutions.
8.	The antibody of item 7, wherein the Fc region comprises an amino acid
	substitution at position 248 according to Kabat numbering convention.
9.	The antibody of item 8, wherein the Fc region comprises a leucine to glutamate
	amino acid substitution at position 248 according to Kabat numbering convention.
10.	The antibody of item 8 or item 9, wherein the amino acid substitution at position
	248 according to Kabat numbering convention inhibits the Fc region from
	interacting with an Fc receptor.
11.	The antibody of any one of items 7-10, wherein the Fc region comprises an amino
	acid substitution at position 241 according to Kabat numbering convention.
12.	The antibody of item 11, wherein the Fc region comprises a serine to proline amino
	acid substitution at position 241 according to Kabat numbering convention.

13.	The antibody of item 11 or item 12, wherein the amino acid substitution at
	position 241 according to Kabat numbering convention prevents arm switching in
	the antibody.
14.	The antibody of any one of items 1-13, wherein the antibody comprises a heavy
	chain variable domain and a light chain variable domain, wherein the heavy chain
	variable domain comprises an amino acid sequence selected from SEQ ID NOs: 1-4,
	or an amino acid sequence with at least about 95% homology to the amino acid
	sequence selected from SEQ ID NOs: 1-4.
15.	The antibody of any one of items 1-14, wherein the light chain variable domain
	comprises an amino acid sequence selected from SEQ ID NOs: 5-8, or an amino
	acid sequence with at least about 90% homology to the amino acid sequence selected
	from SEQ ID NOs: 5-8.
16.	A humanized anti-C1q antibody, or an antigen-binding fragment thereof, the
	antibody comprising a heavy chain variable domain and a light chain variable
	domain, wherein the heavy chain variable domain comprises an amino acid
	sequence selected from SEQ ID NOs: 1-4, or an amino acid sequence with at least
	about 90% homology to the amino acid sequence selected from SEQ ID NOs: 1-4.
17.	A humanized anti-C1q antibody, or an antigen-binding fragment thereof, the
	antibody comprising a heavy chain variable domain and a light chain variable
	domain, wherein the light chain variable domain comprises an amino acid sequence
	selected from SEQ ID NOs: 5-8, or an amino acid sequence with at least about 90%
	homology to the amino acid sequence selected from SEQ ID NOs: 5-8.
18.	A humanized anti-C1q antibody, or an antigen-binding fragment thereof, the
	antibody comprising:
		a) a heavy chain variable domain comprising an amino acid sequence selected
		from SEQ ID NOs: 1-4, or an amino acid sequence with at least about 90%
		homology to the amino acid sequence selected from SEQ ID NOs: 1-4;
		and/or
		b) a light chain variable domain comprising an amino acid sequence selected
		from SEQ ID NOs: 5-8, or an amino acid sequence with at least about 90%
		homology to the amino acid sequence selected from SEQ ID NOs: 5-8.

19.	A humanized anti-C1q antibody, or an antigen-binding fragment thereof, the
antibody comprising:
a) a heavy chain variable domain comprising the amino acid sequence of SEQ
ID NO: 1, or an amino acid sequence with at least about 90% homology to
the amino acid sequence of SEQ ID NO: 1; and
b) a light chain variable domain comprising an amino acid sequence selected
from SEQ ID NOs: 5-8, or an amino acid sequence with at least about 90%
homology to the amino acid sequence selected from SEQ ID NOs: 5-8.
20.	A humanized anti-C1q antibody, or an antigen-binding fragment thereof, the
antibody comprising:
a) a heavy chain variable domain comprising the amino acid sequence of SEQ
ID NO: 2, or an amino acid sequence with at least about 90% homology to
the amino acid sequence of SEQ ID NO: 2; and
b) a light chain variable domain comprising an amino acid sequence selected
from SEQ ID NOs: 5-8, or an amino acid sequence with at least about 90%
homology to the amino acid sequence selected from SEQ ID NOs: 5-8.
21.	A humanized anti-C1q antibody, or an antigen-binding fragment thereof, the
antibody comprising:
a) a heavy chain variable domain comprising the amino acid sequence of SEQ
ID NO: 3, or an amino acid sequence with at least about 90% homology to
the amino acid sequence of SEQ ID NO: 3; and
b) a light chain variable domain comprising an amino acid sequence selected
from SEQ ID NOs: 5-8, or an amino acid sequence with at least about 90%
homology to the amino acid sequence selected from SEQ ID NOs: 5-8.
22.	A humanized anti-C1q antibody, or an antigen-binding fragment thereof, the
antibody comprising:
a) a heavy chain variable domain comprising the amino acid sequence of SEQ
ID NO: 4, or an amino acid sequence with at least about 90% homology to
the amino acid sequence of SEQ ID NO: 4; and
b) a light chain variable domain comprising an amino acid sequence selected
from SEQ ID NOs: 5-8, or an amino acid sequence with at least about 90%
homology to the amino acid sequence selected from SEQ ID NOs: 5-8.

23.	A humanized anti-C1q antibody, or an antigen-binding fragment thereof, the
antibody comprising:
a) a heavy chain variable domain comprising an amino acid sequence selected
from SEQ ID NOs: 1-4, or an amino acid sequence with at least about 90%
homology to the amino acid sequence selected from SEQ ID NOs: 1-4; and
b) a light chain variable domain comprising the amino acid sequence of SEQ
ID NO: 5, or an amino acid sequence with at least about 90% homology to
the amino acid sequence of SEQ ID NO: 5.
24.	A humanized anti-C1q antibody, or an antigen-binding fragment thereof, the
antibody comprising:
a) a heavy chain variable domain comprising an amino acid sequence selected
from SEQ ID NOs: 1-4, or an amino acid sequence with at least about 90%
homology to the amino acid sequence selected from SEQ ID NOs: 1-4; and
b) a light chain variable domain comprising the amino acid sequence of SEQ
ID NO: 6, or an amino acid sequence with at least about 90% homology to
the amino acid sequence of SEQ ID NO: 6.
25.	A humanized anti-C1q antibody, or an antigen-binding fragment thereof, the
antibody comprising:
a) a heavy chain variable domain comprising an amino acid sequence selected
from SEQ ID NOs: 1-4, or an amino acid sequence with at least about 90%
homology to the amino acid sequence selected from SEQ ID NOs: 1-4; and
b) a light chain variable domain comprising the amino acid sequence of SEQ
ID NO: 7, or an amino acid sequence with at least about 90% homology to
the amino acid sequence of SEQ ID NO: 7.
26.	A humanized anti-C1q antibody, or an antigen-binding fragment thereof, the
antibody comprising:
a) a heavy chain variable domain comprising an amino acid sequence selected
from SEQ ID NOs: 1-4, or an amino acid sequence with at least about 90%
homology to the amino acid sequence selected from SEQ ID NOs: 1-4; and
b) a light chain variable domain comprising the amino acid sequence of SEQ
ID NO: 8, or an amino acid sequence with at least about 90% homology to
the amino acid sequence of SEQ ID NO: 8.

27.	The antibody or antigen-binding fragment of any one of items 1-26, wherein the
	antibody binds specifically to both human C1q and mouse C1q.
28.	The antibody or antigen-binding fragment of any one of items 1-26, wherein the
	antibody binds specifically to rat C1q.
29.	The antibody or antigen-binding fragment of any one of items 1-26, wherein the
	antibody binds specifically to human C1q, mouse C1q, and rat C1q.
30.	The antibody or antigen-binding fragment of any one of items 1-26, wherein the
	antibody or antigen-binding fragment thereof binds essentially the same C1q
	epitope as the antibody M1 produced by the hybridoma cell line with ATCC
	Accession Number PTA-120399 or anti-C1q binding fragments thereof.
31.	The antibody or antigen-binding fragment of any one of items 1-26, wherein the
	antibody or antigen-binding fragment thereof inhibits the binding of the monoclonal
	antibody M1 produced by a hybridoma cell line with ATCC Accession Number
	PTA-120399 to human C1q or to mouse C1q.
32.	The antibody or antigen-binding fragment of any one of items 1-31, wherein the
	antibody is of the IgG class.
33.	The antibody or antigen-binding fragment of item 32, wherein the antibody has an
	IgG1, IgG2, IgG3, or IgG4 isotype.
34.	The antibody or antigen-binding fragment of item 32, wherein the antibody has an
	IgG4 isotype.
35.	The antibody or antigen-binding fragment of item 34, wherein the antibody
	comprises a human IgG4 constant region.
36.	The antibody or antigen-binding fragment of item 35, wherein the human IgG4
	heavy chain constant region comprises the amino acid sequence of SEQ ID NO: 37,
	or an amino acid sequence with at least about 90% homology to the amino acid
	sequence of SEQ ID NO: 37.
37.	The antibody or antigen-binding fragment of item 35, wherein the human IgG4
	constant region comprises an Fc region.
38.	The antibody or antigen-binding fragment of item 37, wherein the Fc region is
	incapable of binding the C1q protein.
39.	The antibody or antigen-binding fragment of item 37 or item 38, wherein the Fc
	region is incapable of inducing complement activity.

40.	The antibody or antigen-binding fragment of any one of items 37-39, wherein the
	Fc region is incapable of inducing antibody-dependent cellular cytotoxicity (ADCC).
41.	The antibody or antigen-binding fragment of any one of items 37-40, wherein the
	Fc region comprises one or more modifications.
42.	The antibody or antigen-binding fragment of item 41, wherein the Fc region
	comprises one or more amino acid substitutions.
43.	The antibody or antigen-binding fragment of item 42, wherein the Fc region
	comprises an amino acid substitution at position 248 according to Kabat numbering
	convention.
44.	The antibody or antigen-binding fragment of item 43, wherein the Fc region
	comprises a leucine to glutamate amino acid substitution at position 248 according
	to Kabat numbering convention.
45.	The antibody or antigen-binding fragment of item 43 or item 44, wherein the
	amino acid substitution at position 248 according to Kabat numbering convention
	inhibits the Fc region from interacting with an Fc receptor.
46.	The antibody or antigen-binding fragment of any one of items 42-45, wherein the
	Fc region comprises an amino acid substitution at position 241 according to Kabat
	numbering convention.
47.	The antibody or antigen-binding fragment of item 46, wherein the Fc region
	comprises a serine to proline amino acid substitution at position 241 according to
	Kabat numbering convention.
48.	The antibody or antigen-binding fragment of item 46 or item 47, wherein the
	amino acid substitution at position 241 according to Kabat numbering convention
	prevents arm switching in the antibody.
49.	The antibody or antigen-binding fragment of any one of items 1-34, wherein the
	antibody is a bispecific antibody.
50.	The antibody or antigen-binding fragment of any one of items 1-34, wherein the
	antibody has been engineered to increase brain penetration.
51.	The antibody or antigen-binding fragment of any one of items 1-34, wherein the
	antibody is a bispecific antibody recognizing a first antigen and a second antigen.
52.	The antibody of item 51, wherein the first antigen is a C1q protein and the second
	antigen is an antigen facilitating transport across the blood-brain-barrier.

53. The antibody of item 51 or item 52, wherein the second antigen is selected from
transferrin receptor (TR), insulin receptor (HIR), insulin-like growth factor receptor
(IGFR), low-density lipoprotein receptor related proteins 1 and 2 (LPR-1 and 2),
diphtheria toxin receptor, CRM197, a llama single domain antibody, TMEM 30(A),
a protein transduction domain, TAT, Syn-B, penetratin, a poly-arginine peptide, an
angiopep peptide, and ANG1005.
54. The antibody or antigen-binding fragment of any one of items 1-53, wherein the
antigen-binding fragment is a Fab, F(ab')2 or Fab' fragment.
55. The antibody or antigen-binding fragment of item 54, wherein the antibody
fragment has better brain penetration as compared to its corresponding full-length
antibody.
56. The antibody or antigen-binding fragment of item 54 or item 55, wherein the
antibody fragment has a shorter half-life as compared to its corresponding full-
length antibody.
57. The antibody or antigen-binding fragment of any one of items 1-56, wherein the
antibody has a dissociation constant (KD) for human C1q that ranges from less than
about 10 pM to less than about 5 pM.
58. The antibody or antigen-binding fragment of any one of items 1-57, wherein the
antibody has dissociation constant (KD) for mouse C1q that ranges from less than
about 125 nM to less than about 5 pM.
59. The antibody or antigen-binding fragment of any one of items 1-58, wherein the
antibody specifically binds to and neutralizes a biological activity of C1q.
60. The antibody of item 59, wherein the biological activity is (1) C1q binding to an
autoantibody, (2) C1q binding to C1r, (3) C1q binding to C1s, (4) C1q binding to
phosphatidylserine, (5) C1q binding to pentraxin-3, (6) C1q binding to C-reactive
protein (CRP), (7) C1q binding to globular C1q receptor (gC1qR), (8) C1q binding
to complement receptor 1 (CR1), (9) C1q binding to beta-amyloid, or (10) C1q
binding to calreticulin.
61. The antibody of item 59 or item 60, wherein the biological activity is (1)
activation of the classical complement activation pathway, (2) activation of
antibody and complement dependent cytotoxicity, (3) CH50 hemolysis, (4) synapse
loss, (5) B-cell antibody production, (6) dendritic cell maturation, (7) T-cell
proliferation, (8) cytokine production (9) microglia activation, (10) Arthus reaction,

(11) phagocytosis of synapses or nerve endings, or (12) activation of complement
receptor 3 (CR3/C3) expressing cells.
62. The antibody of item 61, wherein CH50 hemolysis comprises human, mouse,
and/or rat CH50 hemolysis.
63. The antibody of item 61 or item 62, wherein the antibody is capable of
neutralizing from at least about 50%, to at least about 90% of CH50 hemolysis.
64. The antibody of any one of items 61-63, wherein the antibody is capable of
neutralizing at least 50% of CH50 hemolysis at a dose of less than 150 ng, less than
100 ng, less than 50 ng, or less than 20 ng.
65. The antibody of any one of items 61-64, wherein the antibody binds C1q with a
binding stoichiometry that ranges from 20:1 to 1.0:1 or less than 1.0:1.
66. An isolated polynucleotide comprising a nucleic acid sequence encoding the
antibody of any one of items 1-65.
67. An isolated host cell comprising the nucleic acid sequence of item 66.
68. A pharmaceutical composition comprising the antibody of any one of items 1-65
and a pharmaceutically acceptable carrier.
69. A method of treating or preventing a disease associated with complement activation
in an individual in need of such treatment, the method comprising the step of
administering a therapeutically effective dose of the antibody of any one of items
1-65.
70. The method of item 69, wherein the disease associated with complement activation
is a neurodegenerative disorder.
71. The method of item 70, wherein the neurodegenerative disorder is associated with
loss of synapses or loss nerve connections.
72. The method of item 70 or item 71, wherein the neurodegenerative disorder is
associated with synapse loss that is dependent on the complement receptor
3(CR3)/C3 or complement receptor CR1.
73. The method of any one of items 70-72, wherein the neurodegenerative disorder is
associated with pathological activity-dependent synaptic pruning.
74. The method of any one of items 70-73, wherein the neurodegenerative disorder is
associated with synapse phagocytosis by microglia.
75. The method of any one of items 70-74, wherein the neurodegenerative disorder is
selected from Alzheimer's disease, amyotrophic lateral sclerosis, multiple sclerosis,

glaucoma, myotonic dystrophy, Guillain-Barre´ syndrome (GBS), Myastenia
Gravis, Bullous Pemphigoid, spinal muscular atrophy, Down syndrome, Parkinson's
disease, and Huntington's disease.
76. The method of item 69, wherein the disease associated with complement activation
is an inflammatory disease, autoimmune disease, or metabolic disorder.
77. The method of item 76, wherein the inflammatory disease, autoimmune disease, or
metabolic disorder is selected from diabetes, obesity, rheumatoid arthritis (RA),
acute respiratory distress syndrome (ARDS), remote tissue injury after ischemia and
reperfusion, complement activation during cardiopulmonary bypass surgery,
dermatomyositis, pemphigus, lupus nephritis and resultant glomerulonephritis and
vasculitis, cardiopulmonary bypass, cardioplegia-induced coronary endothelial
dysfunction, type II membranoproliferative glomerulonephritis, IgA nephropathy,
acute renal failure, cryoglobulemia, antiphospholipid syndrome, Chronic open-angle
glaucoma, acute closed angle glaucoma, macular degenerative diseases, age-related
macular degeneration (AMD), (AMD-wet), Geographic atrophy choroidal
neovascularization (CNV), uveitis, diabetic retinopathy, ischemia-related
retinopathy, endophthalmitis, intraocular neovascular disease, diabetic macular
edema, pathological myopia, von Hippel-Lindau disease, histoplasmosis of the eye,
Neuromyelitis Optica (NMO), Central Retinal Vein Occlusion (CRVO), corneal
neovascularization, retinal neovascularization, Leber's hereditary optic neuropathy,
optic neuritis, Behcet's retinopathy, ischemic optic neuropathy, retinal vasculitis,
ANCA vasculitis, Purtscher retinopathy, Sjogren's dry eye disease, dry AMD,
sarcoidosis, temporal arteritis, polyarteritis nodosa, multiple sclerosis, allo-
transplantation, hyperacute rejection, hemodialysis, chronic occlusive pulmonary
distress syndrome (COPD), asthma, and aspiration pneumonia.
78. The method of item 69, wherein the disease associated with complement activation
is an autoimmune disease selected from myasthenia gravis, Diabetes mellitus type 1,
Hashimoto's thyroiditis, Addison's disease, Coeliac disease, Crohn's disease,
pernicious anaemia, Pemphigus vulgaris, vitiligo, autoimmune hemolytic anemias,
paraneoplastic syndromes, a vasculitis disease, hypocomplementemic urticarial
vasculitis (HUV), polymyalgia rheumatica, temporal arteritis, and Wegener's
granulomatosis.

79. A kit comprising an antibody of any one of items 1-65, and a package insert
comprising instructions for using the antibody to treat or prevent a disease
associated with complement activation in an individual in need of such treatment.
80. The kit of item 79, wherein the disease associated with complement activation is a
neurodegenerative disorder.
81. The kit of item 80, wherein the neurodegenerative disorder is associated with loss
of synapses or loss nerve connections.
82. The kit of item 80 or 81, wherein the neurodegenerative disorder is associated with
synapse loss that is dependent on the complement receptor 3(CR3)/C3 or
complement receptor CR1.
83. The kit of any one of items 80-82, wherein the neurodegenerative disorder is
associated with pathological activity-dependent synaptic pruning.
84. The kit of any one of items 80-83, wherein the neurodegenerative disorder is
associated with synapse phagocytosis by microglia.
85. The kit of any one of items 80-84, wherein the neurodegenerative disorder is
selected from Alzheimer's disease, amyotrophic lateral sclerosis, multiple sclerosis,
glaucoma, myotonic dystrophy, Down syndrome, Parkinson's disease, and
Huntington's disease.
86. The kit of item 79, wherein the disease associated with complement activation is
an inflammatory disease, autoimmune disease, or metabolic disorder.
87. The kit of item 86, wherein the inflammatory disease, autoimmune disease, or
metabolic disorder is selected from diabetes, obesity, rheumatoid arthritis (RA),
acute respiratory distress syndrome (ARDS), remote tissue injury after ischemia and
reperfusion, complement activation during cardiopulmonary bypass surgery,
dermatomyositis, pemphigus, lupus nephritis and resultant glomerulonephritis and
vasculitis, cardiopulmonary bypass, cardioplegia-induced coronary endothelial
dysfunction, type II membranoproliferative glomerulonephritis, IgA nephropathy,
acute renal failure, cryoglobulemia, antiphospholipid syndrome, Chronic open-angle
glaucoma, acute closed angle glaucoma, macular degenerative diseases, age-related
macular degeneration (AMD), (AMD-wet), Geographic atrophy choroidal
neovascularization (CNV), uveitis, diabetic retinopathy, ischemia-related
retinopathy, endophthalmitis, intraocular neovascular disease, diabetic macular
edema, pathological myopia, von Hippel-Lindau disease, histoplasmosis of the eye,

Neuromyelitis Optica (NMO), Central Retinal Vein Occlusion (CRVO), corneal
neovascularization, retinal neovascularization, Leber's hereditary optic neuropathy,
optic neuritis, Behcet's retinopathy, ischemic optic neuropathy, retinal vasculitis,
ANCA vasculitis, Purtscher retinopathy, Sjogren's dry eye disease, dry AMD,
sarcoidosis, temporal arteritis, polyarteritis nodosa, multiple sclerosis, allo-
transplantation, hyperacute rejection, hemodialysis, chronic occlusive pulmonary
distress syndrome (COPD), asthma, and aspiration pneumonia.
88. The kit of item 79, wherein the disease associated with complement activation is
an autoimmune disease selected from myasthenia gravis, Diabetes mellitus type 1,
Hashimoto's thyroiditis, Addison's disease, Coeliac disease, Crohn's disease,
pernicious anaemia, Pemphigus vulgaris, vitiligo, autoimmune hemolytic anemias,
paraneoplastic syndromes, a vasculitis disease, hypocomplementemic urticarial
vasculitis (HUV), polymyalgia rheumatica, temporal arteritis, and Wegener's
granulomatosis.
89. A diagnostic kit comprising an antibody of any one of items 1-65.
90. A method of detecting synapses in an individual, the method comprising
a) administering an antibody of any one of items 1-65 to the individual; and
b) detecting antibody bound to synapses, thereby detecting synapses in the
individual.
91. The method of item 90, wherein the antibody bound to synapses is detected using
imaging techniques selected from positron emission tomography (PET), X-ray
computed tomography, single-photon emission computed tomography (SPECT),
computed tomography (CT), and computed axial tomography (CAT).
92. The method of item 90 or item 91, wherein the detection of antibody bound to
synapses provides a quantitative measure of the number of synapses in the
individual.
93. The method of any one of items 90-92, wherein the individual has a
neurodegenerative disease or autoimmune disease.
94. The method of any one of items 90-93, wherein the number of synapses in the
individual is measured repeatedly over a period of time and a loss of synapses in the
individual is detected over time.
95. The method of item 94, wherein the loss of synapses over time is a measure for the
efficacy of a treatment for the neurodegenerative disease or autoimmune disease.

96. A method of detecting synapses in a biological sample, the method comprising
a) contacting the biological sample with an antibody of any one of items 1-65;
and
b) detecting antibody bound to synapses, thereby detecting synapses in the
biological sample.
97. The method of item 96, further comprising a step before step a) of obtaining the
biological sample from an individual.
98. The method of item 96 or item 97, wherein the biological sample comprises a
biopsy specimen, a tissue, or a cell.
99. The method of any one of items 96-98, wherein the antibody is detected by
immunofluorescence microscopy, immunocytochemistry, immunohistochemistry,
ELISA, FACS analysis, immunoprecipitation, or micro-positron emission
tomography.

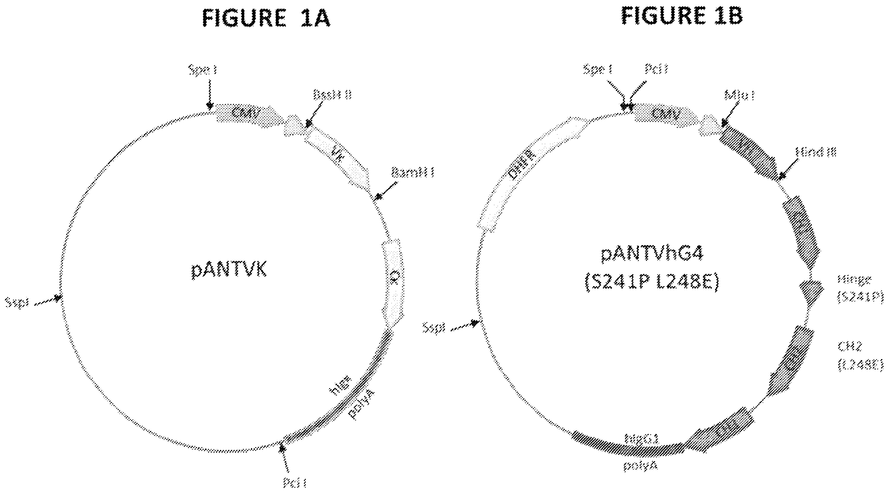

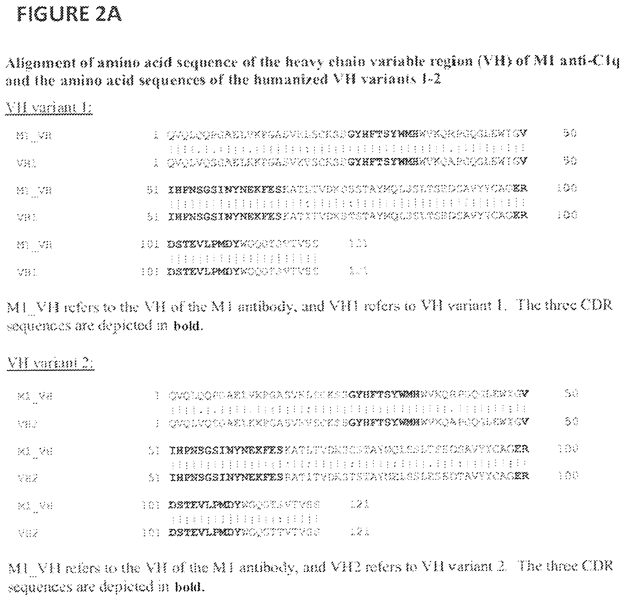

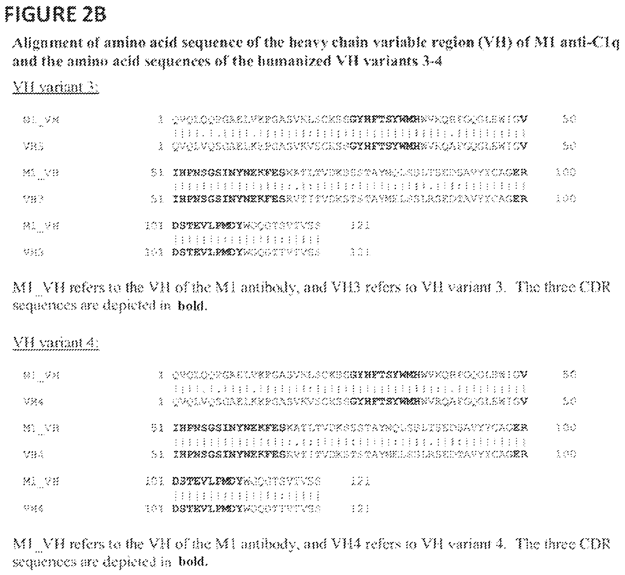

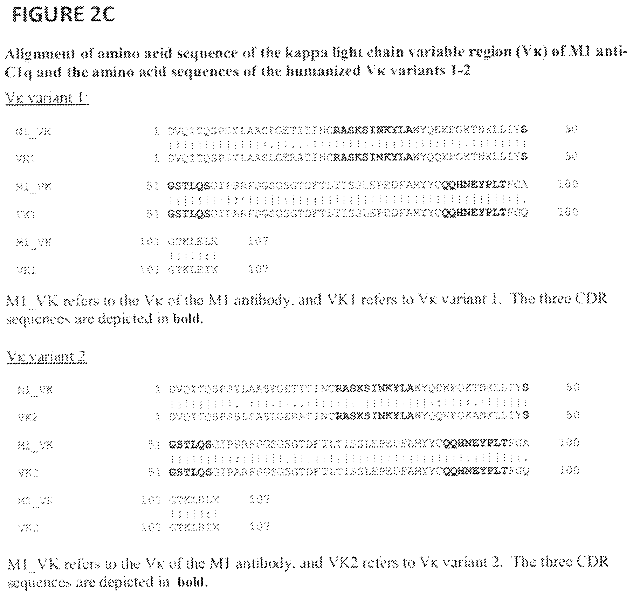

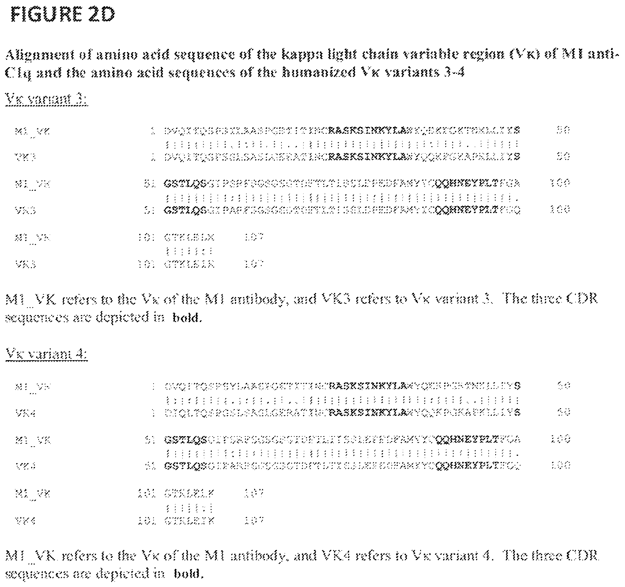

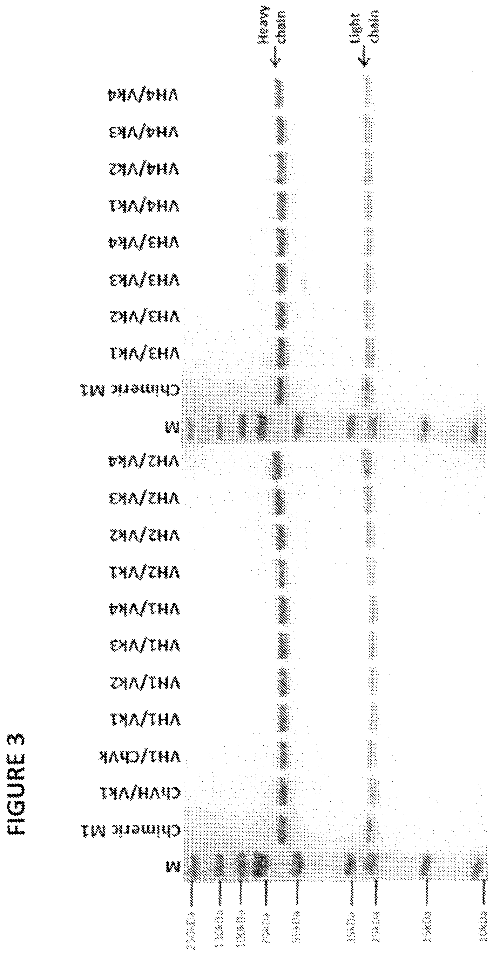

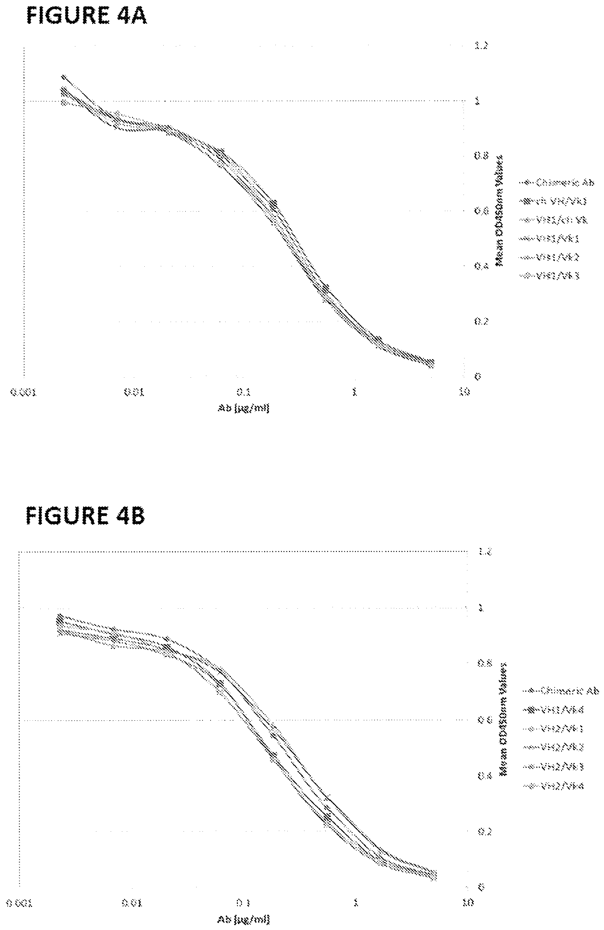

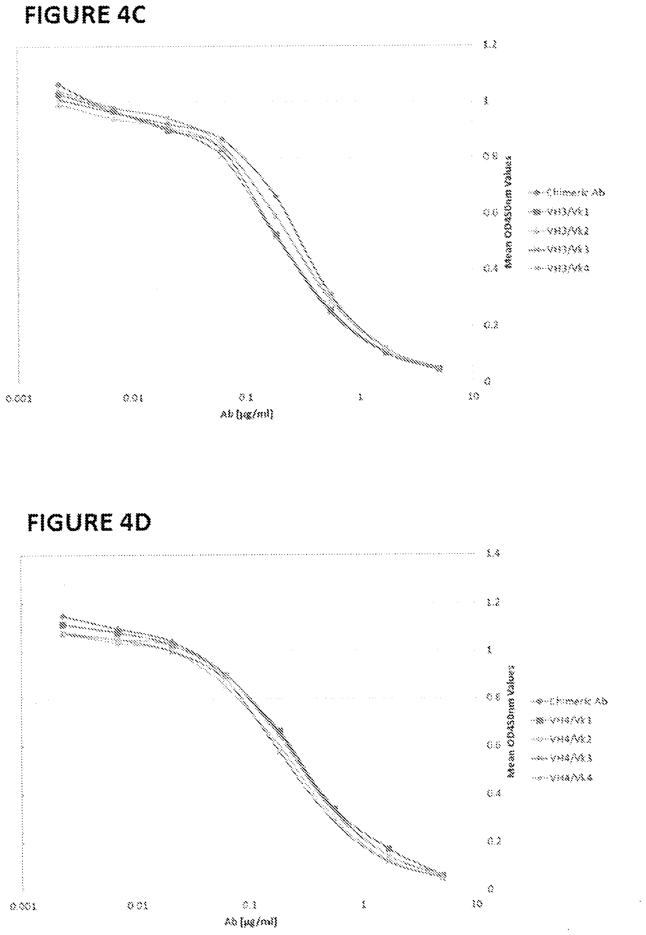

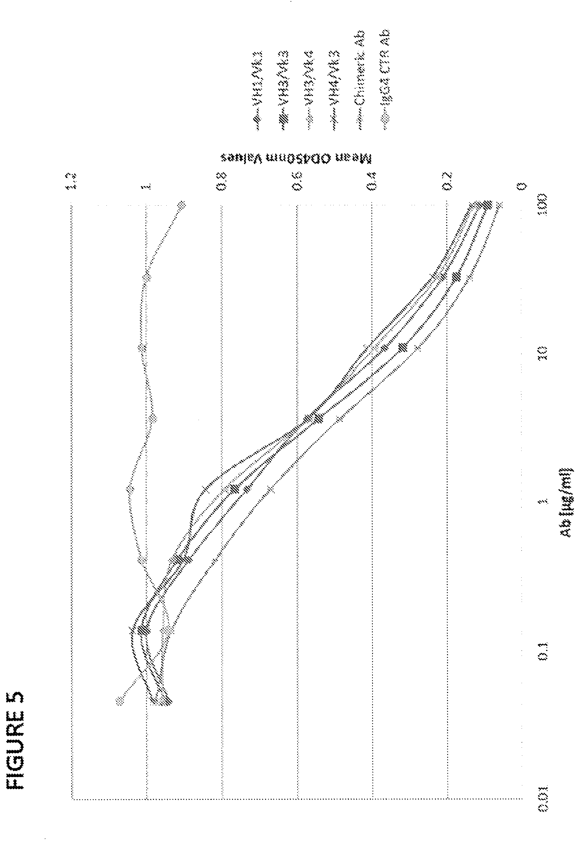

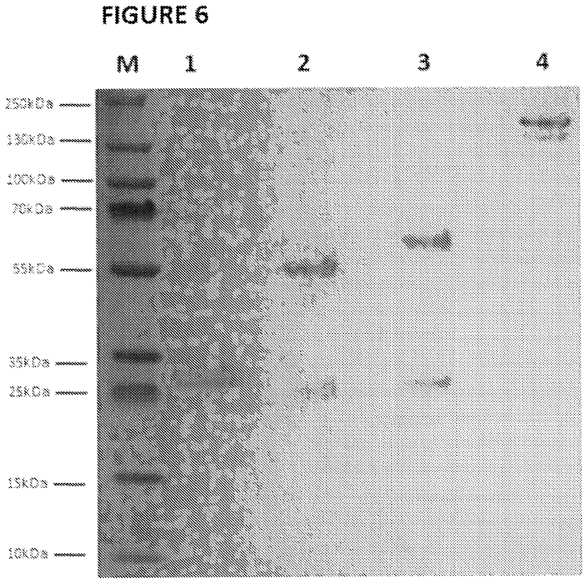

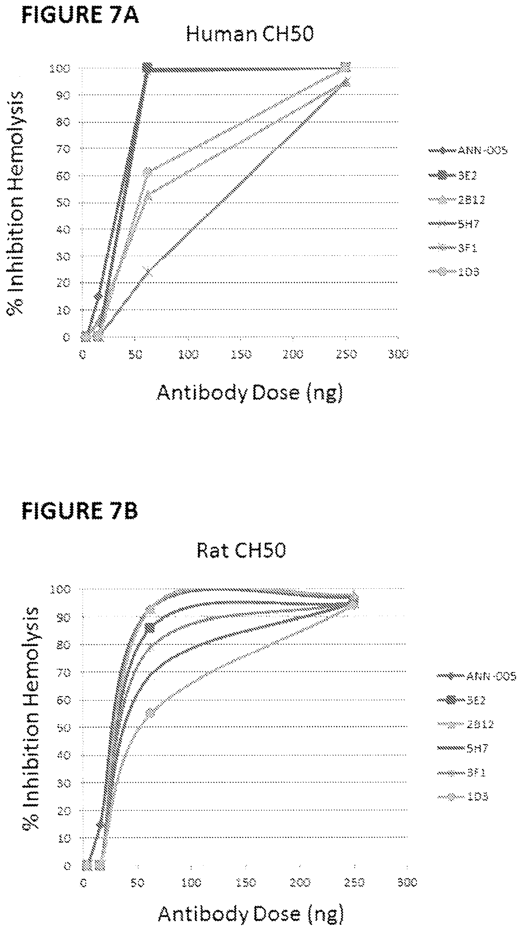

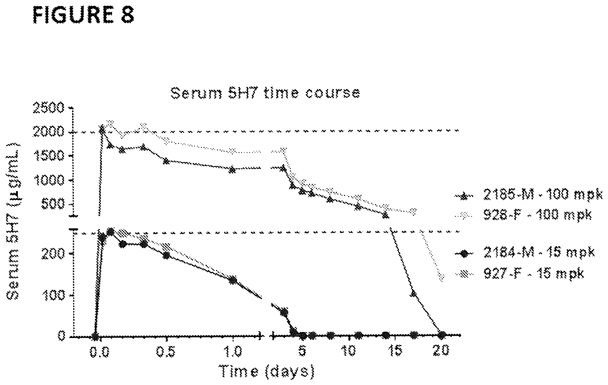

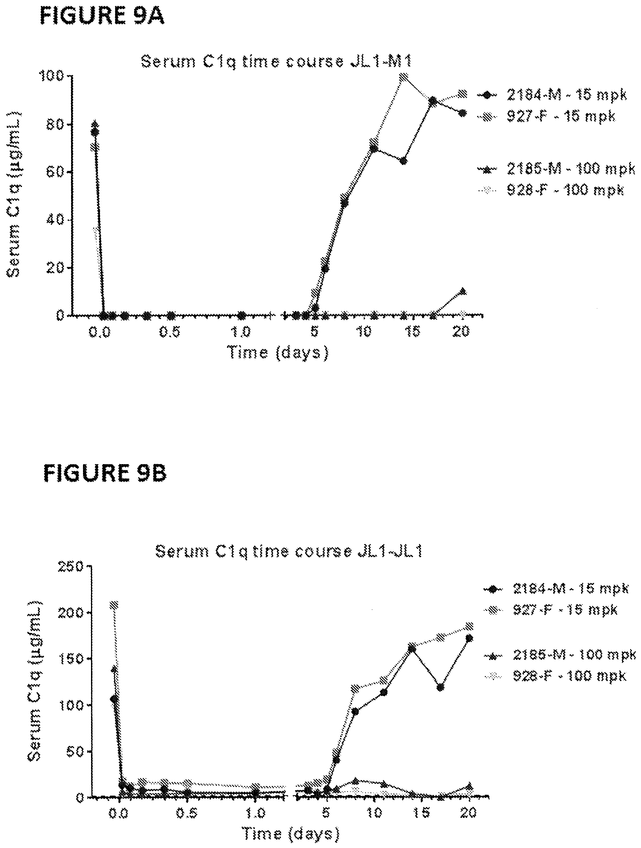

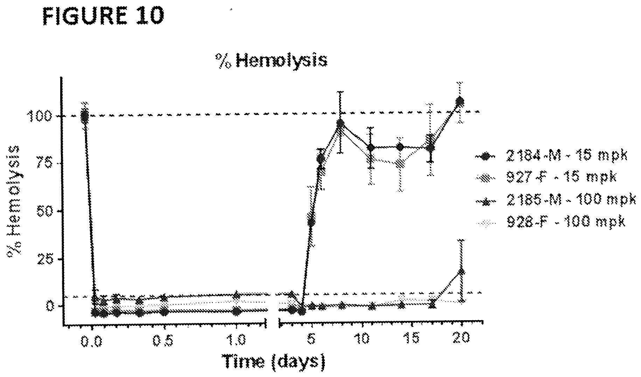

## Claims

1. A method for producing a humanized anti-C1q antibody, or an antigen-binding fragment thereof, wherein the method comprises
(a) culturing a host cell containing a nucleic acid encoding the humanized anti-C1q antibody under conditions suitable for the expression of the humanized anti-C1q antibody, and
(b) optionally recovering the humanized anti-C1q antibody from the host cell or host cell culture medium,
wherein the humanized anti-C1q antibody comprises:
a) a heavy chain variable domain comprising an amino acid sequence of SEQ ID NO: 1 and a light chain variable domain comprising an amino acid sequence of SEQ ID NO: 5;
b) a heavy chain variable domain comprising an amino acid sequence of SEQ ID NO: 2 and a light chain variable domain comprising an amino acid sequence of SEQ ID NO: 6;
c) a heavy chain variable domain comprising an amino acid sequence of SEQ ID NO: 3 and a light chain variable domain comprising an amino acid sequence of SEQ ID NO: 7; or
d) a heavy chain variable domain comprising an amino acid sequence of SEQ ID NO: 4 and a light chain variable domain comprising an amino acid sequence of SEQ ID NO: 8.

2. The method of claim 1 comprising prior to (a)
(1) isolating and inserting a nucleic acid encoding the humanized anti-C1q antibody into one or more vectors,
(2) introducing the one or more vectors into the host cell.

3. The method of claim 1 or 2, wherein the host cell is a prokaryotic cell or a eukaryotic cell, optionally wherein the prokaryotic cell is a yeast cell or *E*. *coli* or the eukaryotic cell is a mammalian cell, optionally wherein the mammalian cell is a Chinese hamster ovary (CHO) cell.

4. A kit comprising humanized anti-C1q antibody, or an antigen-binding fragment thereof, wherein the humanized anti-C1q antibody comprises:
a) a heavy chain variable domain comprising an amino acid sequence of SEQ ID NO: 1 and a light chain variable domain comprising an amino acid sequence of SEQ ID NO: 5;
b) a heavy chain variable domain comprising an amino acid sequence of SEQ ID NO: 2 and a light chain variable domain comprising an amino acid sequence of SEQ ID NO: 6;
c) a heavy chain variable domain comprising an amino acid sequence of SEQ ID NO: 3 and a light chain variable domain comprising an amino acid sequence of SEQ ID NO: 7; or
d) a heavy chain variable domain comprising an amino acid sequence of SEQ ID NO: 4 and a light chain variable domain comprising an amino acid sequence of SEQ ID NO: 8.

5. A kit comprising a humanized anti-C1q antibody, or an antigen-binding fragment thereof produced by the method of any one of claims 1-3.

6. The kit of claim 4 or 5 further including instructions comprising a description of administration of the humanized anti-C1q antibody to treat or diagnose a disease associated with complement activation.

7. The kit of any one of claims 4 to 6, wherein the kit is in a packaging.

8. The method of any one of claims 1 to 3 or the kit of any one of claims 4 to 7, wherein the antibody or antigen-binding fragment comprises a human IgG4 heavy chain constant region.

9. The method or kit of any one of the preceding claims, wherein the human IgG4 heavy chain constant region comprises the amino acid sequence of SEQ ID NO: 37.

10. The method or kit of claim 8, wherein the Fc region comprises an amino acid substitution at position 248 and/or position 241 according to Kabat numbering convention.

11. The method or kit of claim 10, wherein the amino acid substitution at position 248 is a leucine to glutamate amino acid substitution.

12. The method or kit of claim 10, wherein the amino acid substitution at position 241 is a serine to proline amino acid substitution.

13. The method of any one of claims 1 to 3 or the kit of any one of claims 4 to 7, wherein the antigen-binding fragment is a Fab, F(ab')₂ or Fab' fragment.

14. One or more vectors containing nucleic acids that encode an amino acid sequence containing the V_{L} and an amino acid sequence containing the V_{H} of an anti-C1q antibody, wherein anti-C1q antibody comprises:
a) a heavy chain variable domain comprising an amino acid sequence of SEQ ID NO: 1 and a light chain variable domain comprising an amino acid sequence of SEQ ID NO: 5;
b) a heavy chain variable domain comprising an amino acid sequence of SEQ ID NO: 2 and a light chain variable domain comprising an amino acid sequence of SEQ ID NO: 6;
c) a heavy chain variable domain comprising an amino acid sequence of SEQ ID NO: 3 and a light chain variable domain comprising an amino acid sequence of SEQ ID NO: 7; or
d) a heavy chain variable domain comprising an amino acid sequence of SEQ ID NO: 4 and a light chain variable domain comprising an amino acid sequence of SEQ ID NO: 8.

15. The one or more vectors of claim 14, wherein the one or more vectors are one or more expression vectors.
